# EUROPEAN PATENT APPLICATION

(11) **EP 3 882 242 A1**
(43) Date of publication of application: **22.09.2021**
(21) Application number: 19882210.8
(22) Date of filing: 08.11.2019
(51) Int. Cl.: C07D 401/12, C07D 405/14, C07D 409/12, C07D 409/14, C07D 413/14, C07D 417/14, A61K 31/454, A61P 35/00, C07D 513/04

(54) **COMPOUND INHIBITING YAP-TEAD BINDING, AND PHARMACEUTICAL COMPOSITION FOR PREVENTING OR TREATING CANCER, COMPRISING COMPOUND AS ACTIVE INGREDIENT**

(30) Priority: 09.11.2018 KR 20180137079; 07.11.2019 KR 20190141613
(71) Applicant: Korea Research Institute of Chemical Technology, Daejeon 34114 (KR); Industry-Academic Cooperation Foundation, Yonsei University, Seoul 03722 (KR)
(72) Inventor: LIM, Hwan Jung, Daejeon 34114 (KR); PARK, Seong Jun, Daejeon 34114 (KR); LEE, Chang Hoon, Daejeon 34114 (KR); NO, Kyoung Tai, Incheon 22008 (KR); CHOI, Jiwon, Seoul (KR); JEUNG, Hei-Cheul, Seoul (KR); SHIN, You-keun, Dongjak-gu, Seoul (KR); KIM, Jong Wan, Seoul 03724 (KR); JIN, Xuemei, Seoul 03720 (KR)
(74) Representative: dompatent von Kreisler Selting Werner - Partnerschaft von Patent- und Rechtsanwälten mbB
(86) International application number: PCT/KR2019/015172
(87) International publication number: WO 2020/096416

(57) **Abstract**

The present invention relates to a compound which inhibits the binding of Yes associated protein (YAP) and transcriptional enhancer associate domain (TEAD), a prodrug of same, a hydrate of same, a solvate of same or a pharmaceutically acceptable salt of same, and a composition comprising same as an active ingredient, the compound according to the present invention being able to be applied as an inhibitor which can directly inhibit YAP-TEAD binding in the Hippo pathway which plays a crucial role in the occurrence of cancer.

## Description

### [Technical Field]

The present invention relates to a compound inhibiting Yes associated protein (YAP)-transcriptional enhancer associate domain (TEAD) binding, a prodrug thereof, a hydrate thereof, a solvate thereof, or a pharmaceutically acceptable salt thereof, and a pharmaceutical composition including the same for preventing or treating cancer.

### [Background Art]

The latest anticancer drugs which have been developed so far include mainly a material which selectively hinders a function of a protein importantly involved in occurrence and growth of cancer cells as a main component. For example, among various receptor tyrosine kinases (RTK) which play a very important role in cancer cell growth, development of an inhibitor for epidermal growth factor receptor (EGFR), vascular endothelial growth factor (VGFR), or the like was followed by very encouraging clinical results. However, still, many signaling systems are involved in the occurrence and growth of cancer cells, and there are still many cases in which a successful inhibitor has not been developed. In the case in which a novel inhibitor which may regulate a cell signaling system which has yet to be developed is developed, a treatment effect for patients who are not treated with the anticancer drugs to date is expected to be improved.

"Hippo signaling", which is known as a signal system which is very important to occurrence and growth of cancer cells, is one of signaling routes inhibiting growth by regulating a cell cycle (for example, signaling routes in which p53 pathway, APC(adenomatous polyposis coli) gene, NF2(neurofibromatosis type 2) gene, and the like are involved), and since it was first found in drosophilas in 1995, studies therefor have been conducted so far (Genes Dev, Vol.9; pp.534-546). Hippo pathway, which is also known as "Salvador-Warts-Hippo pathway", is an important signaling system and a cell proliferation process which regulates cell proliferation and apoptosis and determine an organ size.

Since the Hippo pathway was found to be a signaling system regulating a tissue size of a drosophila and a mouse 10 years ago, recently, it began to emerge as a strong tumor suppressor in mammalian cells. The Hippo pathway is composed of a kinase cascade of MST1/2-LATS1/2 and is inhibited by phosphorylating a YAP/TAZ transcriptional coactivator which is an oncogene. When the Hippo pathway is inhibited, YAP/TAZ is activated and binds to a TEAD transcriptional regulatory factor, which plays an important role in occurrence, metastasis, drug resistance, and recurrence of various carcinomas as well as cell cycle and growth, stem cells, and regenerative medicine.

That is, when the Hippo signaling is activated, mammalian sterile20-like (MST) kinase forms a complex with salvador family WW domain containing protein 1 (SAV1) to phosphorylate a large tumor suppressor (LATS) kinase and a LATS kinase and an MOB1 cofactor phosphorylate YAP which is a subtarget thereof. The phosphorylated YAP binds to 14-3-3 protein and is decomposed in a protoplasm. Since YAP is decomposed, binding to TEAD family in a nucleus is hindered. That is, when the Hippo signaling is activated, YAP is inhibited and expression of a gene binding to YAP is inhibited to limit the growth of cells and tissues. However, with inactivation of the Hippo signaling, YAP enters into a nucleus to form a complex with TEAD which is a transcription factor and to cause gene expression, and cell growth is promoted and apoptosis is inhibited by secretion of growth factors such as connective tissue growth factor (CTGF), cystein-rich 61 (Cyr61), and fibroblast growth factor (FGF) (Genes Dev, Vol.26; pp.1300-1305). In occurrence and growth of cancer cells, it is seen that YAP protein directly enters into a nucleus and binds to TEAD protein which is a transcription factor to form a transcription complex and produce various growth factors such as CTGF and FGF in an excessive amount, thereby promoting proliferation and growth of cancer cells, and thus, it is seen that it is very important to prevent the function of TEAD protein as a transcription factor from being excessively activated (Cancer Res. 2007; 67: 9055-65). As an example, it is reported that high TEAD1 overexpression is related to poor prognosis of a patient in prostate cancer (Br J Cancer 2008; 99:1849-58), and also related to various human cancers such as basal-like breast cancer, fallopian tube carcinoma, and germ cell tumor (PLoS One 2012; 7:e45498).

Since the Hippo pathway may be involved in proliferation of cancer cells and tumors and expression of resistance to chemotherapy which is a conventional primary prescription therapeutic agent, it is evaluated as a very important mechanism in cancer treatment. According to recent research, it was reported that transcription of YAP-TEAD plays a very important role in expression of resistance to a lung cancer develop and an epidermal growth factor receptor - tyrosine kinase inhibitor (EGFR-TKI) (Biochem. Biophys. Res. Commun. Vol. 491 (2017), PP.493-499; Oncotarget Vol.9 (2018), pp.4637-4646).

In addition, there are important reports of research to show that there is programmed death-ligand 1 (PD-L1) which is important protein to regulate an immune environment in a cancer tissue among transcription targets by YAP-TEAD protein (Cancer Res. 2018 Mar 15; 78(6): 1457-1470; Oncotarget. 2017 Dec 9; 8(70): 114576-114587). According to the research results of the papers, it was reported that TAZ-TEAD transcription complex as well as YAP-TEAD plays a key role in overexpression of PD-L1 which is one of important proteins used as immune evasion mechanism by cancer cells in cancer patients. In addition, there are various reports for immunomodulation of Hippo signaling. As an example, an important pathway regulating a function of CD8+Dendritic cells has been recently reported (Nature 2018, Jun, 558 (7708)). The CD8+Dendritic cells are cells which are known to play an important role in anti-tumor immunity, among dendritic cells, and it was experimentally confirmed that when the Hippo signaling is inactivated, anti-tumor immunity greatly dropped. In addition, it was reported that when glycolysis is increased in human lung cancer cells to increase lactate, transcription of TAZ-TEAD is activated to greatly increase the expression of PD-L1 and G protein-coupled receptor 81 (GRP81) (Oncogene 2017 Oct 19; 36). It is an important research result showing that metabolic change in the majority of cancer tissues is an inevitable process and the resulting decrease in immune function is closely connected thereto, and it is seen that transcription by TEAD protein is very important in this process. Finally, there is an important report showing that YAP-TEAD protein increase a regulatory T cell (Treg cell) which is a core element of immune evasion mechanism in a cancer tissue (Cancer discovery 2018, Jun 15).

According to various reports as such, it is seen that TEAD protein is a transcription factor which plays a very important role in immune evasion mechanism in cancer tissues.

The importance of the roles of TEAD protein in the occurrence, growth, and immune evasion mechanism of cancer cells mentioned above has been clinically proved. The fact that a cancer occurrence process and the prognosis of YAP-TEAD and TAZ-TEAD transcription proteins are clinically correlated was reported, and as a result of analyzing clinical specimens of cancer patients, it was reported that Yes associated protein (YAP), transcriptional coactivator with PDZ-binding motif (TAZ), transcriptional enhancer associate domain (TEAD), and the like which are main factors of the Hippo pathway are overexpressed, and an expression degree thereof and a cancer patient survival period show statistical significance (Sci. Rep. Vol. 8 (2018), 271.; Oncotarget Vol.9 (2018), pp.4637-4646).

Accordingly, development of an inhibitor which may directly inhibit YAP-TEAD binding in the Hippo pathway which plays a key role in a cancer occurrence process is demanded, and it is considered that it will be possible to develop an anticancer drug having a high therapeutic effect and a low toxicity therefrom (Trends in biochem. Sci. 2017, 42, 862-872.; Int. J. of Mol. Sci. 2016, 17, 138).

### [Disclosure]

### [Technical Problem]

Thus, the present inventors found that a compound having a specific structure directly inhibits TEAD protein which is a transcription factor which is the most important transcription factor in Hippo signaling which is known as a very important signal system to occurrence and growth of cancer cells, thereby completing the present invention.

An object of the present invention is to provide a compound inhibiting YAP-TEAD binding, a prodrug thereof, a hydrate thereof, a solvate thereof, or a pharmaceutically acceptable salt thereof.

Another object of the present invention is to provide a pharmaceutical composition for preventing or treating cancer, including the compound inhibiting YAP-TEAD binding, the prodrug thereof, the hydrate thereof, the solvate thereof, or the pharmaceutically acceptable salt thereof as an effective component.

Another object of the present invention is to provide a YAP/TAZ-TEAD inhibitor composition including the compound, the prodrug thereof, the hydrate thereof, the solvate thereof, or the salt thereof as an effective component.

Still another object of the present invention is to provide a health functional food composition for preventing or improving cancer including the compound, the prodrug thereof, the hydrate thereof, the solvate thereof, or the pharmaceutically acceptable salt thereof as an effective component.

### [Technical Solution]

In one general aspect, a compound effectively inhibiting YAP-TEAD binding, represented by the following Chemical Formula 1, a prodrug thereof, a hydrate thereof, a solvate thereof, or a pharmaceutically acceptable salt thereof: 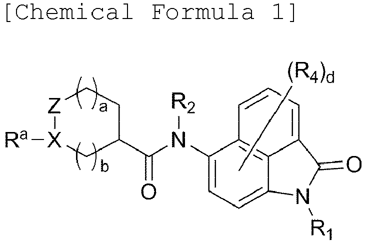 wherein
R^{a} is hydrogen or -L-R₃;
R₁ is hydrogen or -(CH₂)ₙ-R;
R is C1-C10alkoxy, C6-C20aryloxy, hydroxy, C1-C10alkylcarbonyloxy, C6-C20arylcarbonyloxy, cyano, halogen, nitro, C1-C10alkylsulfanyl, C6-C20arylsulfanyl, sulfanyl, - NR^{a1}R^{a2}, C1-C10alkoxyC1-C10alkyl, C6-C20aryloxyC1-C10alkyl, C1-C10alkoxyC1-C10alkoxy, C1-C10alkoxyC1-C10alkoxyC1-C10alkyl, C1-C10alkylsulfonyl, C6-C20arylsulfonyl, C1-C10alkylC6-C20arylsulfonyl, C6-C20arylC1-C10alkylsulfonyl, sulfo, C6-C20aryl, C3-C20cycloalkyl, carbamoyl, C1-C10alkylaminocarbonyl, C6-C20arylaminocarbonyl, sulfamoyl, C1-C10alkylaminosulfonyl, C6-C20arylaminosulfonyl, C1-C10alkylcarbonyl, C6-C20arylcarbonyl, C1-C10alkoxycarbonyl, C6-C20aryloxycarbonyl, carboxyl, formyl, C2-C20heteroaryl, or C3-C20heterocycloalkyl, and the aryl, heteroaryl, and heterocycloalkyl of R may be further substituted by one or more substituents selected from the group consisting of C1-C10alkyl, halogen, nitro, cyano, hydroxy, C1-C10alkoxy, C6-C20aryloxy, C1-C10alkylsulfanyl, haloC1-C10alkylsulfanyl, C6-C20arylsulfanyl, diC1-C10alkylamino, C1-C10alkylsulfonyl, C6-C20arylsulfonyl, C1-C10alkylC6-C20arylsulfonyl, C6-C20arylCl-C10alkylsulfonyl, C3-C20cycloalkyl, carboxyl, sulfo, formyl, carbamoyl, sulfamoyl, amino, C1-C10alkylcarbonyl, C6-C20arylcarbonyl, C1-C10alkoxycarbonyl, C6-C20aryloxycarbonyl, C1-C10alkylcarbonyloxy, C6-C20arylcarbonyloxy, C2-C20heteroaryl, and C3-C20heterocycloalkyl;
R^{a1} and R^{a2} are independently of each other hydrogen, C1-C10alkyl, C1-C10alkylsulfonyl, C6-C20arylsulfonyl, C1-C10alkylC6-C20arylsulfonyl, C6-C20arylC1-C10alkylsulfonyl, sulfo, C6-C20aryl, C3-C20cycloalkyl, or sulfamoyl;
n is an integer of 1 to 10;
R₂ is hydrogen, C1-C10alkyl, C1-C10alkoxyC1-C10alkyl, C3-C20cycloalkyl, C3-C20cycloalkyloxyC1-C10alkyl, C6-C20aryl, or C6-C20aryloxyCl-C10alkyl;
X is CH or N;
Z is -CH₂- or -CO-;
L is -SO₂-, -SO₂-L'-, -NHCO-, -CONH-, C1-C10 alkylene, or -CO-;
L' is -NH- or 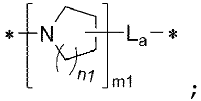
Lₐ is C1-C10alkylene;
n1 is an integer of 1 to 3;
m 1 is an integer of 0 or 1;
with a proviso that (i) when X is CH, L is -SO₂-NH-, and
(ii) when L is C1-C10 alkylene, a case in which R₃ is C1-C10alkyl, C1-C10alkoxy, or C6-C20aryloxy is excluded;
R₃ is C1-C10alkyl, C1-C10alkoxy, C6-C20aryloxy, C3-C20heterocycloalkyl, C6-C20aryl, or C2-C20heteroaryl, and the heterocycloalkyl, aryl, and heteroaryl of R₃ may be further substituted by one or more substituents selected from the group consisting of C1-C10alkyl, halogen, haloC1-C10alkyl, nitro, cyano, C1-C10alkylcarbonylamino, C6-C20arylcarbonylamino, amino, C1-C10alkoxy, haloC1-C10alkoxy, C1-C10alkoxyC1-C10alkoxy, C6-C20aryloxy, hydroxy, C1-C10alkylcarbonyl, haloC1-C10alkylcarbonyl, C6-C20arylcarbonyl, C1-C10alkoxycarbonyl, C6-C20aryloxycarbonyl, carboxyl, formyl, sulfanyl, C1-C10alkylsulfanyl, C6-C20arylsulfanyl, diC1-C10alkylaminoC1-C10alkoxy, dihydroxyaminosulfanyl, C1-C10alkylsulfonyl, C6-C20arylsulfonyl, C1-C10alkylC6-C20arylsulfonyl, C6-C20arylC1-C10alkylsulfonyl, sulfo, carbamoyl, C1-C10alkylaminocarbonyl, C6-C20arylaminocarbonyl, sulfamoyl, C1-C10alkylaminosulfonyl, C6-C20arylaminosulfonyl, C6-C20aryl, C2-C20heteroaryl, C1-C10alkylcarbonyloxy, C6-C20arylcarbonyloxy, and C3-C20heterocycloalkyl;
R₄ is halogen, haloC1-C10alkyl, cyano, C1-C10alkyl, C6-C20aryl, C2-C20heteroaryl, C1-C10alkoxy, C6-C20aryloxy, C2-C20heteroaryloxy, haloC1-C10alkoxy, hydroxy, amino, or aminoC1-C10alkyl;
d is an integer of 0 to 5, and when d is an integer of 2 or more, R₄ may be the same as or different from each other;
a and b are independently of each other an integer of 0, 1, or 2, and a+b is an integer of 1, 2 or 3; and
the heteroaryl and the heterocycloalkyl contains one or more heteroatoms selected from nitrogen, oxygen, and sulfur.

In another general aspect, a pharmaceutical composition for preventing or treating cancer, including the compound of Chemical Formula 1, the prodrug thereof, the hydrate thereof, the solvate thereof, or the pharmaceutically acceptable salt thereof as an effective component is provided.

In another general aspect, a YAP/TAZ-TEAD inhibitor composition including the compound of Chemical Formula 1, the prodrug thereof, the hydrate thereof, the solvate thereof, or the pharmaceutically acceptable salt thereof as an effective component is provided.

In still another general aspect, a health functional food composition for preventing or improving cancer including the compound of Chemical Formula 1, the prodrug thereof, the hydrate thereof, the solvate thereof, or the pharmaceutically acceptable salt thereof as an effective component is provided.

### [Advantageous Effects]

The compound of Chemical formula 1 according to the present invention may efficiently inhibit YAP-TEAD binding in the Hippo pathway known as a very important signal system to the occurrence and growth of cancer cells, thereby significantly inhibiting proliferation and growth of cancer cells, and the compound has a low toxicity to normal cells. Therefore, the compound of Chemical Formula 1 according to the present invention may be used well as an effective component of a pharmaceutical composition treating or preventing cancer.

### [Description of Drawings]

FIG. 1 shows a size and the number of colorectal cancer occurring in each group in an AOM/DSS model. (the number of experimental animal for each experimental group = 3) [initial point = the number of colorectal cancer directly before medication]
FIG. 2 shows weight change during medication in an AOM/DSS model.
FIG. 3 shows results of measuring a change degree in Treg cells by intestinal tissue FACS analysis in an AOM/DSS model.

### [Best Mode]

Hereinafter, the present invention will be described in detail. Technical terms and scientific terms used in the present specification have the general meaning understood by those skilled in the art to which the present invention pertains unless otherwise defined, and a description for the known function and configuration obscuring the present invention will be omitted in the following description.

The following terms used in the present specification are defined as follows, but these are only illustrative and the present invention, the present application, or a use thereof is not limited thereto.

The terms of the present specification "substituent", "radical", "group", "moiety", and "fragment" may be used interchangeably.

The term of the present specification "C_{A}-C_{B}" refers to "the number of carbon atoms being A or more and B or less".

The term of the present specification "alkyl" refers to a monovalent linear or branched saturated hydrocarbon radical composed only carbon and hydrogen atoms. The alkyl may have 1 to 10 carbon atoms, 1 to 6 carbon atoms, or 1 to 4 carbon atoms. An example of the alkyl includes methyl, ethyl, propyl, isopropyl, butyl, isobutyl, t-butyl, pentyl, hexyl, and the like, but is not limited thereto.

The term of the present specification "cycloalkyl" is a saturated or unsaturated carbocyclic radical composed of one or more rings and not an aromatic group. The cycloalkyl may be monocyclic or fused-, spiro-, or crosslinked bicyclic ring system. The cycloalkyl may have 3 to 20, preferably 3 to 10, and more preferably 3 to 7 carbon atoms. Specifically, a monocyclic cycloalkyl ring has 3 to 10 carbon atoms, preferably 3 to 7 carbon atoms in a ring. A bicyclic cycloalkyl ring has 7 to 17 carbon atoms, preferably 7 to 12 carbon atoms in a ring. A preferred bicyclic cycloalkyl ring includes 4-, 5-, 6- or 7-membered ring fused to a 5-, 6-, or 7-membered ring. A specific example of cycloalkyl may include cyclopropyl, cyclobutyl, cyclopentyl, cyclohexyl, and the like, but is not limited thereto.

The term of the present specification "aryl" refers to an organic radical derived from aromatic hydrocarbon by removal of one hydrogen and includes a monocyclic or fused ring system containing suitably 4 to 7, preferably 5 or 6 ring atoms in each ring, and even a form in which a plurality of aryls are linked by a single bond. A specific example thereof includes phenyl, naphthyl, biphenyl, anthryl, indenyl, fluorenyl, and the like, but is not limited thereto.

The term of the present specification "heteroaryl" refers to an aryl group containing 1 to 4 heteroatoms selected from N, O, and S as an aromatic ring backbone atom and carbon as a remaining aromatic ring backbone atom, includes a single or fused ring system, and may be partially saturated. In addition, the heteroaryl in the present specification also includes a form in which one or more heteroaryls are linked by a single bond. An example of the heteroaryl group includes monovalent radicals of an aromatic heterocycle such as pyrrole, quinoline, isoquinoline, pyridine, pyrimidine, oxazole, thiazole, pyrazole, thiadiazole, triazole, imidazole, benzimidazole, isoxazole, benzisoxazole, thiophene, benzothiophene, furan, benzofuran, imidazothiazole, benzothiadiazole, and benzoxadiazole, but is not limited thereto.

The term of the present specification "heterocycloalkyl" refers to a monovalent radical of a non-aromatic heterocycle containing 1 to 4 heteroatoms selected from the group consisting of N, O, and S, in which the non-aromatic heterocycle includes all forms of a saturated or unsaturated monocycle, polycycle or spirocycle and may be bonded via a heteroatom or a carbon atom, and nitrogen, carbon, oxygen, or sulfur atom in the non-aromatic heterocyclic radical is in various oxidation states and may be oxidized if necessary. In addition, a nitrogen atom in the non-aromatic heterocyclic radical may be quaternarized, if necessary. An example of the heterocycloalkyl radical may include monovalent radicals of non-aromatic heterocycles such as aziridine, pyrrolidine, pyrrolidinone, azetidine, piperidine, tetrahydropyridine, piperazine, morpholine, thiomorpholine, 4,5,6,7-tetrahydrobenzo[b]thiophene, 2,3-dihydrobenzofuran, benzo[d][1,3]dioxole, 3,4-dihydro-2H-chromene, naphthalene-2(1H)-one, 2H-chromen-2-one, 2,3-dihydrobenzo[b][1,4]dioxine, benzo[d]oxazol-2(3H)-one, pyrimidin-2,4(1H,3H)-dione, quinazolin-2,4(1H,3H)-dione, benzo[cd]indol-2(1H)-one, 3-azabicyclo[3.1.0]hexane, octahydropyrrolo[3,4-c]pyrrole, 2,7-diazaspiro[4.4]nonane, and 2-azaspiro[4.4]nonane.

The term of the present specification "alkoxy" refers to an -O-alkyl radical and may have 1 to 10 carbon atoms, preferably 1 to 6 carbon atoms, and more preferably 1 to 4 carbon atoms. The "alkyl" herein is as defined above. A specific example thereof includes methoxy, ethoxy, propoxy, isopropoxy, butoxy, isobutoxy, t-butoxy, and the like, but is not limited thereto.

The term of the present specification "aryloxy" refers to an -O-aryl radical, in which "aryl" is as defined above. An example of the aryloxy radical includes phenoxy and the like, but is not limited thereto.

The term of the present specification "halo" or "halogen" refers to a halogen group element, and an example thereof includes fluoro, chloro, bromo, and iodo.

The term of the present specification "haloalkyl" or "haloalkoxy" refers to an alkyl or alkoxy group in which one or more hydrogen atoms are substituted by a halogen atom, respectively, and the alkyl and halogen are as defined above. An example of haloalkyl may include fluoromethyl, difluoromethyl, trifluoromethyl, fluoroethyl, difluoroethyl, perfluoroethyl, and the like, and an example of haloalkoxy may include fluoromethoxy, difluoromethoxy, trifluoromethoxy, fluoroethoxy, difluoroethoxy, perfluoroethoxy, and the like.

In the present specification, the term "hydroxy" refers to -OH, the term "amino" refers to -NH₂, the term "nitro" refers to -NO₂, the term "cyano" refers to -CN, the term "carboxyl" refers to -COOH, the term "formyl" refers to - COH, the term "sulfanyl (or mercapto" refers to -SH, the term "sulfo" refers to -SO₃H, the term "sulfamoyl" refers to -SO₂NH₂, and the term "carbamoyl" refers to -CONH₂.

The term of the present specification "alkylsulfanyl" refers to a -S-alkyl radical, in which "alkyl" is as defined above. An example of the alkylsulfanyl radical includes methylsulfanyl, ethylsulfanyl, and the like, but is not limited thereto.

The term of the present specification "arylsulfanyl" refers to a -S-aryl radical, in which "aryl" is as defined above. An example of the arylsulfanyl radical includes phenylsulfanyl, naphthylsulfanyl, and the like, but is not limited thereto.

The term of the present specification "alkylsulfonyl" refers to a -SO₂-alkyl radical, in which "alkyl" is as defined above. An example of the alkylsulfonyl radical includes methylsulfonyl, ethylsulfonyl, and the like, but is not limited thereto.

The term of the present specification "alkylamino" refers to an amino radical substituted by one or two alkyls, and a specific example thereof includes methylamino (-NHMe), dimethylamino (-NMe₂), ethylamino (-NHEt), diethylamino (-NEt₂), and the like, but is not limited thereto.

The term of the present specification "arylamino" refers to an amino radical substituted by one or two aryls, and a specific example thereof includes pheylamino (-NHPh), diphenylamino (-NPh₂), and the like, but is not limited thereto.

The term of the present specification "alkylcarbonyl" refers to a -C(=O) alkyl radical, in which "alkyl" is as defined above. An example of the alkylcarbonyl radical includes methylcarbonyl, ethylcarbonyl, isopropylcarbonyl, propylcarbonyl, butylcarbonyl, isobutylcarbonyl, t-butylcarbonyl, and the like, but is not limited thereto.

The term of the present specification "alkoxycarbonyl" refers to a -C(=O)alkoxy radical, in which "alkoxy" is as defined above. An example of the alkoxycarbonyl radical includes methoxycarbonyl, ethoxycarbonyl, isopropoxycarbonyl, propoxycarbonyl, butoxycarbonyl, isobutoxycarbonyl, t-butoxycarbonyl, or the like, but not limited thereto.

The term of the present specification "alkylcarbonyloxy" refers to a -OC(=O) alkyl radical, in which "alkyl" is as defined above. An example of the alkylcarbonyloxy radical includes methylcarbonyloxy, ethylcarbonyloxy, isopropylcarbonyloxy, propylcarbonyloxy, butylcarbonyloxy, isobutylcarbonyloxy, t-butylcarbonyloxy, and the like, but is not limited thereto.

The term of the present specification "alkoxycarbonyloxy" refers to a -OC(=O)alkoxy radical, in which "alkoxy" is as defined above. An example of the alkoxycarbonyloxy radical includes methoxycarbonyloxy, ethoxycarbonyloxy, isopropoxycarbonyloxy, propoxycarbonyloxy, butoxycarbonyloxy, isobutoxycarbonyloxy, t-butoxycarbonyloxy, and the like, but is not limited thereto.

The term of the present specification "arylalkyl", "hydroxyalkyl", "akoxyalkyl", "aminoalkyl", or " (alkylaryl) alkyl" refers to an alkyl radical substituted by at least one R, that is, -(alkyl)-R, in which R is aryl, hydroxy, alkoxy, amino, or alkylaryl, and alkyl, aryl, hydroxy, alkoxy, amino, or alkylaryl is as defined above.

The term of the present specification "alkoxyalkoxy" or "hydroxyalkoxy" refers to an alkoxy radical substituted by at least one R, that is, -O-(alkyl)-R, in which R is the alkoxy or hydroxy defined above, and alkyl, alkoxy, or hydroxy is as defined above.

The term of the present specification "pharmaceutically acceptable" represents a characteristic of having no toxicity in an individual such as a cell or a human exposed to the composition, refers to being appropriate for use as a pharmaceutical preparation, is generally regarded as being safe for such use, and means being officially approved by a national management agency or being on the list of Korean pharmacopeia or U.S. pharmacopeia.

The term of the present specification "pharmaceutically acceptable salt" refers to any organic or inorganic adduct salt of the compound of the present invention having a concentration at which the salt is relatively non-toxic to patients and has a harmless effective function, a side effect of which does not decrease advantageous efficacy of the compound of the present invention itself.

The term of the present specification "cancer" refers to a cellular disorder characterized by unregulated or dysregulated cell proliferation, reduced cellular differentiation, inadequate ability to invade surrounding tissues, and/or ability to establish new growth in an ectopic site.

The term of the present specification "prevention" refers to all actions to suppress or delay occurrence, diffusion, and recurrence of cancer diseases by administration of the composition of the present invention.

The term of the present specification "treatment" refers to all actions to improve or cure the symptoms of cancer diseases by administration of the composition of the present invention.

The term of the present specification "improvement" refers to all actions to at least decrease parameters related to a treated state, for example, severity of symptoms by administration of the composition of the present invention.

The term of the present specification "individual" refers to all animals including a human with cancer diseases or having a possibility of occurrence of cancer diseases. The animal may be not only a human but also a mammal in need of treatment of similar symptoms thereto, such as cattle, a horse, sheep, a pig, a goat, a camel, an antelope, a dog, and a cat, but is not limited thereto. In addition, a human may be excluded from the individual in the present invention, but the present invention is not limited thereto.

The term of the present specification "administration" refers to introduction of the composition of the present invention to an individual by any appropriate method, and an administration route of the composition of the present invention may be performed by various routes orally or parenterally as long as the composition may reach to a target tissue by the route.

The term of the present specification "pharmaceutically effective amount" refers to an amount which is sufficient to treat diseases at a reasonable profit-risk ratio applicable to a medical treatment and does not cause a side effect.

The term of the present specification "food" includes meat, sausage, bread, chocolate, candies, snacks, sweets, pizza, ramen, other noodles, gum, dairy products including ice creams, various soups, beverages, tea, drinks, alcoholic beverages, vitamin complexes, health functional foods, health food, and the like, and includes all foods in the usual sense.

The term of the present specification "health functional food" refers to food which is manufactured and processed using raw materials or components having a functionality useful for a human body in accordance with Functional Foods for Health Act No. 6727, and the term "functionality" refers to intake for regulating nutrients for the structure and functions of a human body or obtaining an effect useful for health applications such as physiological action.

The present invention provides a compound represented by the following Chemical Formula 1, a prodrug thereof, a hydrate thereof, a solvate thereof, or a pharmaceutically acceptable salt thereof: 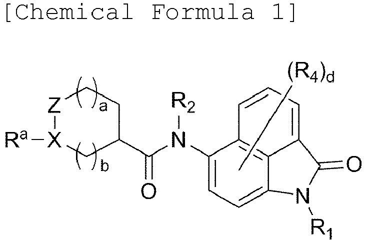 wherein
R^{a} is hydrogen or -L-R₃;
R₁ is hydrogen or -(CH₂)ₙ-R;
R is C1-C10alkoxy, C6-C20aryloxy, hydroxy, C1-C10alkylcarbonyloxy, C6-C20arylcarbonyloxy, cyano, halogen, nitro, C1-C10alkylsulfanyl, C6-C20arylsulfanyl, sulfanyl, - NR^{a1}R^{a2}, C1-C10alkoxyC1-C10alkyl, C6-C20aryloxyC1-C10alkyl, C1-C10alkoxyC1-C10alkoxy, C1-C10alkoxyC1-C10alkoxyC1-C10alkyl, C1-C10alkylsulfonyl, C6-C20arylsulfonyl, C1-C10alkylC6-C20arylsulfonyl, C6-C20arylC1-C10alkylsulfonyl, sulfo, C6-C20aryl, C3-C20cycloalkyl, carbamoyl, C1-C10alkylaminocarbonyl, C6-C20arylaminocarbonyl, sulfamoyl, C1-C10alkylaminosulfonyl, C6-C20arylaminosulfonyl, C1-C10alkylcarbonyl, C6-C20arylcarbonyl, C1-C10alkoxycarbonyl, C6-C20aryloxycarbonyl, carboxyl, formyl, C2-C20heteroaryl, or C3-C20heterocycloalkyl, and the aryl, heteroaryl, and heterocycloalkyl of R may be further substituted by one or more substituents selected from the group consisting of C1-C10alkyl, halogen, nitro, cyano, hydroxy, C1-C10alkoxy, C6-C20aryloxy, C1-C10alkylsulfanyl, haloC1-C10alkylsulfanyl, C6-C20arylsulfanyl, diC1-C10alkylamino, C1-C10alkylsulfonyl, C6-C20arylsulfonyl, C1-C10alkylC6-C20arylsulfonyl, C6-C20arylC1-C10alkylsulfonyl, C3-C20cycloalkyl, carboxyl, sulfo, formyl, carbamoyl, sulfamoyl, amino, C1-C10alkylcarbonyl, C6-C20arylcarbonyl, C1-C10alkoxycarbonyl, C6-C20aryloxycarbonyl, C1-C10alkylcarbonyloxy, C6-C20arylcarbonyloxy, C2-C20heteroaryl, and C3-C20heterocycloalkyl;
R^{a1} and R^{a2} are independently of each other hydrogen, C1-C10alkyl, C1-C10alkylsulfonyl, C6-C20arylsulfonyl, C1-C10alkylC6-C20arylsulfonyl, C6-C20arylC1-C10alkylsulfonyl, sulfo, C6-C20aryl, C3-C20cycloalkyl, or sulfamoyl;
n is an integer of 1 to 10;
R₂ is hydrogen, C1-C10alkyl, C1-C10alkoxyC1-C10alkyl, C3-C20cycloalkyl, C3-C20cycloalkyloxyC1-C10alkyl, C6-C20aryl, or C6-C20aryloxyC1-C10alkyl;
X is CH or N;
Z is -CH₂- or -CO-;
L is -SO₂-, -SO₂-L'-, -NHCO-, -CONH-, C1-C10 alkylene, or -CO-;
L' is -NH- or 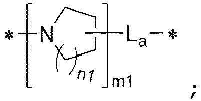
Lₐ is C1-C10alkylene;
n1 is an integer of 1 to 3;
m 1 is an integer of 0 or 1;
with a proviso that (i) when X is CH, L is -SO₂-NH-, and
(ii) when L is C1-C10 alkylene, a case in which R₃ is C1-C10alkyl, C1-C10alkoxy, or C6-C20aryloxy is excluded;
R₃ is C1-C10alkyl, C1-C10alkoxy, C6-C20aryloxy, C3-C20heterocycloalkyl, C6-C20aryl, or C2-C20heteroaryl, and the heterocycloalkyl, aryl, and heteroaryl of R₃ may be further substituted by one or more substituents selected from the group consisting of C1-C10alkyl, halogen, haloC1-C10alkyl, nitro, cyano, C1-C10alkylcarbonylamino, C6-C20arylcarbonylamino, amino, C1-C10alkoxy, haloC1-C10alkoxy, C1-C10alkoxyC1-C10alkoxy, C6-C20aryloxy, hydroxy, C1-C10alkylcarbonyl, haloC1-C10alkylcarbonyl, C6-C20arylcarbonyl, C1-C10alkoxycarbonyl, C6-C20aryloxycarbonyl, carboxyl, formyl, sulfanyl, C1-C10alkylsulfanyl, C6-C20arylsulfanyl, diC1-C10alkylaminoC1-C10alkoxy, dihydroxyaminosulfanyl, C1-C10alkylsulfonyl, C6-C20arylsulfonyl, C1-C10alkylC6-C20arylsulfonyl, C6-C20arylCl-C10alkylsulfonyl, sulfo, carbamoyl, C1-C10alkylaminocarbonyl, C6-C20arylaminocarbonyl, sulfamoyl, C1-C10alkylaminosulfonyl, C6-C20arylaminosulfonyl, C6-C20aryl, C2-C20heteroaryl, C1-C10alkylcarbonyloxy, C6-C20arylcarbonyloxy, and C3-C20heterocycloalkyl;
R₄ is halogen, haloC1-C10alkyl, cyano, C1-C10alkyl, C6-C20aryl, C2-C20heteroaryl, C1-C10alkoxy, C6-C20aryloxy, C2-C20heteroaryloxy, haloC1-C10alkoxy, hydroxy, amino, or aminoC1-C10alkyl;
d is an integer of 0 to 5, and when d is an integer of 2 or more, R₄ may be the same as or different from each other;
a and b are independently of each other an integer of 0, 1, or 2, and a+b is an integer of 1, 2 or 3; and
the heteroaryl and the heterycycloalkyl contains one or more heteroatoms selected from nitrogen, oxygen, and sulfur.

In an exemplary embodiment, the compound of Chemical Formula 1 may be represented by the following Chemical formula 2: 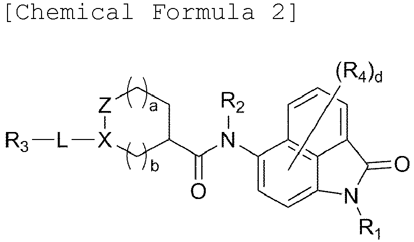 wherein R₁, R₂, X, Z, L, R₃, R₄, a, b and d are as defined in Chemical Formula 1.

The compound of Chemical Formula 1 according to the present invention, particularly preferably the compound of Chemical Formula 2 directly inhibits TEAD protein which is the most important transcription factor in Hippo signaling known as a very important signal system in the occurrence and growth of cancer cells. That is, the compound of Chemical Formula 1, particularly preferably the compound of Chemical Formula 2 shows YAP-TEAD binding inhibition activity in the Hippo pathway, and thus, is useful as a therapeutic agent and a prophylactic agent of cancer which is a disease mediated by YAP-TEAD binding. That is, the compound of Chemical Formula 1, particularly preferably the compound of Chemical Formula 2, binds to TEAD competitively with YAP to inhibit YAP-TEAD interaction to activate the Hippo pathway, thereby controlling the growth of cancer cells. In addition, the compound of Chemical Formula 1 according to the present invention shows a low toxicity to normal cells.

In Chemical Formula 2 according to an exemplary embodiment, a+b may be an integer of 1 or 2.

In Chemical Formula 2 according to an exemplary embodiment, X may be N, a may be an integer of 1 or 2, and b may be an integer of 0.

In Chemical Formula 2 according to an exemplary embodiment, X may be N, a may be an integer of 0, and b may be an integer of 1.

In Chemical Formula 2 according to an exemplary embodiment, X may be N, a may be an integer of 1, and b may be an integer of 1.

In Chemical Formula 2 according to an exemplary embodiment, X may be N, a may be an integer of 0, and b may be an integer of 2.

The compound of Chemical Formula 1 may be specifically a compound represented by the following Chemical Formula 3, 4, 5, 6, or 7: 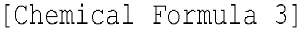 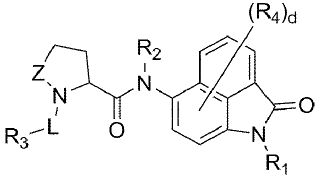 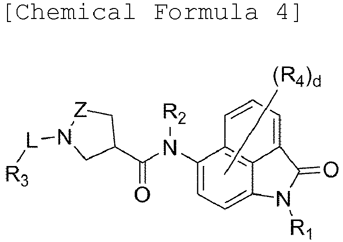 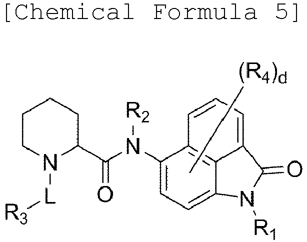 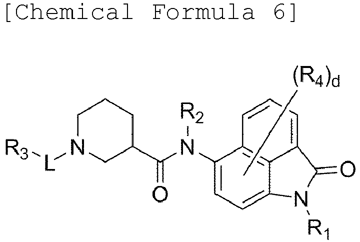 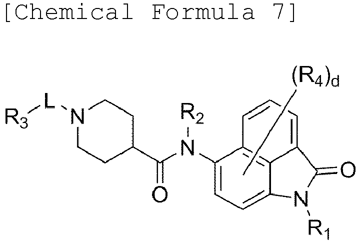 wherein R₁, R₂, Z, L, R₃, R₄, and d are as defined in Chemical Formula 2.

In Chemical Formula 2 according to an exemplary embodiment, X may be CH, a may be an integer of 0, b may be an integer of 2, and L may be -SO₂-NH-.

In Chemical Formula 2 according to an exemplary embodiment, X may be CH, a may be an integer of 1, b may be an integer of 1, and L may be -SO₂-NH-.

In Chemical Formula 2 according to an exemplary embodiment, X may be CH, a may be an integer of 2, b may be an integer of 0, and L may be -SO₂-NH-.

The compound of Chemical Formula 2 may be specifically a compound represented by the following Chemical Formula 8, 9, or 10:

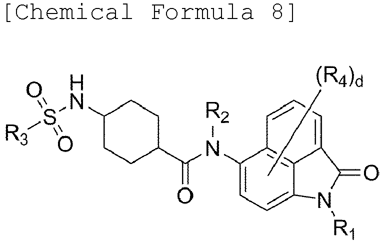

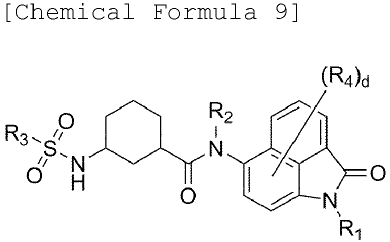

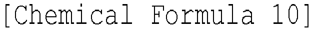

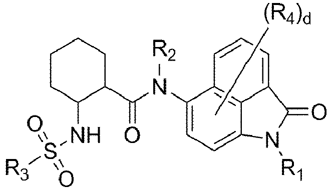

wherein R₁, R₂, R₃, R₄, and d are as defined in Chemical Formula 2.

In Chemical Formula 2 according to an exemplary embodiment, X may be N, a may be an integer of 1, b may be an integer of 0, Z may be -CH₂-, and L may be -SO₂-.

In Chemical Formula 2 according to an exemplary embodiment, X may be N, a may be an integer of 1, b may be an integer of 0, Z may be -CO-, and L may be -SO₂- or C1-C5alkylene.

In Chemical Formula 2 according to an exemplary embodiment, X may be N, a may be an integer of 0, b may be an integer of 1, Z may be -CH₂-, and L may be -SO₂-.

In Chemical Formula 2 according to an exemplary embodiment, X may be N, a may be an integer of 2, b may be an integer of 0, L may be -SO₂-, -CO-, -NHCO-, 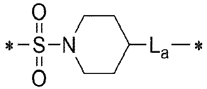 , or 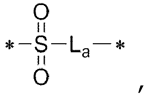 and Lₐ may be C1-C5alkylene.

In Chemical Formula 2 according to an exemplary embodiment, X may be N, a may be an integer of 1, b may be an integer of 1, L may be -SO₂-, 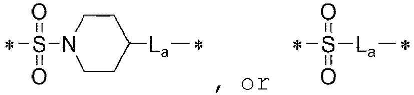 , and Lₐ may be C1-C5alkylene.

In Chemical Formula 2 according to an exemplary embodiment, X may be N, a may be an integer of 0, b may be an integer of 2, L may be -SO₂-, 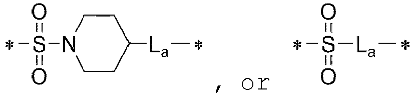 , and Lₐ may be C1-C5alkylene.

The compound of Chemical Formula 3 may be specifically a compound represented by the following Chemical Formula 11, 12, or 13:

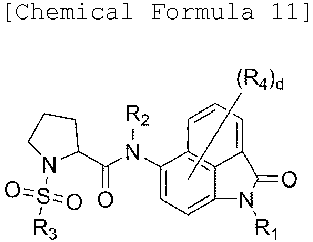

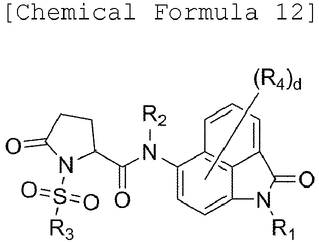

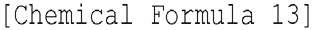

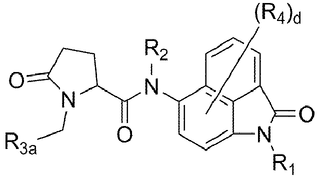

wherein R₁, R₂, R₃, R₄, and d are as defined in Chemical Formula 2; and

R₃ₐ is C3-C20heterocycloalkyl, C6-C20aryl, or C2-C20heteroaryl, and the heterocycloalkyl, aryl, and heteroaryl of R₃ₐ may be further substituted by one or more substituents selected from the group consisting of C1-C10alkyl, halogen, haloC1-C10alkyl, nitro, cyano, C1-C10alkylcarbonylamino, C6-C20arylcarbonylamino, amino, C1-C10alkoxy, haloC1-C10alkoxy, C1-C10alkoxyC1-C10alkoxy, C6-C20aryloxy, hydroxy, C1-C10alkylcarbonyl, haloC1-C10alkylcarbonyl, C6-C20arylcarbonyl, carboxyl, formyl, sulfanyl, C1-C10alkylsulfanyl, C6-C20arylsulfanyl, diC1-C10alkylaminoC1-C10alkoxy, dihydroxyaminosulfanyl, C1-C10alkylsulfonyl, C6-C20arylsulfonyl, sulfo, carbamoyl, C1-C10alkylaminocarbonyl, C6-C20arylaminocarbonyl, sulfamoyl, C1-C10alkylaminosulfonyl, C6-C20arylaminosulfonyl, C6-C20aryl, C2-C20heteroaryl, and C3-C20heterocycloalkyl.

The compound of Chemical Formula 4 may be specifically a compound represented by the following Chemical Formula 14:

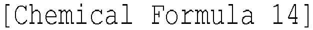

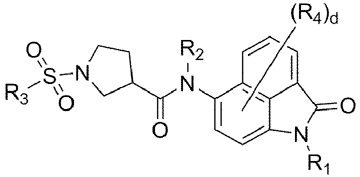

wherein R₁, R₂, R₃, R₄, and d are as defined in Chemical Formula 2.

The compound of Chemical Formula 5 may be specifically a compound represented by the following Chemical Formula 15, 16, 17, 18, or 19: 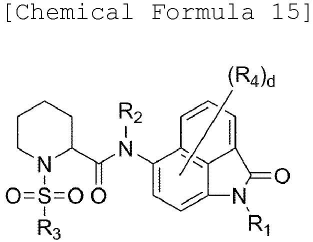 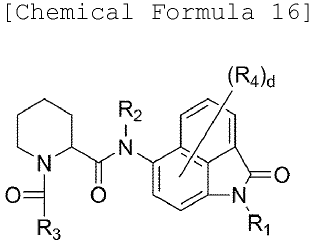 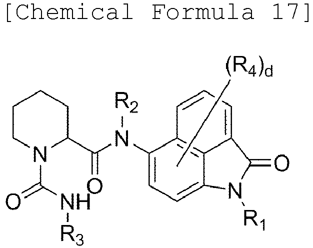 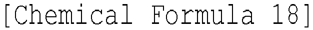 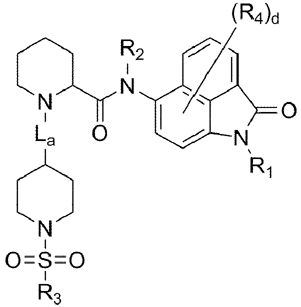 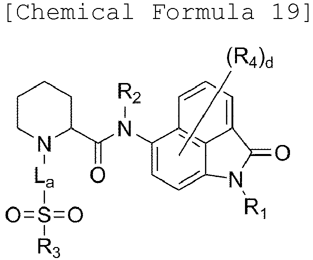
wherein R₁, R₂, R₃, R₄, and d are as defined in Chemical Formula 2; and
Lₐ is C1-C5alkylene.

The compound of Chemical Formula 6 may be specifically a compound represented by the following Chemical Formula 20, 21, or 22: 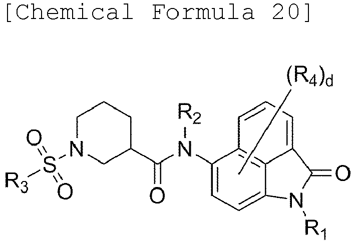 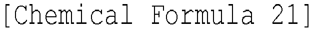 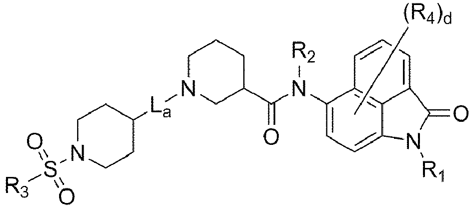 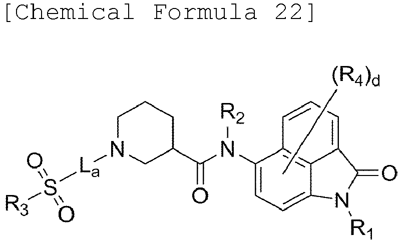
wherein R₁, R₂, R₃, R₄, and d are as defined in Chemical Formula 2; and
Lₐ is C1-C5alkylene.

The compound of Chemical Formula 7 may be specifically a compound represented by the following Chemical Formula 23, 24, or 25: 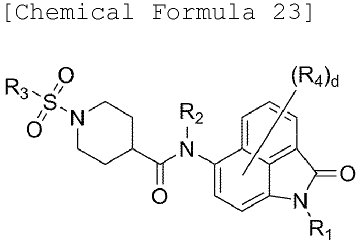 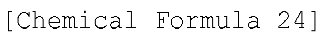 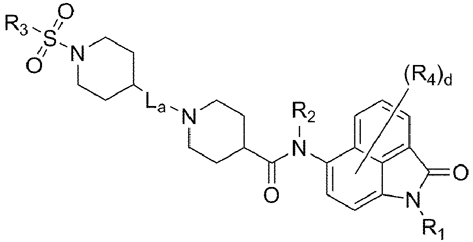 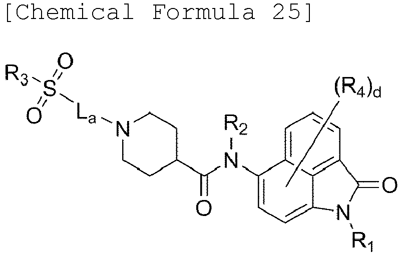
wherein R₁, R₂, R₃, R₄, and d are as defined in Chemical Formula 2; and
Lₐ is C1-C5alkylene.

In the compounds of Chemical Formulae 8 to 25 according to an exemplary embodiment:
R₁ may be hydrogen or -(CH₂)ₙ-R;
R may be C1-C6alkoxy, C6-C12aryloxy, hydroxy, cyano, halogen, nitro, C1-C6alkylsulfanyl, C6-C12arylsulfanyl, sulfanyl, -NR^{a1}R^{a2}, C1-C6alkoxyC1-C6alkyl, C6-C12aryloxyC1-C6alkyl, C1-C6alkoxyC1-C6alkoxy, C1-C6alkoxyC1-C6alkoxyC1-C6alkyl, C1-C6alkylsulfonyl, C6-C12arylsulfonyl, C1-C6alkylC6-C12arylsulfonyl, C6-C12arylC1-C6alkylsulfonyl, sulfo, C6-C12aryl, C1-C6alkoxycarbonyl, C6-C12aryloxycarbonyl, carboxyl, formyl, C2-C12heteroaryl, or C3-C12heterocycloalkyl, and the aryl, heteroaryl, and heterocycloalkyl of R may be further substituted by one or more substituents selected from the group consisting of C1-C6alkyl, halogen, nitro, cyano, hydroxy, C1-C6alkoxy, C6-C12aryloxy, C1-C6alkylsulfonyl, C6-C12arylsulfonyl, C1-C6alkylC6-C12arylsulfonyl, C6-C12arylC1-C6alkylsulfonyl, carboxyl, sulfo, formyl, carbamoyl, sulfamoyl, and amino;
R^{a1} and R^{a2} are independently of each other hydrogen, C1-C6alkyl, C1-C6alkylsulfonyl, C6-C12arylsulfonyl, C1-C6alkylC6-C12arylsulfonyl, C6-C12arylC1-C6alkylsulfonyl, sulfo, or sulfamoyl;
n may be an integer of 1 to 5;
R₂ may be hydrogen, C1-C6alkyl, C1-C6alkoxyC1-C6alkyl, or C6-C12aryloxyC1-C6alkyl;
R₃ may be C1-C6alkyl, C1-C6alkoxy, C6-C12aryloxy, C3-C12heterocycloalkyl, C6-C12aryl, or C2-C12heteroaryl;
R₃ₐ may be C3-C12heterocycloalkyl, C6-C12aryl, or C2-C12heteroaryl;
the heterocycloalkyl, aryl, and heteroaryl of R₃ and R₃ₐ may be further substituted by one or more substituents selected from the group consisting of C1-C6alkyl, halogen, haloC1-C6alkyl, nitro, cyano, C1-C6alkylcarbonylamino, C6-C12arylcarbonylamino, amino, C1-C6alkoxy, haloC1-C6alkoxy, C1-C6alkoxyC1-C6alkoxy, C6-C12aryloxy, hydroxy, C1-C6alkylcarbonyl, haloC1-C6alkylcarbonyl, C6-C12arylcarbonyl, carboxyl, formyl, sulfanyl, C1-C6alkylsulfanyl, C6-C12arylsulfanyl, diC1-C6alkylaminoC1-C6alkoxy, dihydroxyaminosulfanyl, C1-C6alkylsulfonyl, C6-C12arylsulfonyl, sulfo, carbamoyl, C1-C6alkylaminocarbonyl, C6-C12arylaminocarbonyl, sulfamoyl, C1-C6alkylaminosulfonyl, C6-C12arylaminosulfonyl, C6-C12aryl, C2-C12heteroaryl, and C3-C12heterocycloalkyl;
Lₐ may be C1-C5alkylene;
R₄ may be halogen, haloC1-C6alkyl, cyano, C1-C6alkyl, C6-C12aryl, C2-C12heteroaryl, C1-C6alkoxy, C6-C12aryloxy, C2-C12heteroaryloxy, haloC1-C6alkoxy, hydroxy, amino, or aminoCl-C6alkyl; and
d may be an integer of 0 to 5, and when d is an integer of 2 or more, R₄ may be the same as or different from each other.
In an exemplary embodiment, the compound of Chemical Formula 2 may be preferably a compound selected from Chemical Formulae 8 to 25.
In the compound according to an exemplary embodiment, R₁ may be hydrogen or -(CH₂)ₙ-R;
R may be C1-C6alkoxy, hydroxy, cyano, halogen, C1-C6alkylsulfanyl, -NR^{a1}R^{a2}, C1-C6alkoxyC1-C6alkyl, C1-C6alkoxyC1-C6alkoxy, C1-C6alkoxyC1-C6alkoxyC1-C6alkyl, C1-C6alkylsulfonyl, C6-C12arylsulfonyl, C6-C12aryl, C1-C6alkoxycarbonyl, C6-C12aryloxycarbonyl, C2-C12heteroaryl, or C3-C12heterocycloalkyl, and the aryl, heteroaryl, and heterocycloalkyl of R may be further substituted by one or more substituents selected from the group consisting of C1-C6alkyl, halogen, nitro, cyano, hydroxy, C1-C6alkoxy, C6-C12aryloxy, C1-C6alkylsulfonyl, C6-C12arylsulfonyl, C1-C6alkylC6-C12arylsulfonyl, C6-C12arylC1-C6alkylsulfonyl, carboxyl, sulfo, and formyl;
R^{a1} and R^{a2} may be independently of each other C1-C6alkyl, C1-C6alkylsulfonyl, or C6-C12arylsulfonyl;
n may be an integer of 1 to 3;
R₂ may be hydrogen or C1-C6alkoxyC1-C6alkyl;
R₃ may be C1-C6alkyl, C1-C6alkoxy, C3-C12heterocycloalkyl, C6-C12aryl, or C2-C12heteroaryl;
R₃ₐ may be C3-C12heterocycloalkyl, C6-C12aryl, or C2-C12heteroaryl;
the heterocycloalkyl, aryl, and heteroaryl of R₃ and R₃ₐ may be further substituted by one or more substituents selected from the group consisting of C1-C6alkyl, halogen, haloC1-C6alkyl, nitro, cyano, C1-C6alkylcarbonylamino, C6-C12arylcarbonylamino, amino, C1-C6alkoxy, haloC1-C6alkoxy, C1-C6alkoxyC1-C6alkoxy, hydroxy, C1-C6alkylcarbonyl, haloC1-C6alkylcarbonyl, C6-C12arylcarbonyl, carboxyl, formyl, sulfanyl, C1-C6alkylsulfanyl, C6-C12arylsulfanyl, diC1-C6alkylaminoC1-C6alkoxy, dihydroxyaminosulfanyl, C1-C6alkylsulfonyl, C6-C12arylsulfonyl, sulfo, carbamoyl, sulfamoyl, C6-C12aryl, C2-C12heteroaryl, and C3-C12heterocycloalkyl;
R₄ may be halogen, haloC1-C6alkyl, cyano, C1-C6alkyl, C6-C12aryl, C2-C12heteroaryl, C1-C6alkoxy, C6-C12aryloxy, C2-C12heteroaryloxy, haloC1-C6alkoxy, hydroxy, amino, or aminoCl-C6alkyl; and
d may be an integer of 0 to 5, and when d is an integer of 2 or more, R₄ may be the same as or different from each other.

In the compound according to an exemplary embodiment, R₁ may be hydrogen.

In the compound according to an exemplary embodiment, preferably, R₁ may be -(CH₂)ₙ-OR₁₁, -(CH₂)ₙ-OH, -(CH₂)ₙ-CN, - (CH₂)ₙ-X₁, - (CH₂)ₙ-SR₁₂, - (CH₂)ₙ-NR₁₃R₁₄, - (CH₂)ₙ-NR₁₃-SO₂R₁₅, - (CH₂)ₙ-L₁-ORₙ, - (CH₂)ₙ-SO₂R₁₆, or - (CH₂)ₙ-C(=O)OR₁₇, or may be selected from the following structures: 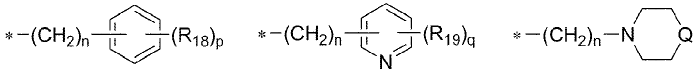 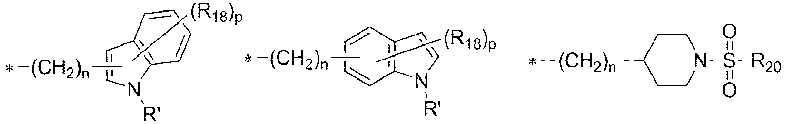
wherein R₁₁ is C1-C4alkyl or C1-C4alkoxyC1-C4alkyl;
X₁ is halogen;
R₁₂ to R₁₄ are independently of each other C1-C4alkyl;
R₁₅, R₁₆, R₁₇, and R₂₀ are independently of one another C1-C4alkyl or C6-C12aryl;
L₁ is C₁-C₄ alkylene;
R₁₈ and R₁₉ are independently of each other C1-C4alkyl, halogen, nitro, cyano, hydroxy, C1-C4alkoxy, C6-C12aryloxy, carboxyl, sulfo, or formyl;
R' is hydrogen or C1-C4alkyl;
Q is CH₂, NH, O, or S;
n is an integer of 1 to 3;
p is an integer of 0 to 5, and when p is an integer of 2 or more, R₁₈ may be the same as or different from each other; and
q is an integer of 0 to 4, and when q is an integer of 2 or more, R₁₉ may be the same or different from each other.

More preferably, R₁ may be selected from the following structures:

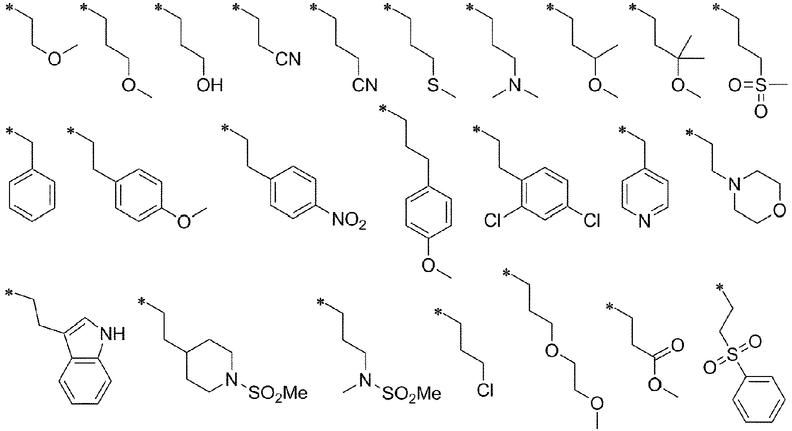

In the compound according to an exemplary embodiment, R₃ may be C1-C6alkyl, C1-C6alkoxy, C3-C12heterocycloalkyl, C6-C12aryl, or C2-C12heteroaryl, and the heterocycloalkyl, aryl, and heteroaryl of R₃ may be further substituted by one or more substituents selected from the group consisting of C1-C6alkyl, halogen, haloC1-C6alkyl, nitro, C1-C6alkylcarbonylamino, amino, C1-C6alkoxy, haloC1-C6alkoxy, C1-C6alkoxyC1-C6alkoxy, hydroxy, haloC1-C6alkylcarbonyl, carboxyl, diC1-C6alkylaminoC1-C6alkoxy, dihydroxyaminosulfanyl, C1-C6alkylsulfonyl, C6-C12aryl, and C2-C12heteroaryl.

In the compound according to an exemplary embodiment, R₂ may be hydrogen or C1-C4alkoxyC1-C4alkyl;
R₃ may be selected from C1-C4alkyl, C1-C4alkoxy, or the following structures: 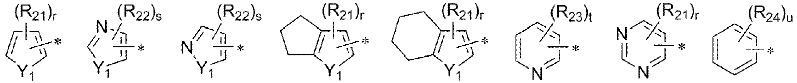 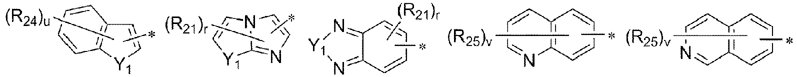 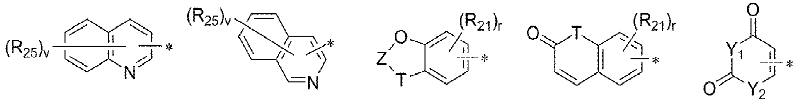 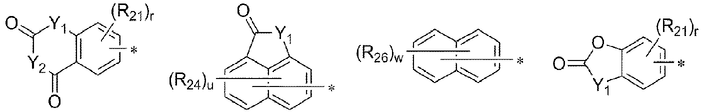 ,
R₂₁ to R₂₆ may be independently of one another C1-C4alkyl, halogen, haloC1-C4alkyl, nitro, cyano, C1-C4alkylcarbonylamino, C6-C12arylcarbonylamino, amino, C1-C4alkoxy, haloC1-C4alkoxy, C1-C4alkoxyC1-C4alkoxy, hydroxy, C1-C4alkylcarbonyl, haloC1-C4alkylcarbonyl, C6-C12arylcarbonyl, carboxyl, formyl, sulfanyl, C1-C4alkylsulfanyl, C6-C12arylsulfanyl, diC1-C4alkylaminoC1-C4alkoxy, dihydroxyaminosulfanyl, C1-C4alkylsulfonyl, C6-C12arylsulfonyl, sulfo, carbamoyl, sulfamoyl, C6-C12aryl, C2-C12heteroaryl, or C3-C12heterocycloalkyl;
Y₁ and Y₂ may be independently of each other NR", O, or S;
Z may be - (CR₂₇R₂₈)ₓ-;
R₂₇ and R₂₈ may be independently of each other hydrogen, C1-C4alkyl, or halogen;
x may be an integer of 1 to 3;
T may be CH₂ or O;
R" may be hydrogen or C1-C4alkyl;
r may be an integer of 0 to 3, and when r is an integer of 2 or more, R₂₁ may be the same as or different from each other; s may be an integer of 0 to 2, and when s is an integer of 2 or more, R₂₂ may be the same as or different from each other; t may be an integer of 0 to 4, and when t is an integer of 2 or more, R₂₃ may be the same as or different from each other; u may be an integer of 0 to 5, and when u is an integer of 2 or more, R₂₄ may be the same as or different from each other; v may be an integer of 0 to 6, and when v is an integer of 2 or more, R₂₅ may be the same as or different from each other; and w may be an integer of 0 to 7, and when w is an integer of 2 or more, R₂₆ may be the same as or different from each other;
R₄ may be halogen, haloC1-C4alkyl, cyano, C1-C4alkyl, Ar₁, HET₁, C1-C4alkoxy, -O-Ar₁, -O-HET₁, haloC1-C4alkoxy, hydroxy, amino, or aminoC1-C4alkyl;
Ar₁ may be phenyl, biphenyl, or naphthyl;
HET₁ may be pyrrolyl, furyl, thienyl, pyrazolyl, isoxazolyl, isothiazolyl, imidazolyl, oxazolyl, thiazolyl, triazolyl, oxadiazolyl, thiadiazolyl, pyridinyl, pyridazinyl, pyrimidyl, pyrazinyl, triazinyl, quinolinyl, isoquinolinyl, indolyl, benzofuranyl, benzothienyl, isoindolyl, indazolyl, benzoisoxazolyl, benzoisothiazolyl, benzoimidazolyl, benzoxazolyl, benzothiazolyl, or benzotriazolyl; and
d may be an integer of 0 to 3, and when d is an integer of 2 or more, R₄ may be the same as or different from each other.

In the compound according to an exemplary embodiment, preferably, R₄ may be halogen, haloC1-C4alkyl, or C1-C4alkoxy.

In the compound according to an exemplary embodiment, R₃ may be selected from C1-C4alkyl, C1-C4alkoxy, or the following structures: 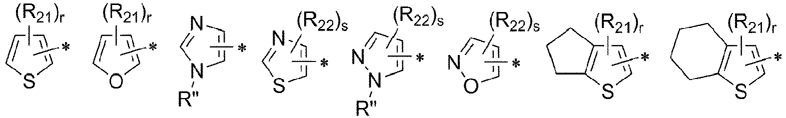 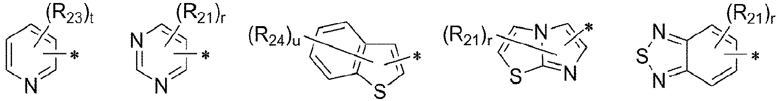 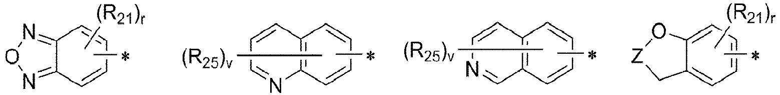 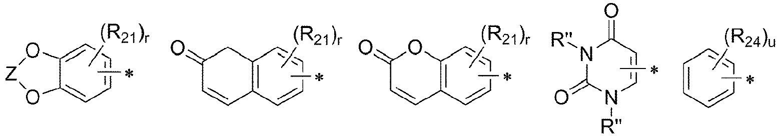 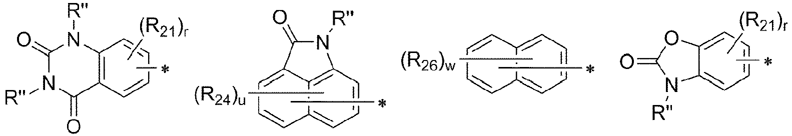
wherein R₂₁ to R₂₆ are independently of one another C1-C4alkyl, halogen, haloC1-C4alkyl, nitro, C1-C4alkylcarbonylamino, amino, C1-C4alkoxy, haloC1-C4alkoxy, C1-C4alkoxyC1-C4alkoxy, hydroxy, haloC1-C4alkylcarbonyl, carboxyl, diC1-C4alkylaminoC1-C4alkoxy, dihydroxyaminosulfanyl, C1-C4alkylsulfonyl, C6-C12aryl, or C2-C12heteroaryl;
Z is -(CR₂₇R₂₈)ₓ-;
R₂₇ and R₂₈ are independently of each other hydrogen, C1-C4alkyl, or halogen;
x is an integer of 1 to 3;
R" is hydrogen or C1-C4alkyl; and
r is an integer of 0 to 3, and when r is an integer of 2 or more, R₂₁ may be the same as or different from each other; s is an integer of 0 to 2, and when s is an integer of 2 or more, R₂₂ may be the same as or different from each other; t is an integer of 0 to 4, and when t is an integer of 2 or more, R₂₃ may be the same as or different from each other; u is an integer of 0 to 5, and when u is an integer of 2 or more, R₂₄ may be the same as or different from each other; v is an integer of 0 to 6, and when v is an integer of 2 or more, R₂₅ may be the same as or different from each other; and w is an integer of 0 to 7, and when w is an integer of 2 or more, R₂₆ may be the same as or different from each other.

More specifically, R₃ may be methyl, ethyl, propyl, butyl, methoxy, ethoxy, propoxy, or butoxy, or may be selected from the following structures:

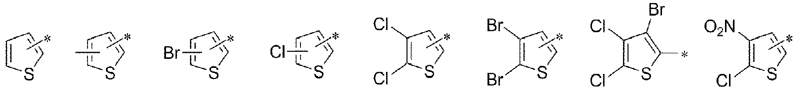

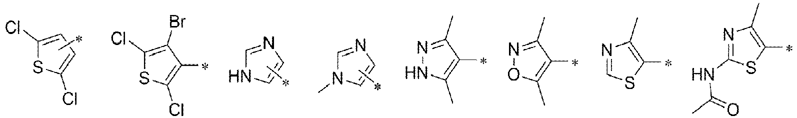

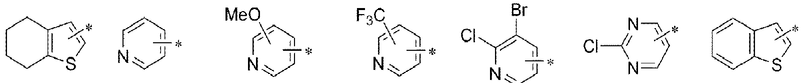

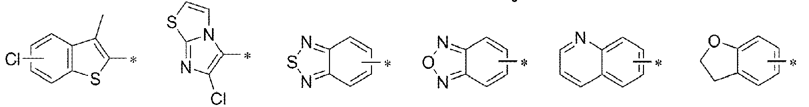

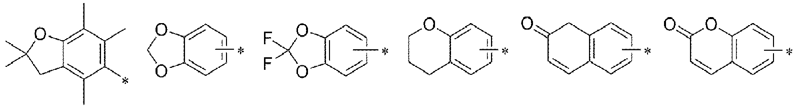

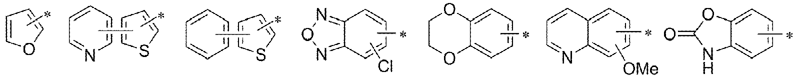

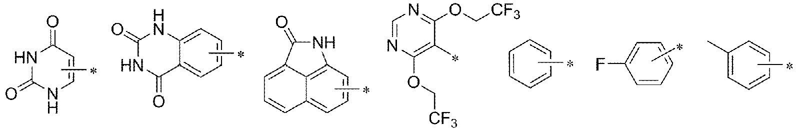

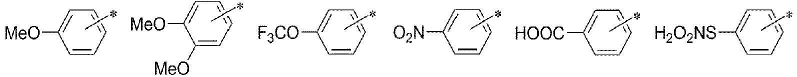

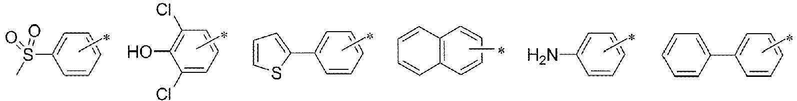

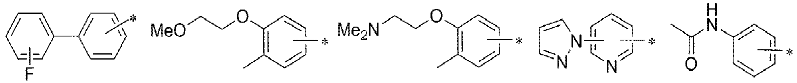

More specifically, R₄ may be bromo, fluoro, chloro, trifluoromethyl, cyano, methyl, ethyl, propyl, butyl, Ar₁, HET₁, C1-C4alkoxy, -O-Ar₁, -O-HET₁, trifluoromethoxy, hydroxy, amino, aminomethyl, aminoethyl, or aminopropyl; Ar₁ may be phenyl, biphenyl, or naphthyl; HET₁ may be pyrrolyl, furyl, thienyl, pyrazolyl, isoxazolyl, isothiazolyl, imidazolyl, oxazolyl, thiazolyl, triazolyl, oxadiazolyl, thiadiazolyl, pyridinyl, pyridazinyl, pyrimidyl, pyrazinyl, triazinyl, quinolinyl, isoquinolinyl, indolyl, benzofuranyl, benzothienyl, isoindolyl, indazolyl, benzoisoxazolyl, benzoisothiazolyl, benzimidazolyl, benzoxazolyl, benzothiazolyl, or benzotriazolyl; and d may be an integer of 0 to 3, and when d is an integer of 2 or more, R₄ may be the same as or different from each other.

In an exemplary embodiment, the compound of Chemical Formula 1 may be represented by the following Chemical formula 26: 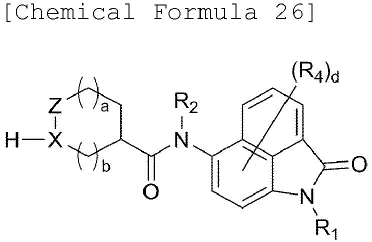 wherein R₁, R₂, X, Z, R₄, a, b, and d are as defined in Chemical Formula 1.

In the compound of Chemical formula 26 according to an exemplary embodiment, X may be N, a may be an integer of 2, and b may be an integer of 0.

In the compound of Chemical formula 26 according to an exemplary embodiment, X may be N, a may be an integer of 1, and b may be an integer of 1.

In the compound of Chemical formula 26 according to an exemplary embodiment, X may be N, a may be an integer of 0, and b may be an integer of 2.

The compound of Chemical Formula 26 may be specifically a compound represented by the following Chemical Formula 27, 28, or 29: 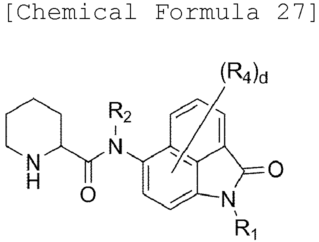 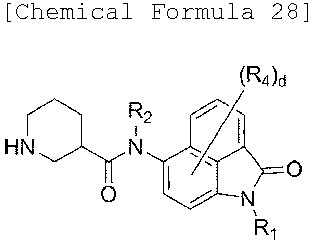 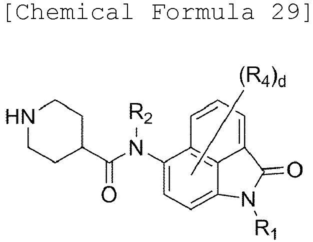 wherein R₁, R₂, R₄, and d are as defined in Chemical Formula 1.

The compound according to an exemplary embodiment may be more preferably selected from the following group of compounds, but is not limited thereto:

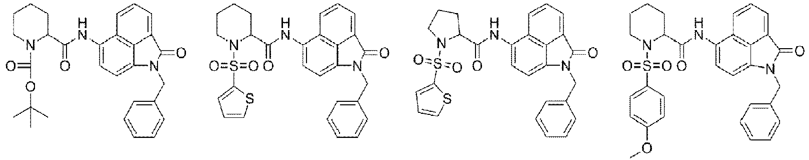

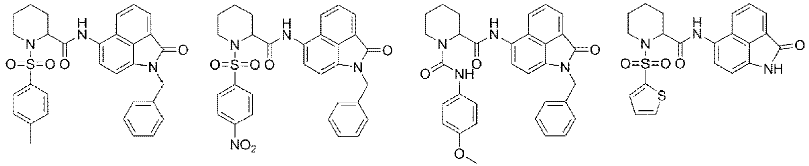

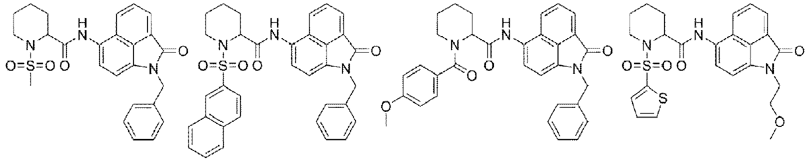

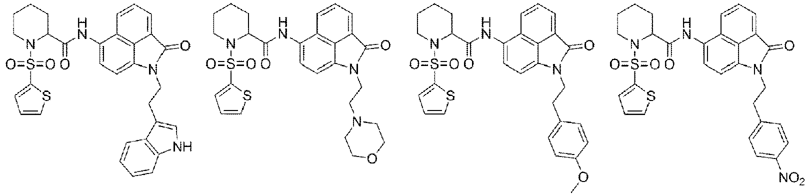

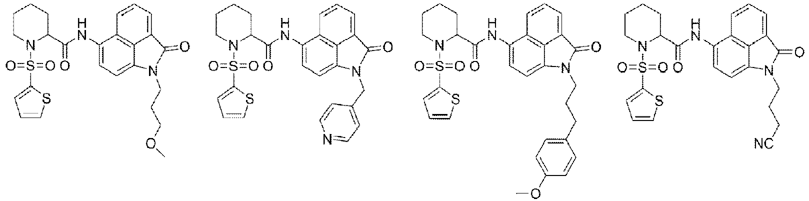

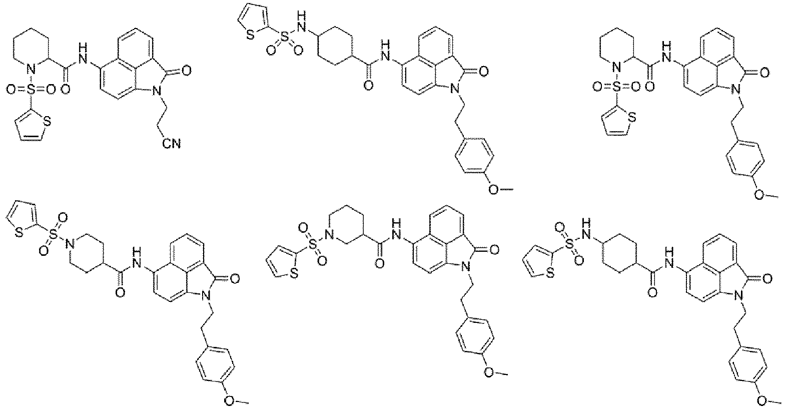

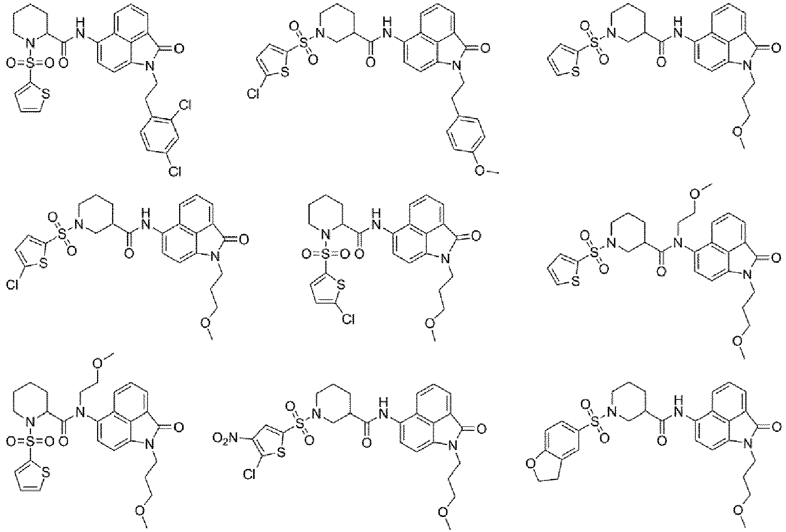

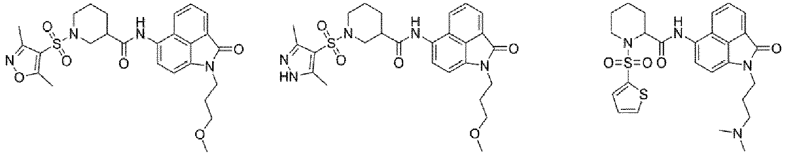

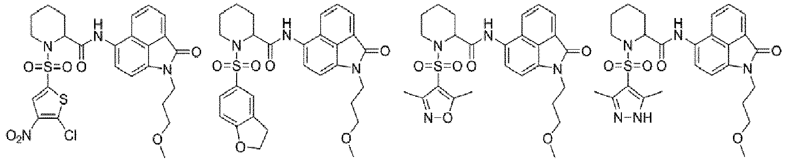

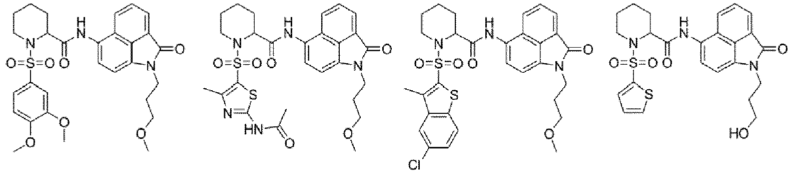

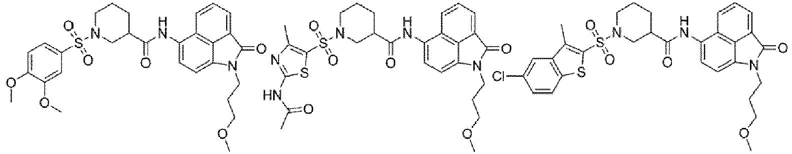

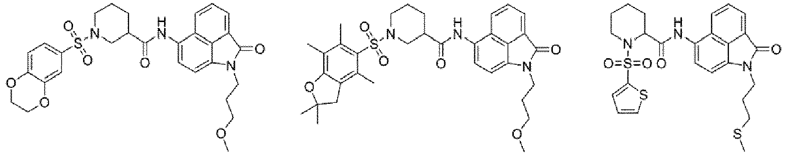

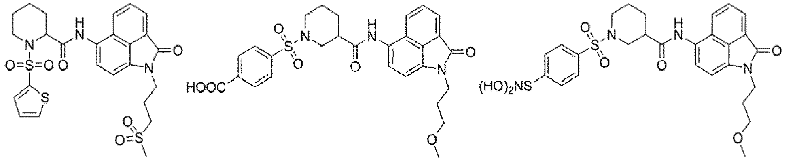

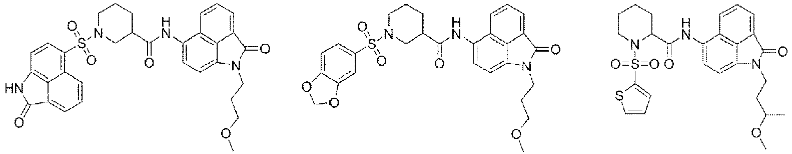

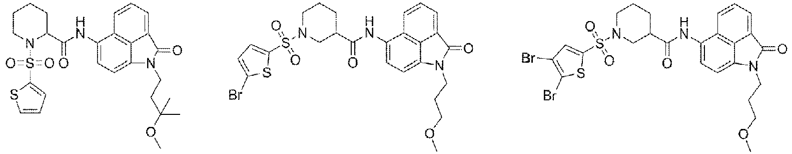

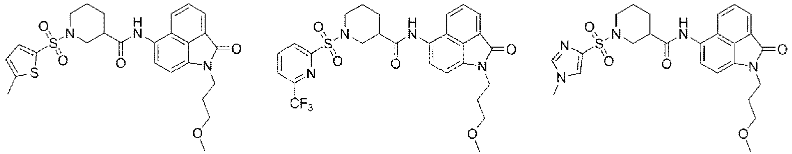

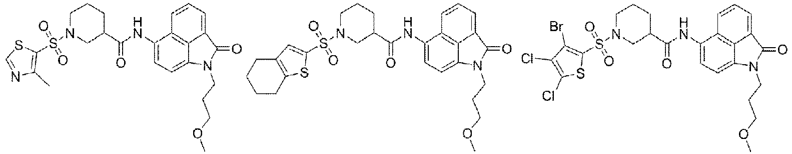

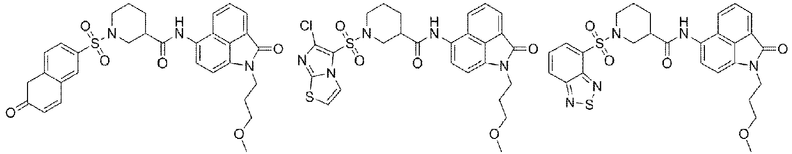

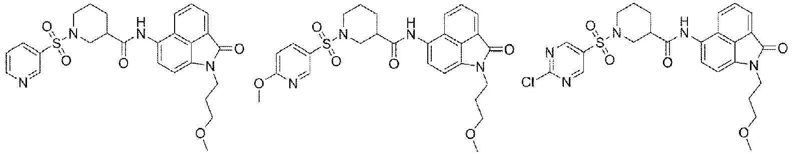

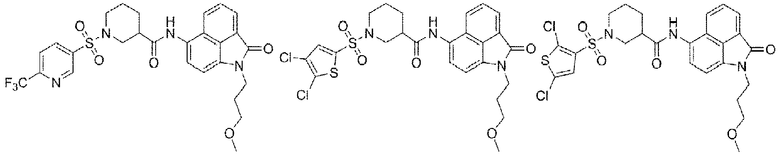

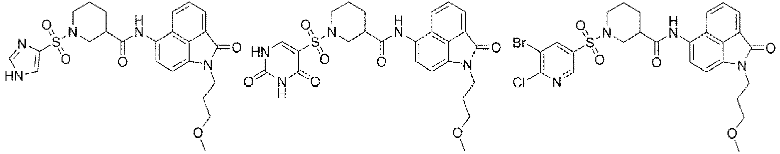

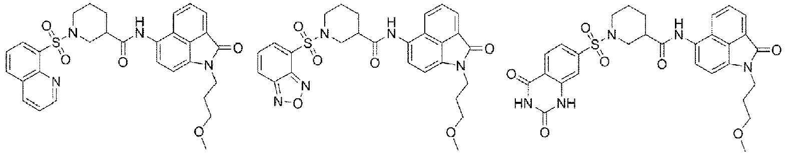

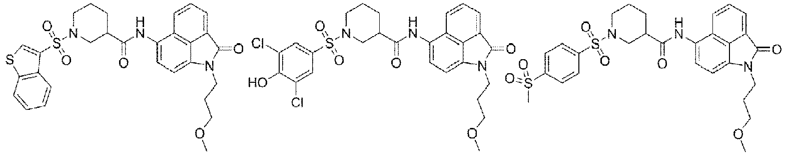

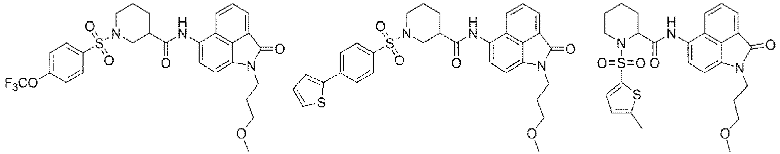

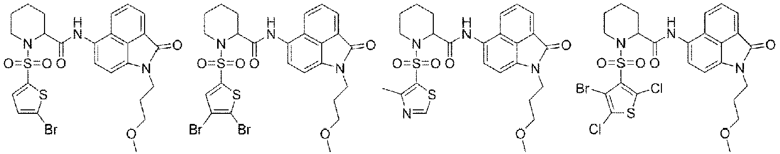

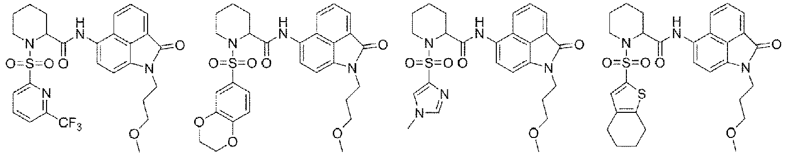

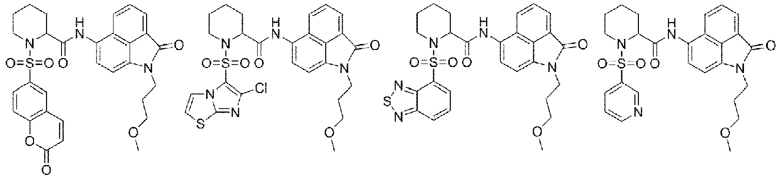

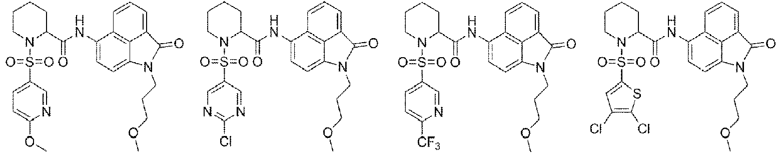

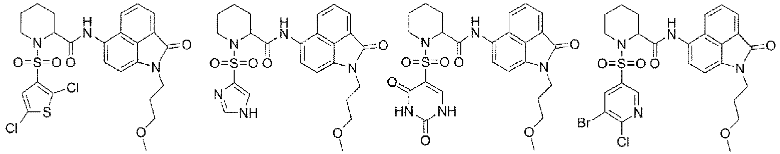

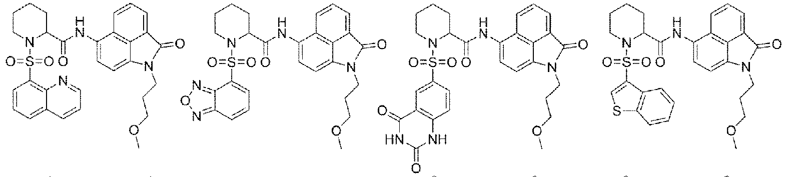

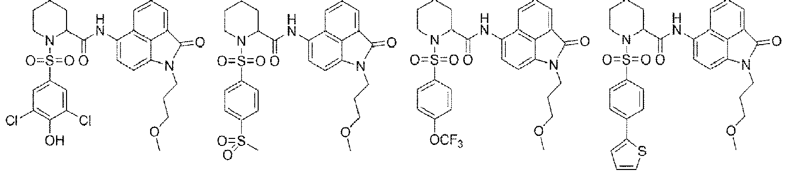

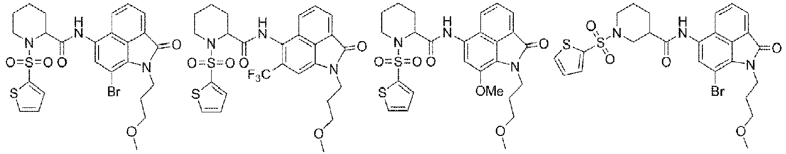

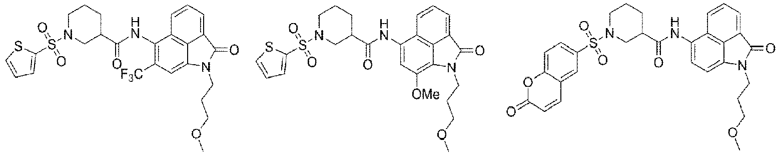

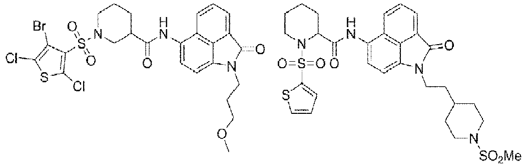

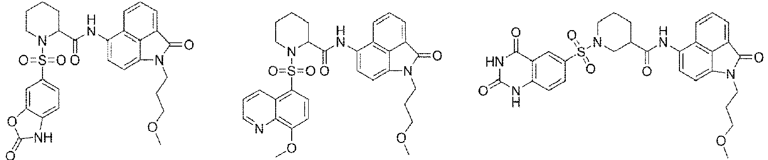

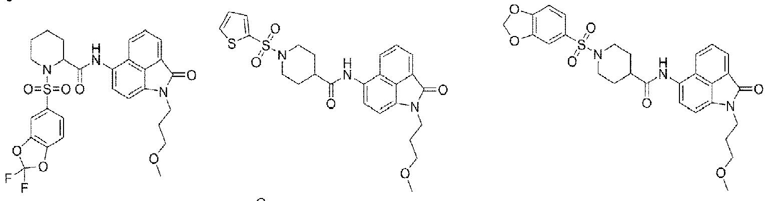

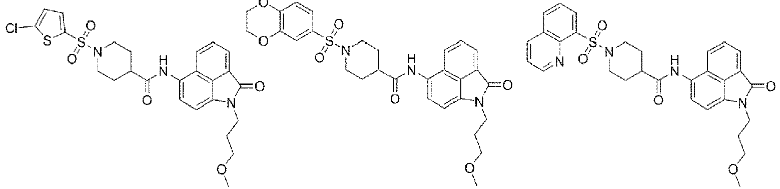

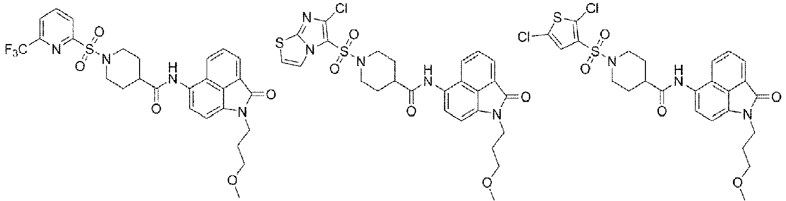

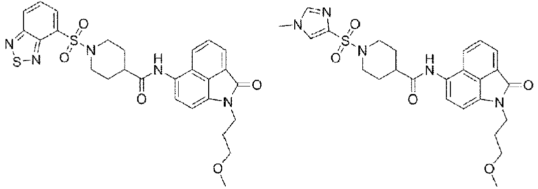

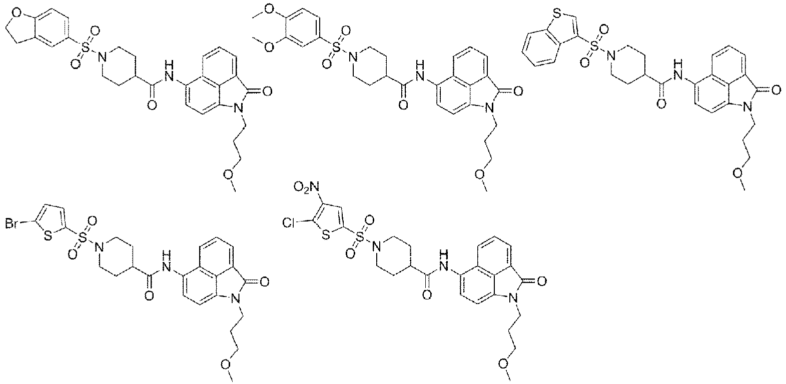

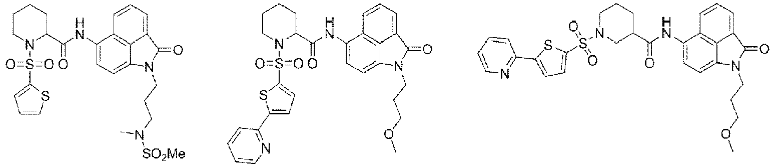

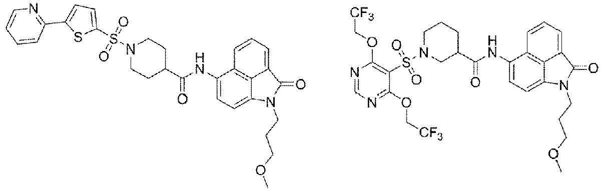

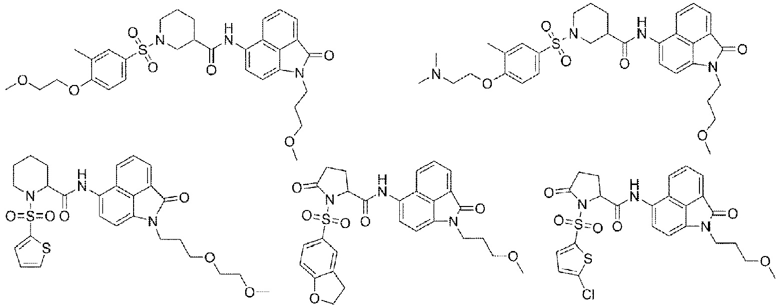

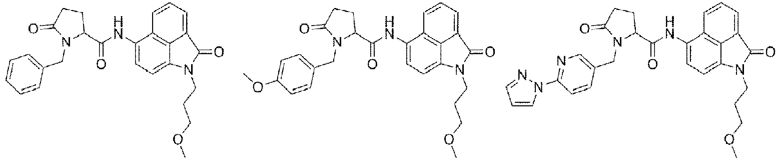

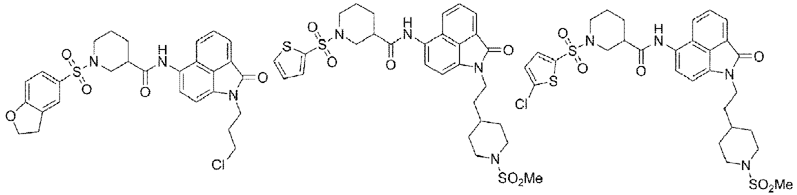

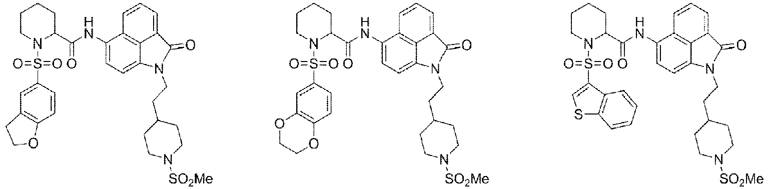

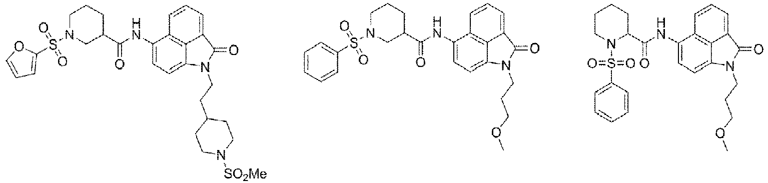

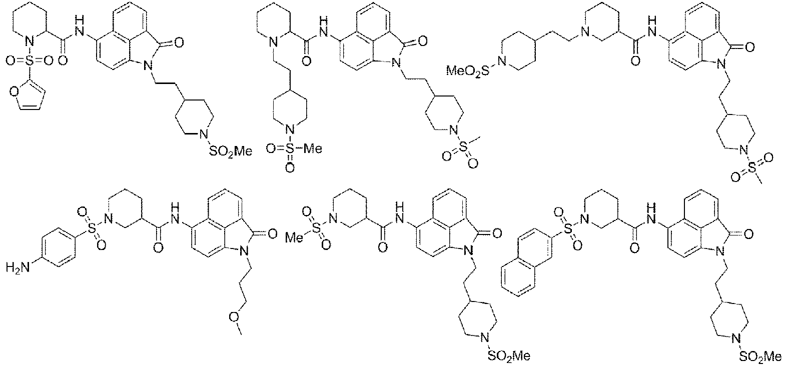

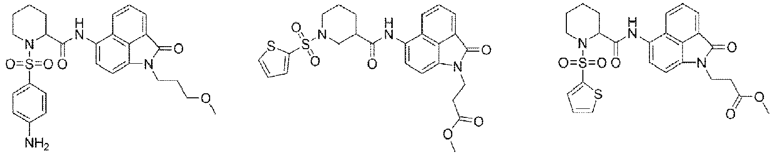

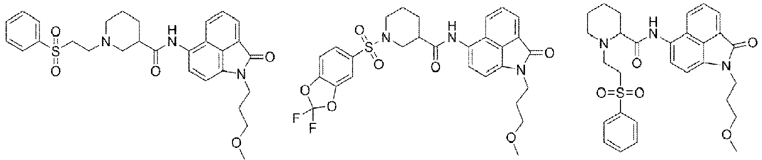

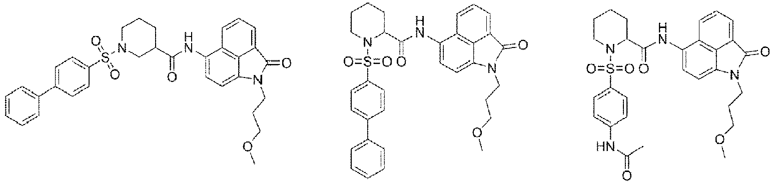

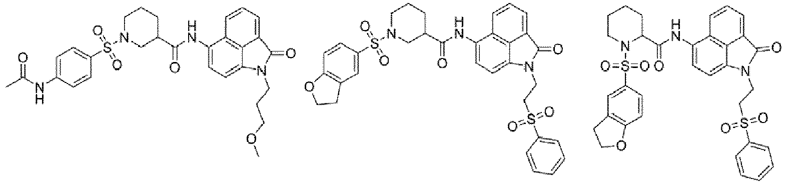

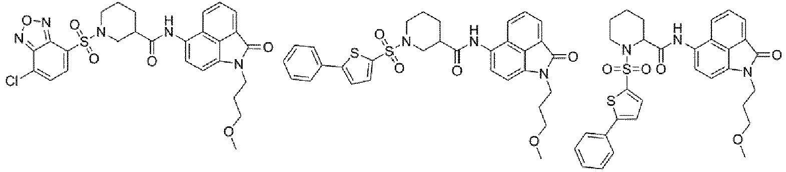

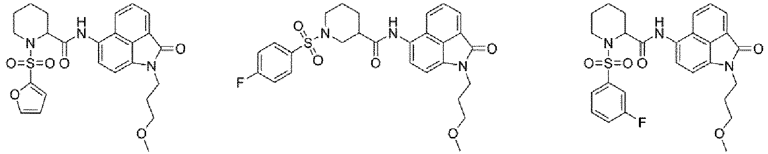

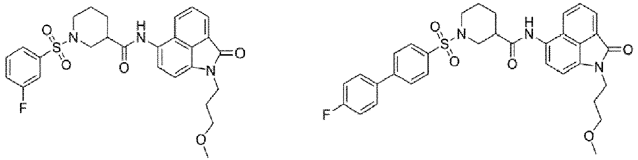

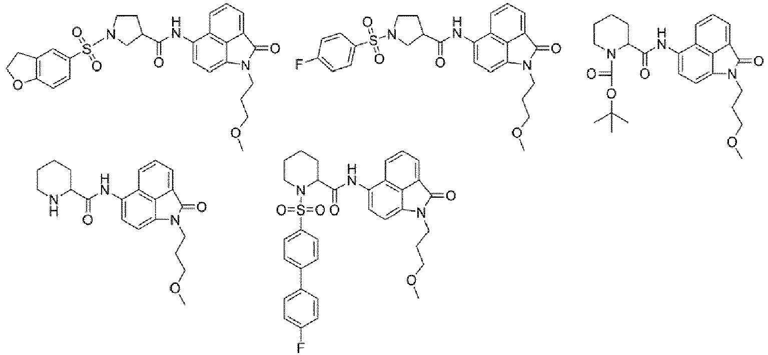

The compound of Chemical Formula 1 may be prepared by various known methods depending on the kinds of substituents, and it will be apparent to a person skilled in the art that the preparation may be performed using the method known in the art or by appropriately modifying the method, of course.

Since the compound of the present invention may be used in the form of a prodrug, a hydrate, a solvent, and a pharmaceutically acceptable salt for promoting in-vivo absorption and increasing solubility, not only the compound of Chemical Formula 1 but also a form of the prodrug thereof, the hydrate thereof, the solvate thereof, or the salt thereof is included in the scope of the present invention.

The compound of the present invention may be used in the form of a pharmaceutically acceptable salt, and the pharmaceutically acceptable salt is a salt prepared by a common method in the art, and the preparation method thereof is known to a person skilled in the art. Specifically, the pharmaceutically acceptable salt includes salts derived from the following pharmacologically or physiologically acceptable free acids and bases, but is not limited thereto.

The pharmaceutically acceptable salt of the compound of the present invention refers to a salt prepared according to a method common in the art, and the preparation method as such is known to a person skilled in the art. Specifically, the pharmaceutically acceptable salt includes salts derived from the following pharmacologically or physiologically acceptable inorganic acids and organic acids and bases, but is not limited thereto.

An acid adduct salt is prepared by a common method, for example, by dissolving the compound of the present invention in an excessive amount of an acid aqueous solution and precipitating the salt using a water-compatible organic solvent, for example, methanol, ethanol, acetone, or acetonitrile. The compound and an acid or alcohol (for example, glycol monomethyl ether) in water at the same molar amounts are heated, and then the mixture may be dried by evaporation or the precipitated salt may be suction filtered. Here, as the free acid, an organic acid and an inorganic acid may be used, and as the inorganic acid, hydrochloric acid, phosphoric acid, sulfuric acid, nitric acid, stannic acid, and the like may be used and as the organic acid, methane sulfonic acid, p-toluene sulfonic acid, acetic acid, trifluoroacetic acid, maleic acid, succinic acid, oxalic acid, benzoic acid, tartaric acid, fumaric acid, maldelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like may be used, but the present invention is not limited thereto.

In addition, a pharmaceutically acceptable metal salt may be prepared using a base. An alkali metal salt or an alkali earth metal salt is obtained by, for example, dissolving the compound of the present invention in an excess amount of an alkali metal hydroxide or an alkali earth metal hydroxide solution, filtering out an undissolved compound salt of the present invention, and evaporating and drying the filtrate. Here, it is pharmaceutically appropriate to prepare, in particular, a sodium, potassium, or calcium salt as the metal salt, but the present invention is not limited thereto. In addition, a silver salt corresponding thereto may be obtained by reacting an alkali metal or an alkali earth metal salt with a suitable silver salt (for example, silver nitrate).

The pharmaceutically acceptable salt of the compound of the present invention includes, a salt of an acid or basic group which may be present in the compound of Chemical Formula 1, unless otherwise stated. For example, the pharmaceutically acceptable salt may include a sodium, calcium, and potassium salts of a hydroxyl group and the like, and other pharmaceutically acceptable salt of an amino group may include hydrobromides, sulfates, hydrogen sulfate salts, phosphates, hydrogen phosphates, dihydrogen phosphates, acetates, succinates, citrates, tartrates, lactates, mandelates, methanesulfonate (mesylate), p-toluenesulfonate (tosylate), and the like, and may be prepared by a preparation method of salts known in the art.

The hydrate of the compound of the present invention refers to the compound of the present invention and the salt thereof including a stoichiometric or non-stoichiometric amount of water bonded by non-covalent intermolecular force.

The solvate of the compound of the present invention refers to the compound of the present invention and the salt thereof including a stoichiometric or non-stoichiometric amount of a solvent bonded by non-covalent intermolecular force. Preferred solvents therefor include volatile and non-toxic solvents.

The compound of the present invention may be administered in the form of a prodrug which is decomposed in a human or animal body to provide the compound of the present invention as an effective component. The prodrug may be used for modifying and/or improving a physical and/or pharmacokinetic profile of a parent compound, and may be formed when the parent compound contains an appropriate group or substituent which may be derived to form the prodrug.

In the case in which a certain compound (prodrug) is decomposed in the body to produce the compound of the present invention or the salt thereof, the compound is also included in the scope of the present invention. Unless otherwise used and indicated in the present specification, the term "prodrug" refers to the compound of the present invention which may be hydrolyzed, oxidized, and may undergo another reaction under biological conditions (ex vivo or in vivo) for supplying an active compound, in particular, the compound of the present invention. Examples of the prodrug include compounds which include a bio-hydrolyzable part such as bio-hydrolyzable amides, bio-hydrolyzable esters, bio-hydrolyzable carbamates, bio-hydrolyzable carbonates, bio-hydrolyzable ureides, and bio-hydrolyzable phosphate analogs and are bio-hydrolyzed to produce the compound of the present invention, but are not limited to specific embodiments. Preferably, the prodrug of a compound having a carboxyl functional group is a lower alkyl ester of a carboxylic acid. A carboxylic ester is usually formed by esterifying a part of a carboxylic acid present in a molecule. The prodrug may be easily prepared using a known method such as those described in Burger's Medicinal Chemistry and Drug Discovery 6thed. (Donald J. Abrahamed., 2001, Wiley) and Design and Application of Prodrugs (H. Bundgaard ed., 1985, Harwood Academic Publishers Gmfh).

In addition, the present invention provides a pharmaceutical composition for preventing or treating cancer, including the compound of Chemical Formula 1, the prodrug thereof, the hydrate thereof, the solvate thereof, or the pharmaceutically acceptable salt thereof as an effective component.

Here, the compound of Chemical Formula 1, preferably Chemical Formula 2, the prodrug thereof, the hydrate thereof, the solvate thereof, or the pharmaceutically acceptable salt thereof may prevent or treat cancer by activity of inhibiting YAP-TEAD binding in the Hippo pathway. That is, the compound of Chemical Formula 1, preferably Chemical Formula 2 may bind to TEAD competitively with YAP to inhibit a YAP-TEAD interaction to activate the Hippo pathway, thereby efficiently controlling growth of cancer.

In the pharmaceutical composition according to an exemplary embodiment, the cancer may be lung cancer, colorectal cancer, colon cancer, rectal cancer, breast cancer, prostate cancer, bladder cancer, blood cancer, leukemia, myelogenous leukemia, lymphoma, cervical carcinoma, osteosarcoma, glioblastoma, melanoma, pancreatic cancer, gastric cancer, liver cancer, kidney cancer, gallbladder cancer, biliary tract cancer, esophageal cancer, ovarian cancer, or neuroblastoma, preferably colorectal cancer, colon cancer, or rectal cancer.

In an exemplary embodiment, the pharmaceutical composition may further include a usual non-toxic pharmaceutically acceptable carrier, excipient, or diluent in addition to the effective component to be formulated into a preparation common in the pharmaceutical field, that is, a preparation for oral administration or parenteral administration.

The pharmaceutically acceptable carrier, excipient, or diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, or the like.

The pharmaceutical composition of the present invention may be formulated in various forms such as oral formulations such as powders, granules, tablets, capsules, suspensions, emulsions, syrups, and aerosols, and injections of a sterile injection solution for use, and may be administrated by oral administration or by various routes including intravenous, intraperitoneal, subcutaneous, rectal, and topical administration.

In addition, the pharmaceutical composition of the present invention may further include a filler, an anticoagulant, a lubricant, a wetting agent, a fragrance, an emulsifier, a preservative, and the like.

A solid preparation for oral administration includes tablets, pills, powders, granules, capsules, and the like, and the solid preparation is formulated by mixing the composition with at least one excipient, for example, starch, calcium carbonate, sucrose, lactose, gelatin, and the like. In addition, a lubricant such as talc and magnesium stearate may be used in addition to a simple excipient.

An example of a liquid preparation for oral administration may include suspensions, oral liquids, emulsions, syrups, and the like, and include various excipients, for example, a wetting agent, a sweetener, an aromatic, a preservative, and the like, in addition to water and liquid paraffin which are a commonly used simple diluent.

The preparation for parenteral administration includes a sterile aqueous solution, a non-aqueous solvent, a suspension, an emulsion, a freeze-dried preparation, a suppository, and the like. As a non-aqueous solvent and a suspension solvent, propylene glycol, polyethylene glycol, a vegetable oil such as an olive oil, injectable ester such as ethyl oleate, and the like may be used. As a base of the suppository, witepsol, microgol, tween 61, cacao butter, laurin butter, glycerogelatin, and the like may be used. Meanwhile, an injection may further include a conventional additive such as solubilizers, tonicity agents, suspending agents, emulsifiers, stabilizers, and preservatives.

The pharmaceutical composition of the present invention may be sterilized, or further include an adjuvant such as preservatives, stabilizers, thickeners, hydrating agents, or emulsifying accelerators, a salt for regulating osmotic pressure, and/or a buffer, and other therapeutically useful materials, and may be formulated according to a conventional method such as dissolution, dispersion, gelation.

The pharmaceutical composition of the present invention may be administered in a pharmaceutically effective amount, as an individual therapeutic agent or in combination with other therapeutic agents, sequentially or simultaneously with a conventional therapeutic agent, or in a single or multiple. It is important to administer an amount for obtaining a maximum effect with a minimum amount without any side effect, considering all of the above elements, and this will be easily determined by a person skilled in the art.

Specifically, the effective amount of the compound in the composition of the present invention may be varied with the age, gender and weight of a patent, and may be administered at 1 to 100 mg, preferably 5 to 60 mg per 1kg of a body weight, every day or every other day, or administered in a divided amount 1 to 3 times a day. However, since the amount may be increased or decreased depending on the administration route, severity of the disease, gender, weight, age, and the like, the administration amount in no way limits the scope of the present invention.

In addition, the present invention provides a method of preventing or treating cancer diseases including administrating the pharmaceutical composition to an individual having a cancer disease or a risk of a cancer disease.

The pharmaceutical composition of the present invention is administered in a pharmaceutically effective amount.

An effective dose level may be easily determined by a person skilled in the art depending on elements including the gender, age, weight, and health condition of a patient, type of disease, severity, drug activity, drug sensitivity, administration method, administration time, administration route, excretion rate, therapeutic period, and drugs used in combination or at the same time, and other elements which are well known in the medical field. The pharmaceutical composition of the present invention may be administered as an individual therapeutic agent, or in combination with other therapeutic agents, sequentially or simultaneously with the conventional therapeutic agent, and in a single dose or multiple doses. It is important to administer an amount for obtaining a maximum effect with a minimum amount without any side effect, considering all of the above elements, and this will be easily determined by a person skilled in the art.

The administration route and the administration method of administering the pharmaceutical composition of the present invention is not particularly limited, and as long as a composition including the composition reaches a corresponding target area, any administration route and administration method may be followed. Specifically, the composition may be administered through oral or parenteral various routes, and a non-limiting example of the administration route include oral, rectal, topical, intravenous, intraperitoneal, intramuscular, intraarterial, transdermal, nasal, or inhalation administration, and the like.

In addition, the method of preventing or treating cancer of the present invention may be used in combination with methods using surgery, radiation therapy, hormone therapy, chemotherapy, and a biological response modifier.

In addition, the present invention provides a YAP/TAZ-TEAD inhibitor composition including the compound represented by Chemical Formula 1, the prodrug thereof, the hydrate thereof, the solvate thereof, or the pharmaceutically acceptable salt thereof at an effective amount as an active component, as an effective component.

In addition, the present invention provides a health functional food composition for preventing or improving cancer, including the compound represented by Chemical Formula 1, the prodrug thereof, the hydrate thereof, the solvate thereof, or the pharmaceutically acceptable salt thereof at an effective amount as an active component, as an effective component.

The health functional food composition may be provided in the form of powders, granules, tablets, capsules, syrups, or beverages, and the health functional food is used with other food or food additives in addition to the compound of Chemical Formula 1 as an effective component and may be appropriately used according to a common method. A mixed amount of the effective component may be appropriately determined depending on the purpose of use, for example, prevention, health, or therapeutic treatment.

The compound of Chemical Formula 1 included in the health functional food composition may be used according to the effective dose of the pharmaceutical composition, but in the case of long-term ingestion for health and hygiene or for health control, the effective dose may be less than the above range, and since the effective component has no problem in terms of safety, it may be used more than the amount in the above range, of course.

The health functional food composition may be formulated in various formulations such as meat, sausage, bread, chocolate, candy, snacks, confectionery, pizza, ramen, other noodles, gum, dairy products including ice creams, various soups, beverages, tea, drinks, alcoholic beverages, and vitamin complexes.

Hereinafter, the present invention will be described in detail through the preferred Examples. However, the Examples are suggested for illustration of the present invention, and the scope of a right of the present invention is in no sense limited by the Example, and the scope of a right of the present invention is only defined by the scope of the appended claims described later.

**Preparation Example A**

A nitration reaction was performed using benzo[cd]indol-2(1H)-one, which is available from a reagent company, as a starting material to obtain an intermediate a-1, which was alkylated by N-alkylation to obtain an intermediate b-1-1, of which the nitro group was reduced to obtain an amine intermediate c-1-1, which was amide-coupled with a Boc-protected piperidine carboxylic acid compound d-1-1 to obtain an intermediate e-1-1. Thereafter, amine f-1-1 obtained after Boc-deprotection was sulfonylated with a sulfonyl chloride compound to obtain a final compound g-1-1. A general method of each reaction is specified below.

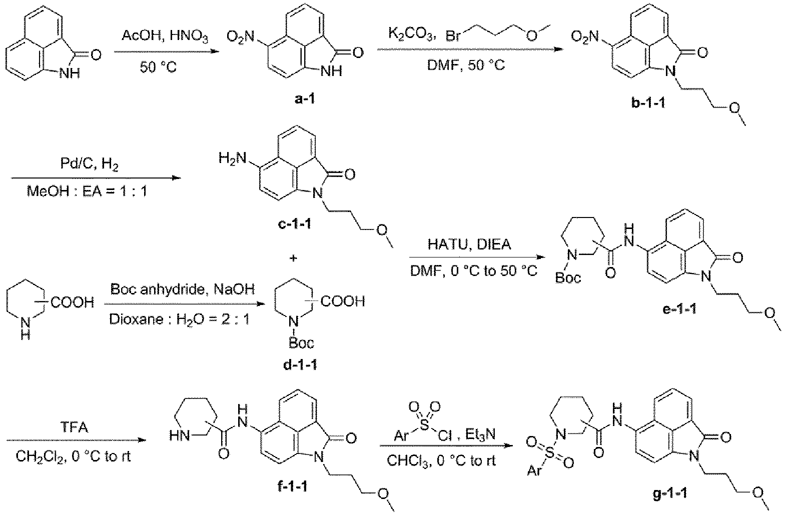

### Step 1: Synthesis of compound a-1

Benzo[cd]indol-2(1H)-one (10 g, 59.1 mmol) was dissolved in acetic acid (45 mL) and then stirred at 10°C. Nitric acid (60%, 5.88 mL, 76.8 mmol) was slowly added, and stirring was performed at 50°C for 24 hours. The reaction mixture was cooled down to room temperature, water was added, filtration was performed, and washing with water was performed to obtain an ochre solid compound a-1 (12.6 g, 100 %).
¹H NMR (300 MHz, DMSO-d₆) δ 11.41 (s, 1H), 8.89 (d, J = 8.4 Hz, 1H), 8.63 (d, J = 8.1 Hz, 1H), 8.18 (d, J = 6.9 Hz, 1H), 8.07 (dd, J = 8.5, 7.0 Hz, 1H), 7.13 (d, J = 8.0 Hz, 1H)

### Step 2: Synthesis of compound b-1-1

Compound a-1 (5.24 g, 24.5 mmol) prepared in Step 1 was dissolved in DMF (20 mL) and stirred at 0°C. K₂CO₃ (6.76 g, 48.9 mmol) and 1-bromo-3-methoxypropane (3.01 mL, 26.9 mmol) were added, the temperature was raised to 50°C, and stirring was performed for 24 hours. The reaction mixture was cooled down to room temperature, water was added, filtration was performed, and washing with water was performed to obtain an ochre solid compound b-1-1 (6.99 g, 100 %).

### Step 3: Synthesis of compound c-1-1

Compound b-1-1 (3.63 g, 12.7 mmol) prepared in Step 2 was dissolved in methanol/ethyl acetate (1/1 v/v, 40 mL), palladium 10% on carbon (363 mg) was added, and stirring was performed for 24 hours under a hydrogen gas. The reaction mixture was filtrated using celite and concentrated under reduced pressure to obtain a red solid compound c-1-1 (90%-100%).

### Step 4: Synthesis of compound d-1-1

Piperidine carboxylic acid (803 mg, 6.22 mmol) was dissolved in dioxane/water (2/1 v/v, 15 mL), NaOH (274 mg, 6.84 mmol) was added with stirring, and then stirring was performed at 0°C. Boc anhydride (1.57 mL, 6.84 mmol) was added, stirring was performed at room temperature for 24 hours, concentration under reduced pressure was performed to leave the solvent a little, and cooling to 0°C was performed. Acidification with 1M HCl was performed to adjust pH to 2 and extraction with ethyl acetate (10 mL×3) was performed. The obtained organic layer was dried with MgSO₄ and concentration under reduced pressure was performed to obtain a white solid compound d-1-1 (1.42 g, 100%).

### Step 5: Synthesis of compound e-1-1

Compound d-1-1 (111 mg, 0.482 mmol) prepared in Step 4 was dissolved in DMF (4 mL) and stirred at 0°C. DIEA (252 µL, 1.45 mmol) and HATU (367 mg, 0.965 mmol) were added and stirring was performed for 1 hour. Compound c-1-1 (124 mg, 0.482 mmol) prepared in Step 3 was dissolved in DMF (2 mL), the solution was slowly added to the reaction mixture, the temperature was raised to 60°C, and stirring was performed for 24 hours. The reaction was completed by adding water, extraction with ethyl acetate (5 mL×3) was performed, drying with anhydrous Na₂SO₄ was performed, and purification with flash chromatography was performed to obtain a yellow solid compound e-1-1 (40%-77%).

### Step 6: Synthesis of compound f-1-1

Compound e-1-1 (170 mg, 0.364 mmol) prepared in Step 5 was dissolved in CH₂Cl₂ (5 mL) and stirred at 0°C. Trifluoroacetic acid (278 µL, 3.64 mmol) was added and stirring was performed at room temperature. After completing the reaction, pH was basified to pH 8 with sat. NaHCO₃, extraction with CHCl₃ (10 mL×3) was performed, drying with anhydrous Na₂SO₄ was performed, and purification with flash chromatography was performed to obtain a yellow solid compound f-1-1 (78%-97%).

### Step 7: Synthesis of compound g -1-1

Compound f-1-1 (17.8 mg, 0.0484 mmol) prepared in Step 6 was dissolved in CHCl₃ (2 mL) and stirred at 0°C. Triethylamine (10 µL, 0.0727 mmol) and compound Ar-sulfonyl-Cl (1.2 eq) were added and stirring was performed at room temperature for 12 hours. The reaction mixture was concentrated under reduced pressure to remove the solvent and purification with flash chromatography was performed to obtain a yellow solid compound g-1-1 (20%-99%).

### Preparation Example B

Unlike Preparation Example A, piperidine acid was first sulfonylated to obtain an intermediate d-1-2, which was then amide-coupled with an amine intermediate c-1-2, thereby synthesizing a final compound g-1-2.

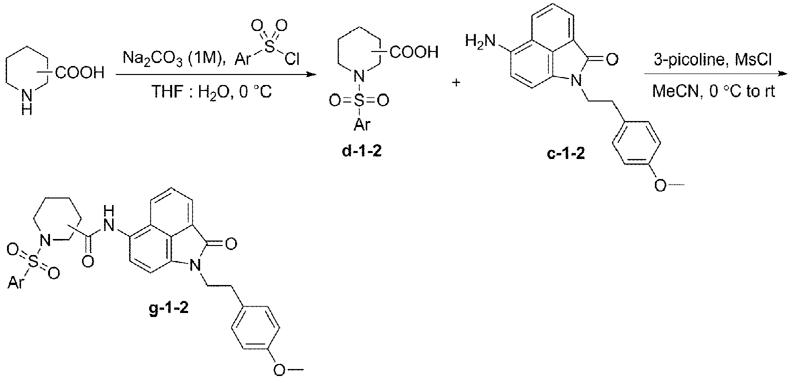

### Step 1: Synthesis of compound d-1-2

Piperidine carboxylic acid (1 g, 7.74 mmol) was dissolved in THF/H₂O=1/1 (v/v) (100 mL), Na₂CO₃ (17 mL, 17.0 mmol) was added with stirring at 0°C, stirring was performed for 30 minutes, a compound Ar-sulfonyl-Cl (1.2 eq) was added, and stirring was performed for 12 hours. The reaction mixture was extracted with ether (150 mL×2) to obtain a water layer, to which a KHSO₄ solid was added to adjust pH to 3, and then extraction was performed with ethyl acetate (15 mL×3). The obtained organic layer was dried with anhydrous Na₂SO₄ and then was concentrated under reduced pressure to obtain a compound d-1-2 (100%).

### Step 2: Synthesis of compound g-1-2

Compound d-1-2 (1 eq) prepared in Step 1 was dissolved in MeCN, 3-picoline (2.2 eq) and methanesulfonyl chloride (1.2 eq) were added with stirring at 0°C, and stirring was performed for 1 hour. Compound c-1-2 (1.2 eq) was dissolved in MeCN and slowly added thereto, and stirring was performed at room temperature for 24 hours. The reaction mixture was concentrated under reduced pressure to remove the solvent and then purification with flash chromatography was performed to obtain a yellow solid compound g-1-2 (20%-70%)

### Preparation Example C

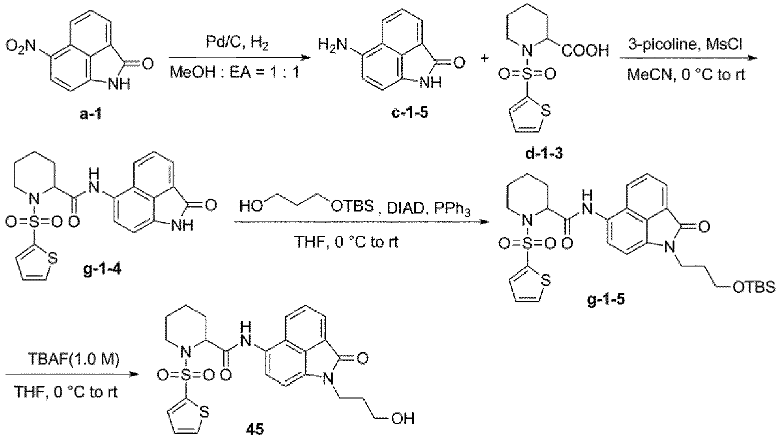

### Step 1: Synthesis of compound c-1-5

Compound c-1-5 was obtained in the same manner as in Step 3 of Preparation Example A, except that compound a-1 was used instead of compound b-1-1.

### Step 2: Synthesis of compound g-1-4

Compound g-1-4 was obtained in the same manner as in Step 2 of Preparation Example B, except that compound c-1-5 was used instead of compound c-1-2 and compound d-1-3 was used instead of compound d-1-2.

### Step 3: Synthesis of compound g-1-5

3-((*tert*-butyldimethylsilyl)oxy)-propanol (39 mg, 0.204 mmol) and PPh₃ (54 mg, 0.204 mmol) were dissolved in THF (1 mL) and stirred at 0°C. Compound g-1-4 (30 mg, 0.0679 mmol) was dissolved in THF (1 mL) and added thereto, and stirring was performed at the same temperature for 15 minutes. DIAD (40 µL, 0.204 mmol) was added, the temperature was raised to 70°C, and stirring was performed for 12 hours. The reaction mixture was concentrated under reduced pressure to remove the solvent and purification with flash chromatography was performed to obtain a yellow liquid compound g-1-5.

### Step 4: Synthesis of Compound 45

Compound g-1-5 (67 mg, 0.109 mmol) was dissolved in THF (2 mL), TBAF (1.0 M, 328 µL, 0.328 mmol) was added with stirring at 0°C, and stirring was performed at room temperature. The reaction mixture was concentrated under reduced pressure to remove the solvent and purification with flash chromatography was performed to obtain a yellow solid compound 45 (5.7 mg, 17 %).

### [Examples 1 to 169]

Compounds 1 to 169 of Table 1 were prepared according to the methods of Preparation Examples A to C, and the identification data of compounds 1 to 169 prepared is listed in the following Table 2.

**[Table 1]**

| No. | Structure | No. | Structure |
|---|---|---|---|
| 1 | 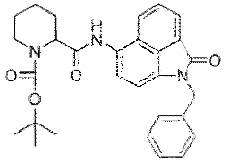 | 2 | 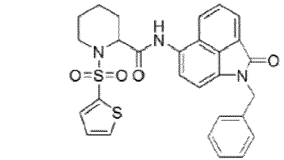 |
| 3 | 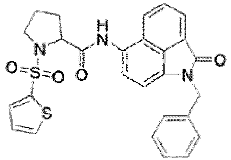 | 4 | 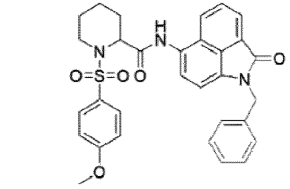 |
| 5 | 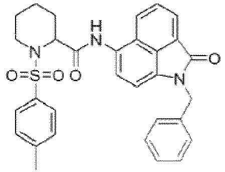 | 6 | 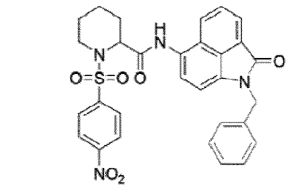 |
| 7 | 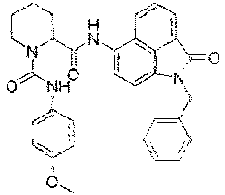 | 8 | 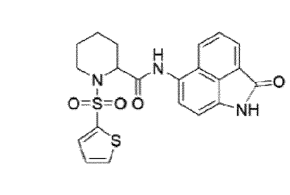 |
| 9 | 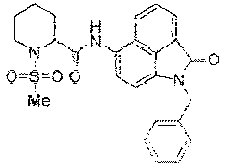 | 10 | 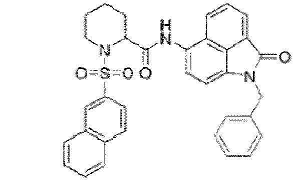 |
| 11 | 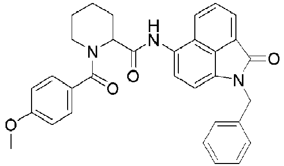 | 12 | 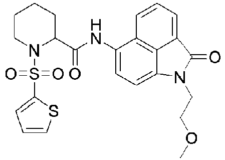 |
| 13 | 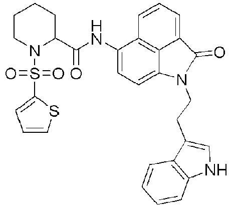 | 14 | 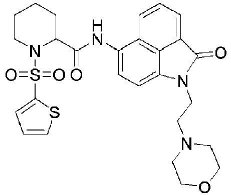 |
| 15 | 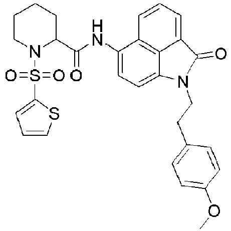 | 16 | 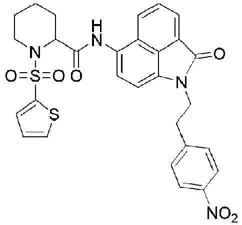 |
| 17 | 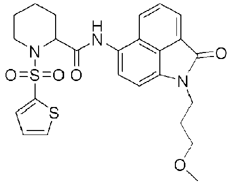 | 18 | 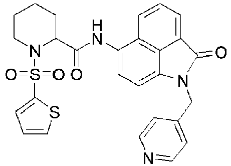 |
| 19 | 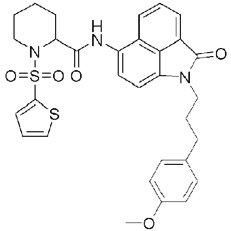 | 20 | 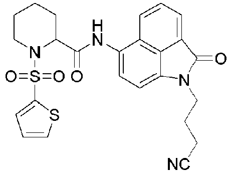 |
| 21 | 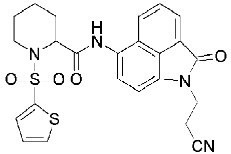 | 22 | 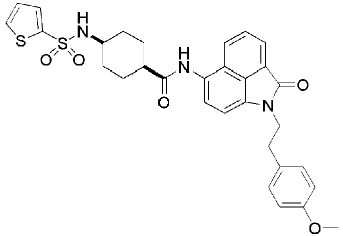 |
| 23 | 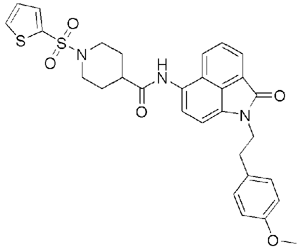 | 24 | 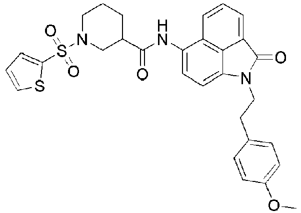 |
| 25 | 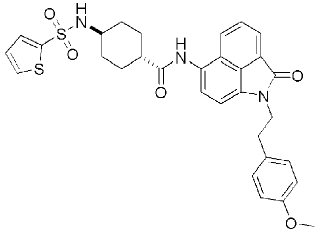 | 26 | 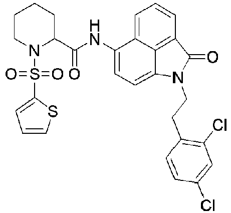 |
| 27 | 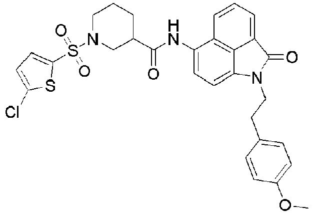 | 28 | 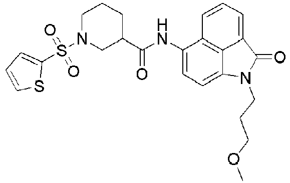 |
| 29 | 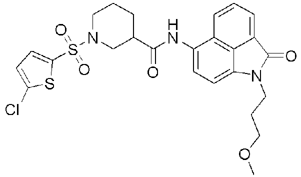 | 30 | 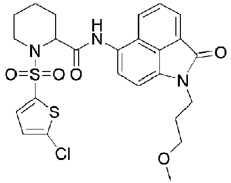 |
| 31 | 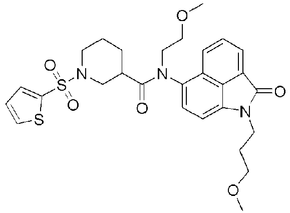 | 32 | 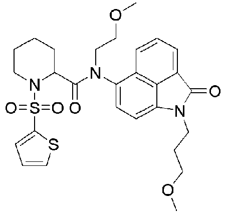 |
| 33 | 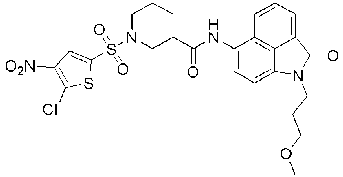 | 34 | 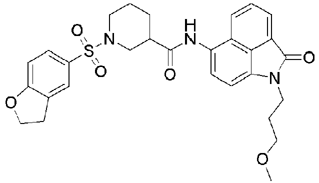 |
| 35 | 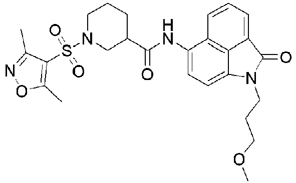 | 36 | 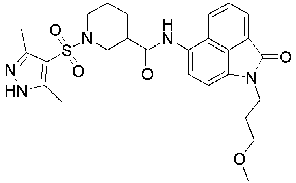 |
| 37 | 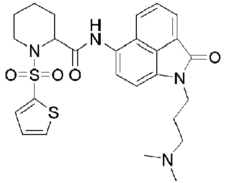 | 38 | 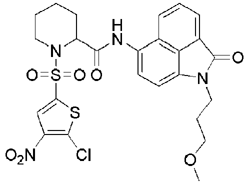 |
| 39 | 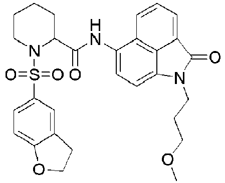 | 40 | 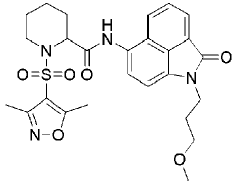 |
| 41 | 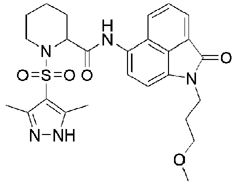 | 42 | 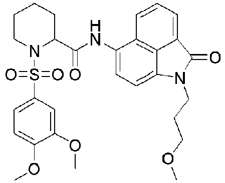 |
| 43 | 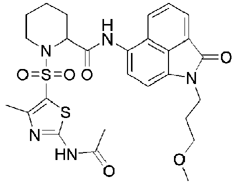 | 44 | 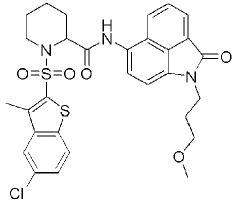 |
| 45 | 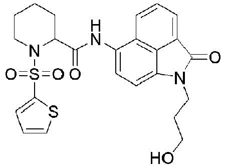 | 46 | 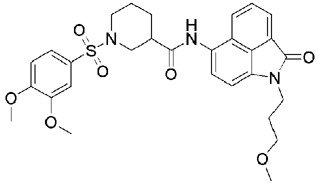 |
| 47 | 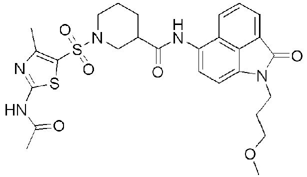 | 48 | 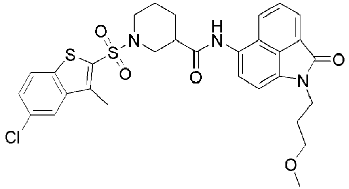 |
| 49 | 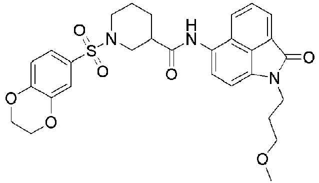 | 50 | 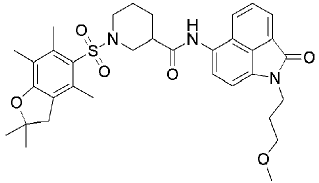 |
| 51 | 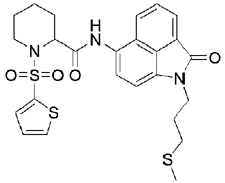 | 52 | 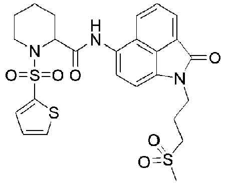 |
| 53 | 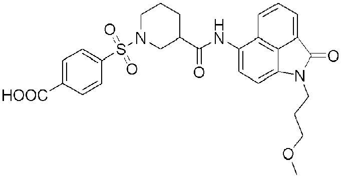 | 54 | 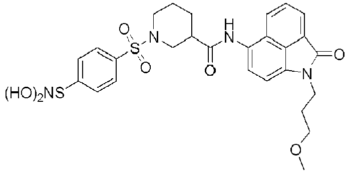 |
| 55 | 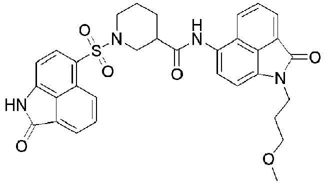 | 56 | 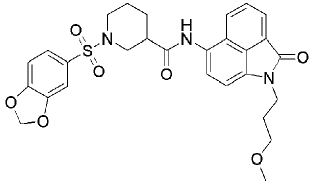 |
| 57 | 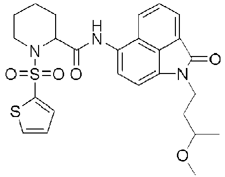 | 58 | 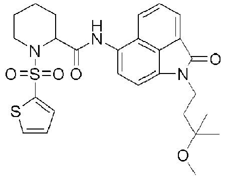 |
| 59 | 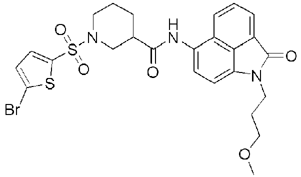 | 60 | 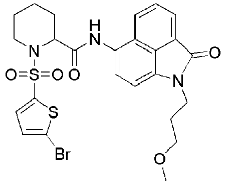 |
| 61 | 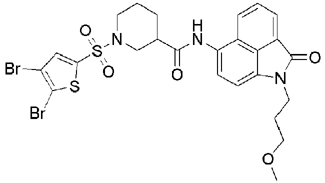 | 62 | 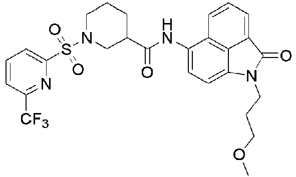 |
| 63 | 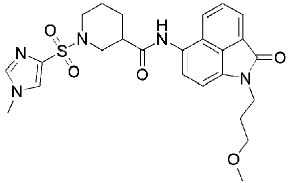 | 64 | 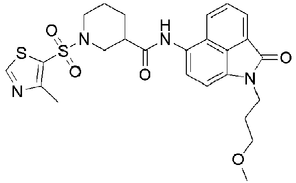 |
| 65 | 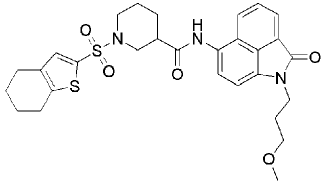 | 66 | 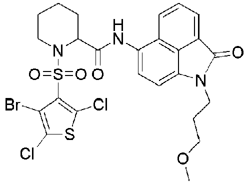 |
| 67 | 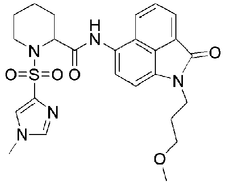 | 68 | 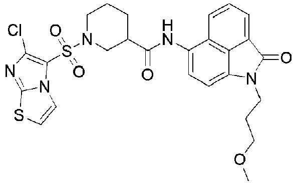 |
| 69 | 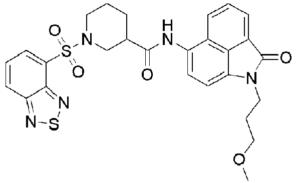 | 70 | 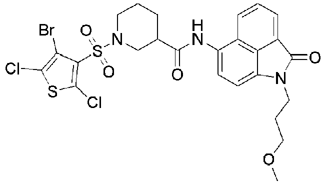 |
| 71 | 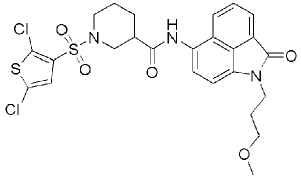 | 72 | 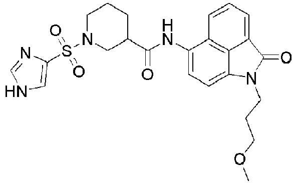 |
| 73 | 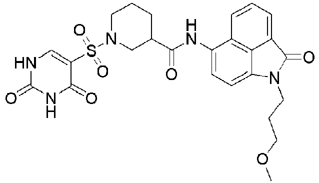 | 74 | 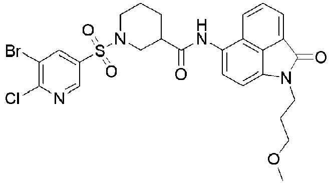 |
| 75 | 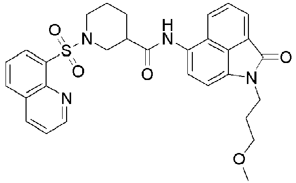 | 76 | 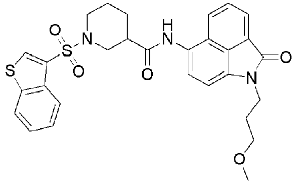 |
| 77 | 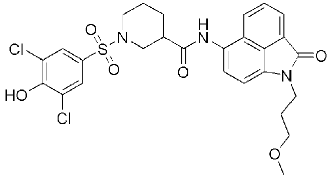 | 78 | 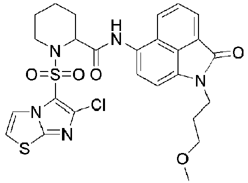 |
| 79 | 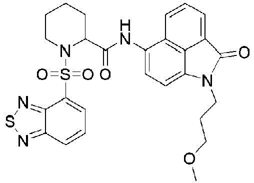 | 80 | 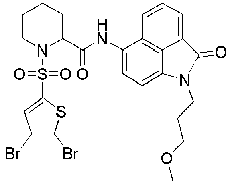 |
| 81 | 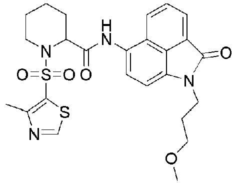 | 82 | 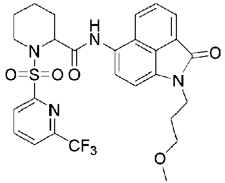 |
| 83 | 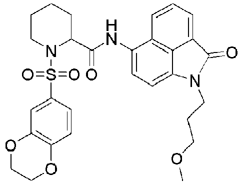 | 84 | 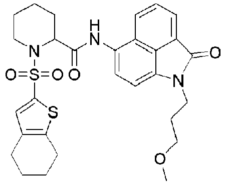 |
| 85 | 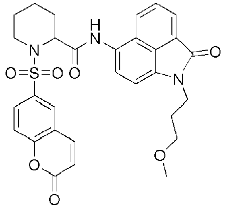 | 86 | 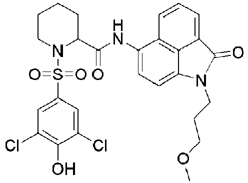 |
| 87 | 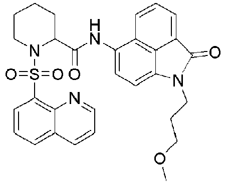 | 88 | 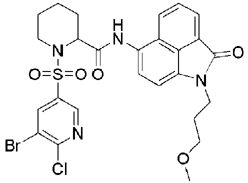 |
| 89 | 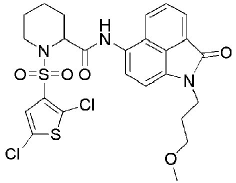 | 90 | 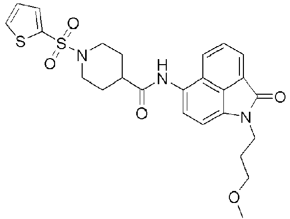 |
| 91 | 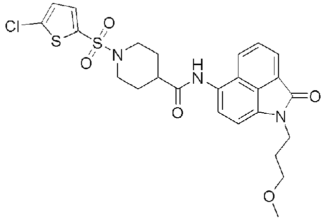 | 92 | 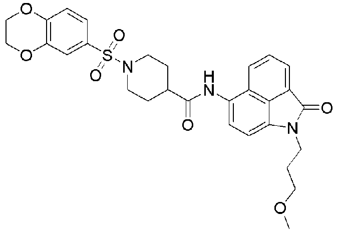 |
| 93 | 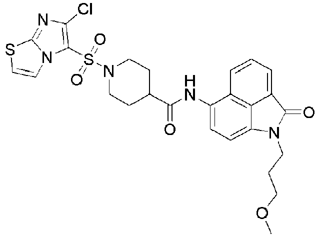 | 94 | 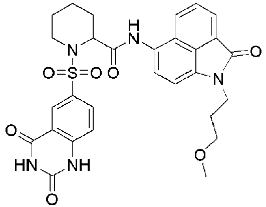 |
| 95 | 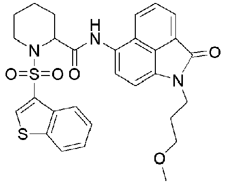 | 96 | 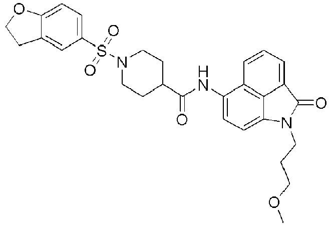 |
| 97 | 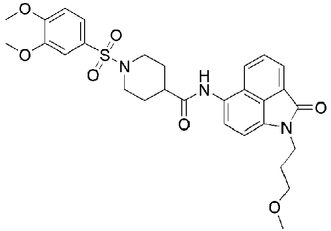 | 98 | 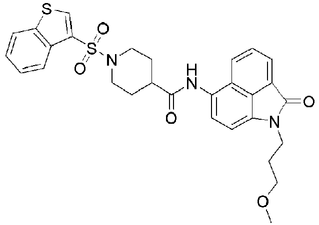 |
| 99 | 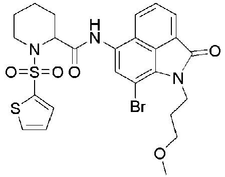 | 100 | 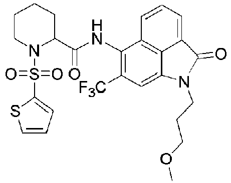 |
| 101 | 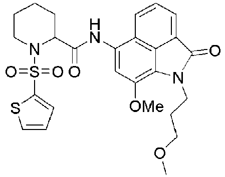 | 102 | 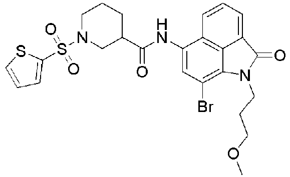 |
| 103 | 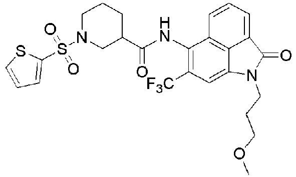 | 104 | 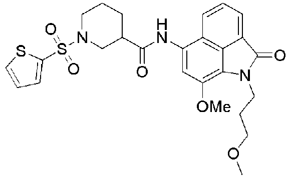 |
| 105 | 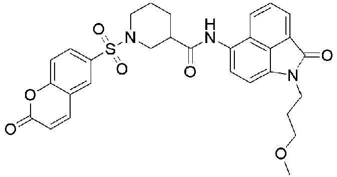 | 106 | 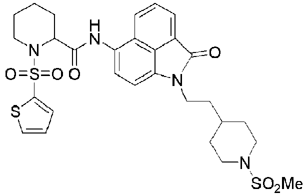 |
| 107 | 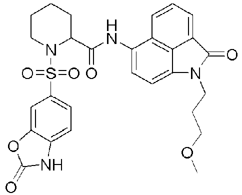 | 108 | 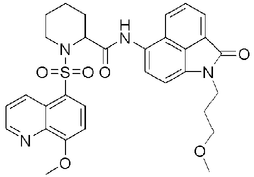 |
| 109 | 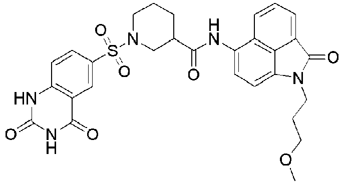 | 110 | 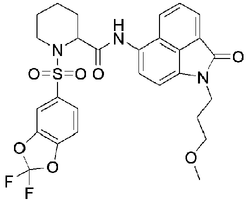 |
| 111 | 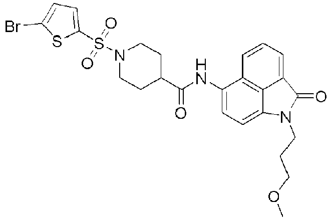 | 112 | 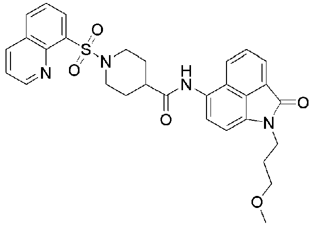 |
| 113 | 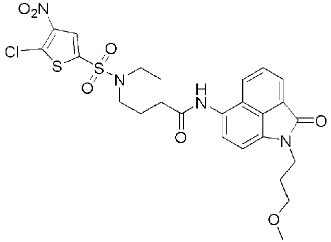 | 114 | 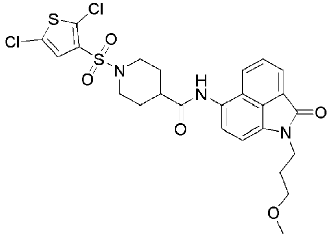 |
| 115 | 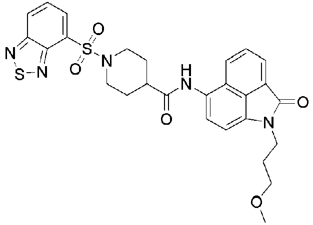 | 116 | 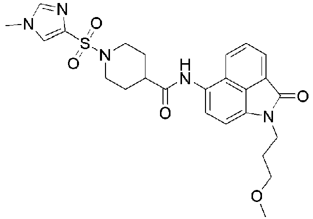 |
| 117 | 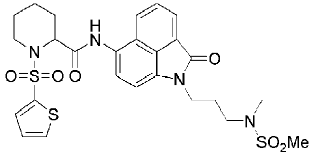 | 118 | 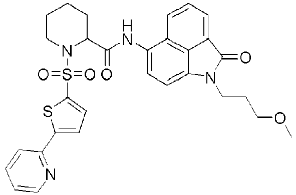 |
| 119 | 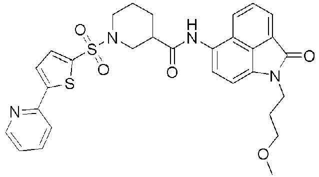 | 120 | 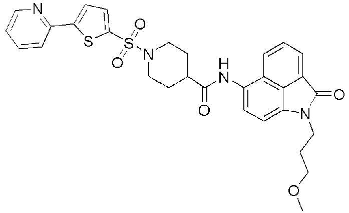 |
| 121 | 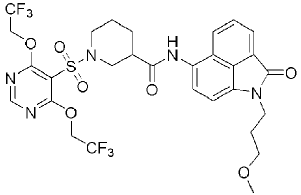 | 122 | 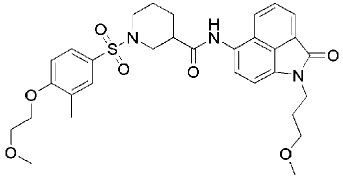 |
| 123 | 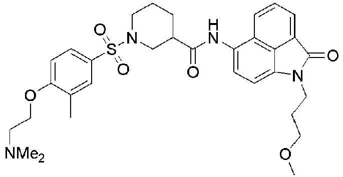 | 124 | 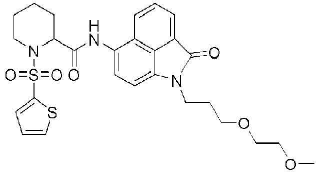 |
| 125 | 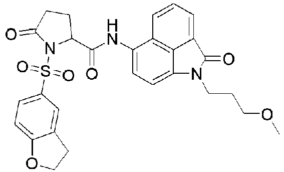 | 126 | 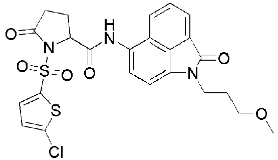 |
| 127 | 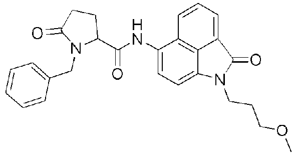 | 128 | 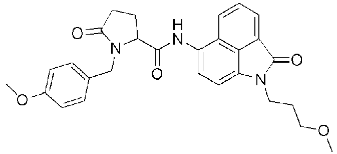 |
| 129 | 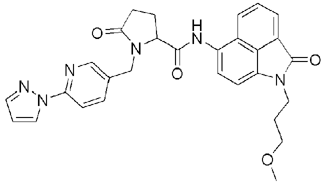 | 130 | 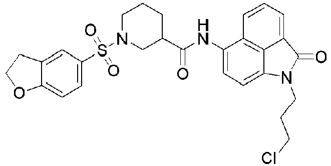 |
| 131 | 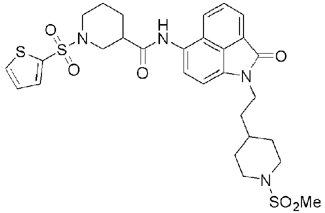 | 132 | 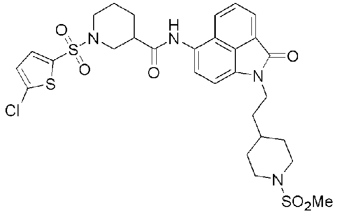 |
| 133 | 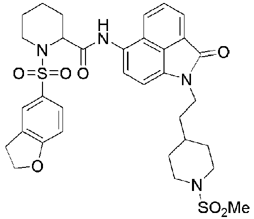 | 134 | 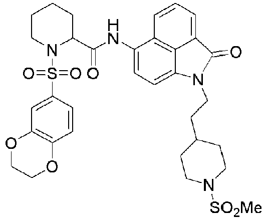 |
| 135 | 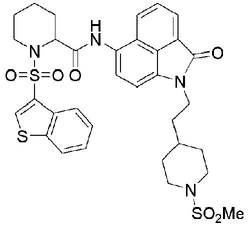 | 136 | 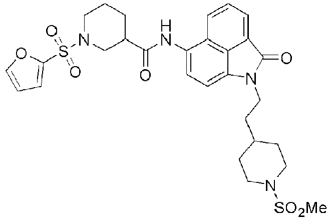 |
| 137 | 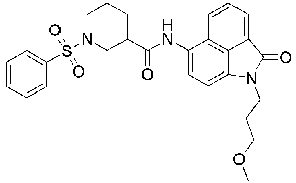 | 138 | 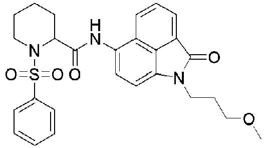 |
| 139 | 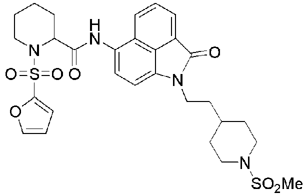 | 140 | 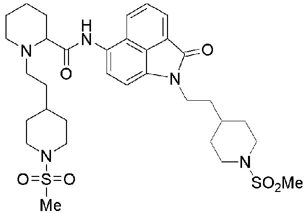 |
| 141 | 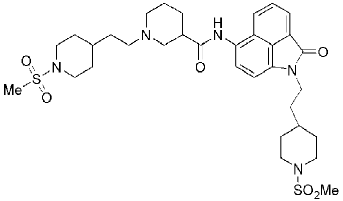 | 142 | 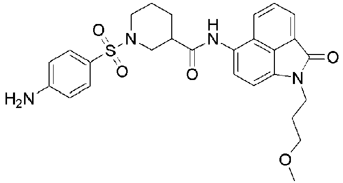 |
| 143 | 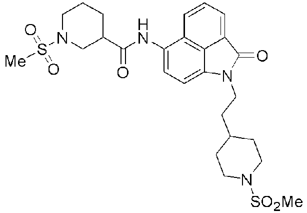 | 144 | 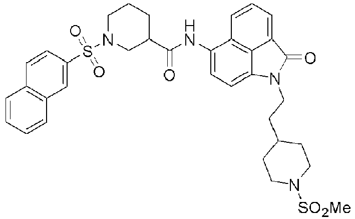 |
| 145 | 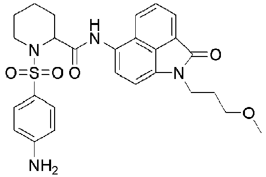 | 146 | 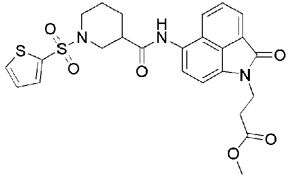 |
| 147 | 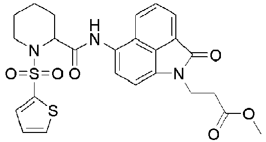 | 148 | 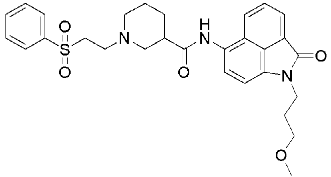 |
| 149 | 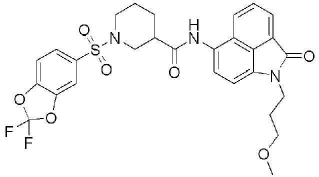 | 150 | 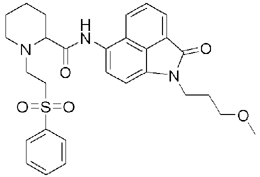 |
| 151 | 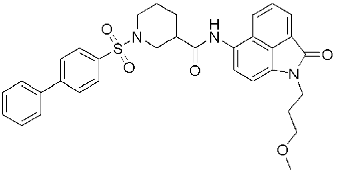 | 152 | 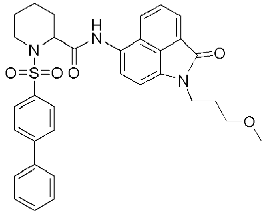 |
| 153 | 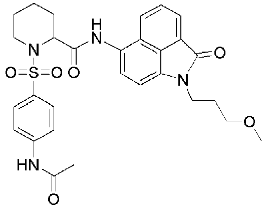 | 154 | 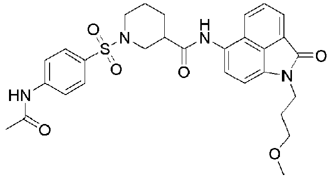 |
| 155 | 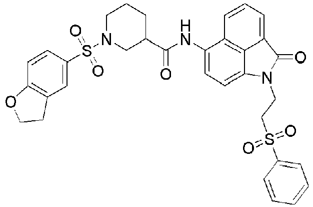 | 156 | 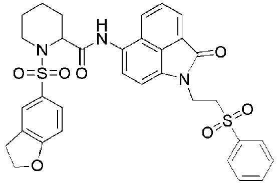 |
| 157 | 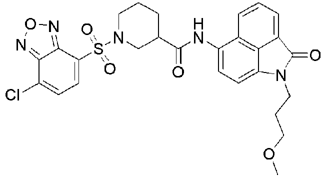 | 158 | 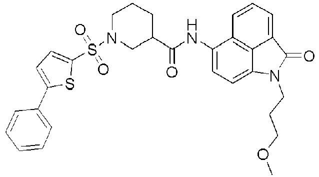 |
| 159 | 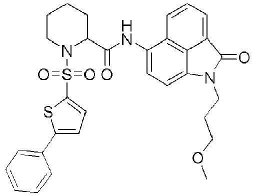 | 160 | 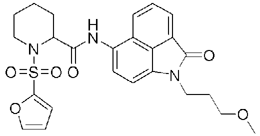 |
| 161 | 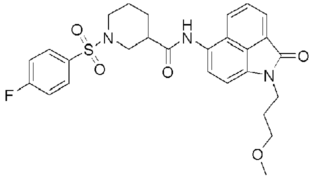 | 162 | 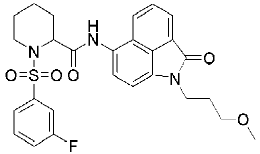 |
| 163 | 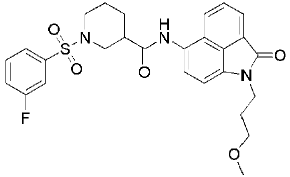 | 164 | 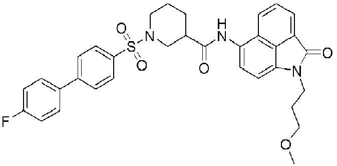 |
| 165 | 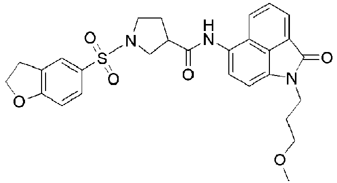 | 166 | 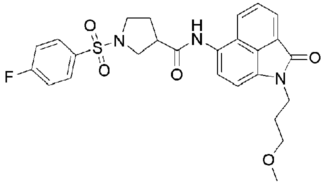 |
| 167 | 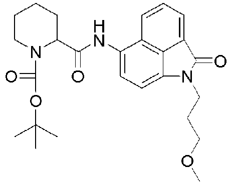 | 168 | 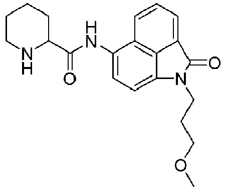 |
| 169 | 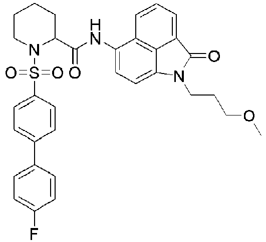 | | |

**[Table 2]**

| No. | Identification data (¹H NMR; LC/MS) |
|---|---|
| 1 | ¹H NMR (300 MHz, Chloroform-*d*) δ 8.92 (s, 1H), 8.13 (d, *J* = 7.0 Hz, 1H), 8.03 (d, *J* = 11.0 Hz, 2H), 7.74 (dd, *J* = 8.3, 7.0 Hz, 1H), 7.40 - 7.27 (m, 4H), 6.73 (d, *J* = 7.8 Hz, 1H), 5.12 (d, *J* = 3.7 Hz, 2H), 4.98 (d, *J* = 5.1 Hz, 1H), 4.12 (q, *J* = 7.5 Hz, 1H), 2.89 {t, *J* = 12.7 Hz, 1H), 2.37 (d, *J* = 13.0 Hz, 1H), 1.78 - 1.59 (m, 4H), 1.48 (s, 9H). |
| 2 | ¹H NMR (300 MHz, Chloroform-*d*) δ 8.94 (s, 1H), 8.15 (d, *J* = 7.0 Hz, 1H), 8.06 (d, *J* = 8.3 Hz, 1H), 7.94 (d, *J* = 7.7 Hz, 1H), 7.80 (dd, *J* = 8.3, 7.0 Hz, 1H), 7.70 (ddd, *J* = 10.3, 4.4, 1.4 Hz, 2H), 7.40 - 7.27 (m, 5H), 7.17 (dd, *J* = 5.1, 3.8 Hz, 1H), 6.75 (d, *J* = 7,8 Hz, 1H), 5.13 (s, 2H), 4.77 (d, *J* = 5.4 Hz, 1H), 4.22 - 4.07 (m, 1H), 3.22 (t, *J* = 13.5 Hz, 1H), 2.40 (d, *J* = 13.9 Hz, 1H), 1.44 (s, 5H), 1.26 (q, *J* = 6.9, 5.4 Hz, 4H), 0.87 (d, *J* = 7.1 Hz, 1H). |
| 3 | ¹H NMR (300 MHz, Chloroform-o) δ 9.20 (s, 1H), 8.21 (d, *J* = 8.3 Hz, 1H), 8.14 (d, *J* = 7.0 Hz, 1H), 7.87 (d, *J* = 7.7 Hz, 1H), 7.81 (dd, *J* = 8.3, 7.0 Hz, 1H), 7.33 (q, *J* = 7.9 Hz, 4H), 7.25 - 7.20 (m, 1H), 6.75 (d, *J* = 7.7 Hz, 1H), 5.13 (s, 2H), 4.36 (dd, *J* = 8.8, 2.4 Hz, 1H), 3.74 (ddd, *J* = 10.4, 7.2, 2.9 Hz, 1H), 3.35 (td, *J* = 9.8, 6.7 Hz, 1H), 2.46 (d, *J* = 6.0 Hz, 1H), 2.01 - 1.61 (m, 3H). |
| 4 | ¹H NMR (300 MHz, Chloroform-*d*) δ 9.08 (s, 1H), 8.12 (dd, *J* = 15.4, 7.6 Hz, 2H), 7.97 (d, *J* = 7.8 Hz, 1H), 7.87 (d, *J* = 8.6 Hz, 2H), 7.80 (dd, *J* = 8.3, 7.0 Hz, 1H), 7.40 - 7.27 (m, 5H), 7.04 (d, *J* = 8.6 Hz, 2H), 6.75 (d, *J* = 7.7 Hz, 1H), 5.13 (s, 2H), 4.69 (d, *J* = 5.3 Hz, 1H), 4.18 - 4.02 (m, 1H), 3.16 (t, *J* = 13.5 Hz, 1H), 2.36 (d, *J* = 13.7 Hz, 1H), 1.55 - 1.38 (m, 2H), 1.23 - 1.06 (m, 2H). |
| 5 | ¹H NMR (300 MHz, Chloroform-*d*) δ 9.06 (s, 1H), 8.12 (dd, *J* = 15.3, 7.5 Hz, 2H), 7.97 (d, *J* = 7.7 Hz, 1H), 7.82 (dd, *J* = 7.8, 5.9 Hz, 3H), 7.34 (dt, *J* = 18.5, 8.0 Hz, 7H), 7.24 (d, *J* = 6.0 Hz, 1H), 6.75 (d, *J* = 7.7 Hz, 1H), 5.13 (s, 2H), 4.70 (d, *J* = 5.3 Hz, 1H), 4.17 - 4.02 (m, 2H), 3.47 (s, 1H), 3.16 (t, *J* = 13.9 Hz, 1H), 2.47 (s, 3H), 2.35 (d, *J* = 13.6 Hz, 1H), 1.64 - 1.52 (m, 3H), 1.16 (ddd, *J* = 18.3, 11.7, 5.0 Hz, 2H). |
| 6 | ¹H NMR (300 MHz, Chloroform-*d*) δ 8.84 (s, 1H), 8.44 - 8.35 (m, 2H), 8.11 (dd, *J =* 7.9, 2.7 Hz, 3H), 8.04 (d, *J* = 8.2 Hz, 1H), 7.85 (d, *J* = 7.6 Hz, 1H), 7.81 - 7.72 (m, 1H), 7.40 - 7.27 (m, 5H), 6.72 (d, *J* = 7.7 Hz, 1H), 5.11 (s, 2H), 4.77 (d, *J* = 5.3 Hz, 1H), 4.12 - 4.01 (m, 1H), 3.29 (td, *J* = 14.3, 13.7, 2.6 Hz, 1H), 2.37 (d, *J* = 13.9 Hz, 1H), 1.68 - 1.51 (m, 4H), 1.24 - 1.10 (m, 2H). |
| 7 | ¹H NMR (300 MHz, Methanol-*d₄*) δ 8.12 (dd, *J* = 10.3, 7.7 Hz, 2H), 7.81 (d, *J* = 7.6 Hz, 1H), 7.73 (d, *J* = 7.6 Hz, 1H), 7.42 (s, 1H), 7.37 - 7.18 (m, 7H), 6.90 - 6.82 (m, 2H), 6.79 (d, *J* = 7.7 Hz, 1H), 3.88 (d, *J* = 13.6 Hz, 2H), 3.78 (s, 3H), 3.25 (t, *J* = 11.9 Hz, 1H), 2.33 (d, *J* = 9.6 Hz, 1H), 1.77 (t, *J* = 6.7 Hz, 4H), 1.63 (s, 1H). |
| 8 | ¹H NMR (300 MHz, Chloroform-*d*) δ 8.99 (s, 1H), 8.11 (dd, *J* = 7.6, 3.0 Hz, 2H), 8.03 (d, *J* = 7.7 Hz, 1H), 7.87 - 7.63 (m, 4H), 7.18 (dd, *J* = 5.0, 3.7 Hz, 1H), 6.95 (d, *J* = 7.7 Hz, 1H), 4.79 (d, *J* = 5.7 Hz, 1H), 4.17 (d, *J* = 14.6 Hz, 1H), 3.26 (t, *J* = 13.6 Hz, 1H), 2.42 (d, *J* = 13.7 Hz, 1H), 1.62 (s, 2H), 1.54 (s, 1H), 1.41 - 1.21 (m, 4H). |
| 9 | ¹H NMR (300 MHz, Chloroform-*d*) δ 8.77 (s, 1H), 8.11 (d, *J* = 7.0 Hz, 1H), 8.02 (d, *J* = 8.3 Hz, 1H), 7.87 (d, *J* = 7.7 Hz, 1H), 7.75 (dd, *J* = 8.3, 7.0 Hz, 1H), 7.39 - 7.27 (m, 4H), 6.72 (d, *J* = 7.7 Hz, 1H), 5.11 (s, 2H), 4.74 (d, *J* = 3.9 Hz, 1H), 3.96 (d, *J* = 14.1 Hz, 1H), 3.23 (td, *J* = 14.3, 13.4, 2.6 Hz, 1H), 3.05 (s, 3H), 2.52 (d, *J* = 6.9 Hz, 1H), 1.93 - 1.44 (m, 6H). |
| 10 | ¹H NMR (300 MHz, Chloroform-*d*) δ 9.08 (s, 1H), 8.55 - 8.49 (m, 1H), 8.16 - 7.83 (m, 7H), 7.79 - 7.60 (m, 3H), 7.38 - 7.27 (m, 5H), 6.74 (d, *J* = 7.7 Hz, 1H), 5.13 (s, 2H), 4.80 (d, *J* = 5.3 Hz, 1H), 4.19 (dd, *J* = 14.8, 3.8 Hz, 1H), 3.22 (t, *J* = 12.5 Hz, 1H), 2.34 (d, *J* = 13.6 Hz, 1H), 1.63 (s, 1H), 1.59 - 1.47 (m, 3H), 1.23 - 1.11 (m, 2H). |
| 11 | ¹H NMR (300 MHz, Chloroform-*d*) δ 9.90 (s, 1H), 8.20 (dd, *J* = 31.8, 7.7 Hz, 3H), 7.81 (dd, *J* = 8.3, 7.0 Hz, 1H), 7.51 (d, *J* = 8.7 Hz, 2H), 7.41 - 7.27 (m, 4H), 7.22 (d, *J* = 6.8 Hz, 1H), 6.95 (d, *J* = 8.8 Hz, 2H), 6.73 (d, *J* = 7.8 Hz, 1H), 5.33 (s, 1H), 5.21 - 5.04 (m, 2H), 3.88 (d, *J* = 7.7 Hz, 1H), 3.85 (s, 3H), 3.04 (ddd, *J* = 14.3, 11.3, 4.1 Hz, 1H), 2.43 (d, *J* = 13.5 Hz, 1H), 2.12 (d, *J* = 14.3 Hz, 1H), 1.94 - 1.58 (m, 5H), 1.49 - 1.30 (m, 1H), 0.94 - 0.80 (m, 1H). |
| 12 | ¹H NMR (300 MHz, Chloroform-*d*) δ 8.95 (s, 1H), 8.15 - 7.99 (m, 3H), 7.83 - 7.65 (m, 3H), 7.18 (dd, *J* = 5.0, 3.8 Hz, 1H), 7.01 (d, *J* = 7.8 Hz, 1H), 4.79 (d, *J* = 5.5 Hz, 1H), 4.19 (s, 1H), 4.11 (t, *J* = 5.6 Hz, 2H), 3.71 (t, *J* = 5.5 Hz, 2H), 3.34 (s, 3H), 3.25 (t, *J* = 13.6 Hz, 1H), 2.42 (d, *J* = 13.4 Hz, 1H), 1.62 (s, 2H), 0.86 (s, 1H). |
| 13 | ¹H NMR (300 MHz, Chloroform-*d*) δ 10.05 (s, 1H), 8.21 (d, *J* = 7.8 Hz, 1H), 8.16 - 8.05 (m, 5H), 8.00 (d, *J* = 14.4 Hz, 1H), 7.89 - 7.73 (m, 2H), 7.68 (s, 1H), 7.63 (d, *J* = 7.8 Hz, 1H), 7.38 (d, *J* = 7.9 Hz, 1H), 7.25-7.06 (m, 4H), 6.96 (d, *J* = 7.7 Hz, 1H), 4.46 (t, *J* = 7.1 Hz, 2H), 3.87 (s, 3H), 3.65 (s, 1H), 3.15 (t, *J* = 7.1 Hz, 2H), 2.64 (s, 2H), 1.80 (s, 1H). |
| 14 | ¹H NMR (300 MHz, Chloroform-*d*) δ 9.00 (s, 1H), 8.15 - 8.07 (m, 2H), 8.02 (d, *J* = 7.8 Hz, 1H), 7.86 - 7.76 (m, 1H), 7.75 - 7.65 (m, 2H), 7.24-7.13 (m, 1H), 4.62 (s, 1H), 4.42 (t, *J* = 11.8 Hz, 3H), 4.14 (s, 2H), 3.81 (d, *J* = 11.6 Hz, 5H), 3.58 - 3.15 (m, 8H), 2.39 (s, 1H), 2.24 (d, *J* = 7.4 Hz, 1H), 2.02 (d, *J* = 11.9 Hz, 3H), 1.27 (d, *J* = 8.4 Hz, 7H), 1.03 - 0.77 (m, 3H). |
| 15 | ¹H NMR (300 MHz, Chloroform-*d*) δ 8.96 (s, 1H), 8.07 (dd, *J* = 9.6, 7.6 Hz, 2H), 7.99 (d, *J* = 7.6 Hz, 1H), 7.82 - 7.66 (m, 3H), 7.21 - 7.11 (m, 3H), 6.80 (d, *J* = 8.3 Hz, 2H), 6.73 (d, *J* = 7.7 Hz, 1H), 4.78 (d, *J* = 5.5 Hz, 1H), 4.25 - 4.04 (m, 3H), 3.77 (s, 3H), 3.25 (t, *J* = 13.5 Hz, 1H), 3.02 (t, *J* = 7.4 Hz, 2H), 2.42 (d, *J* = 13.7 Hz, 1H), 2.02 (s, 2H). |
| 16 | ¹H NMR (500 MHz, Chloroform-*d*) δ 9.03 (s, 1H), 8.16 - 8.03 (m, 5H), 7.86 - 7.80 (m, 1H), 7.78 - 7.70 (m, 2H), 7.43-7.38 (m, 2H), 7.29 (s, 5H), 7.21 (dd, *J* = 5.0, 3.7 Hz, 1H), 6.78 (d, *J* = 7.7 Hz, 1H), 4.81 (d, *J* = 5.6 Hz, 1H), 4.24 (td, *J* = 7.1, 2.7 Hz, 2H), 4.20 (s, 1H), 3.25 (q, *J* = 7.4 Hz, 3H), 2.44 (d, *J* = 13.7 Hz, 1H), 1.57 (s, 7H), 1.39 - 1.30 (m, 3H), 0.03 (s, 4H). |
| 17 | ¹H NMR (300 MHz, Chloroform-*d*) δ 8.96 (s, 1H), 8.13 - 7.98 (m, 3H), 7.83 - 7.64 (m, 3H), 7.18 (dd, *J* = 5.0, 3.8 Hz, 1H), 6.95 (d, *J* = 7.7 Hz, 1H), 4.79 (d, *J* = 5.5 Hz, 1H), 4.16 (dd, *J* = 13.9, 3.8 Hz, 1H), 4.02 (t, *J* = 6.8 Hz, 2H), 3.42 (t, *J* = 6.0 Hz, 2H), 3.33 (s, 3H), 3.25 (t, *J* = 12.9 Hz, 1H), 2.42 (d, *J* = 13.7 Hz, 1H), 2.05 (p, *J* = 6.4 Hz, 3H), 1.58 (d, *J* = 14.2 Hz, 5H). |
| 18 | ¹H NMR (300 MHz, Chloroform-*d*) δ 9.00 (s, 1H), 8.60 - 8.51 (m, 2H), 8.15 (dd, *J =* 15.6, 7.6 Hz, 2H), 7.99 (d, *J* = 7.8 Hz, 1H), 7.91 - 7.77 (m, 1H), 7.76 - 7.65 (m, 2H), 7.24 (d, *J* = 5.2 Hz, 1H), 7.18 (t, *J* = 4.4 Hz, 1H), 6.71 (d, *J* = 7.7 Hz, 1H), 5.14 (s, 2H), 4.77 (d, *J* = 5.5 Hz, 1H), 4.18 (s, 1H), 3.22 (t, *J* = 13.5 Hz, 1H), 2.40 (d, *J* = 14.0 Hz, 1H), 1.27 (q, *J* = 7.2, 6.7 Hz, 3H). |
| 19 | ¹H NMR (300 MHz, Chloroform-*d*) δ 8.96 (s, 1H), 8.13 - 7.97 (m, 3H), 7.78 (dd, *J =* 8.3, 7.0 Hz, 1H), 7.73 (dd, *J* = 3.8, 1.4 Hz, 1H), 7.69 (dt, *J* = 5.1, 1.5 Hz, 1H), 7.18 (dd, *J* = 5.1, 3.7 Hz, 1H), 7.12 (d, *J* = 8.5 Hz, 2H), 6.81 (dd, *J* = 8.1, 5.5 Hz, 3H), 4.79 (d, *J* = 5.5 Hz, 1H), 4.16 (d, *J* = 15.0 Hz, 1H), 3.95 (t, *J* = 7.1 Hz, 2H), 3.78 (s, 3H), 3.25 (t, *J* = 13.6 Hz, 1H), 2.68 (t, *J* = 7.7 Hz, 2H), 2.42 (d, *J* = 14.0 Hz, 1H), 2.17 - 2.07 (m, 2H), 1.31 (s, 2H). |
| 20 | ¹H NMR (300 MHz, Chloroform-*d*) δ 9.00 (s, 1H), 8.14 - 8.06 (m, 3H), 7.85 - 7.77 (m, 1H), 7.75 - 7.67 (m, 2H), 7.18 (dd, *J* = 5.1, 3.7 Hz, 1H), 6.97 (d, *J* = 7.7 Hz, 1H), 4.79 (d, *J* = 5.7 Hz, 1H), 4.16 (d, *J* = 17.4 Hz, 1H), 4.07 (t, *J* = 6.6 Hz, 2H), 3.25 (t, *J* = 12.4 Hz, 1H), 2.52 - 2.36 (m, 3H), 2.21 (q, *J* = 7.0 Hz, 2H). |
| 21 | ¹H NMR (300 MHz, Chloroform-*d*) δ 9.02 (s, 1H), 8.15 - 8.09 (m, 3H), 7.81 (dd, *J* = 8.3, 7.0 Hz, 1H), 7.75 - 7.66 (m, 2H), 7.18 (dd, *J* = 5.0, 3.7 Hz, 1H), 7.01 (d, *J* = 7.8 Hz, 1H), 4.78 (d, *J* = 5.6 Hz, 1H), 4.34 - 4.06 (m, 4H), 3.32 - 3.17 (m, 1H), 2.87 (t, *J* = 6.9 Hz, 2H), 2.41 (d, *J* = 14.0 Hz, 1H), 1.65 (s, 2H), 1.44-1.27 (m, 3H). |
| 22 | ¹H NMR (300 MHz, Chloroform-o) δ 8.04 (d, *J* = 6.9 Hz, 1H), 7.87 (d, *J* = 8.3 Hz, 1H), 7.75 - 7.49 (m, 5H), 7.14 (d, *J* = 8.6 Hz, 2H), 7.09 - 7.04 (m, 1H), 6.80 (d, *J* = 8.5 Hz, 2H), 6.68 (d, *J* = 7.7 Hz, 1H), 5.10 (d, *J* = 7.4 Hz, 1H), 4.16 - 4.04 (m, 2H), 3.77 (s, 3H), 3.62 (s, 1H), 3.00 (t, *J* = 7.5 Hz, 2H), 2.49 (s, 1H), 2.04 (s, 1H), 2.03 - 1.79 (m, 6H), 1.66 (s, 3H). |
| 23 | ¹H NMR (300 MHz, Chloroform-*d*) δ 8.07 (d, *J* = 6.9 Hz, 1H), 7.86 (d, *J* = 8.4 Hz, 1H), 7.76 - 7.55 (m, 4H), 7.41 (s, 1H), 7.20 - 7.09 (m, 3H), 6.79 (d, *J* = 8.3 Hz, 2H), 6.69 (d, *J* = 7.7 Hz, 1H), 4.09 (t, *J* = 7.4 Hz, 2H), 3.89 (d, *J* = 11.8 Hz, 2H), 3.77 (s, 3H), 3.00 (t, *J* = 7.4 Hz, 2H), 2.64 (t, *J* = 11.7 Hz, 2H), 2.43 (s, 1H), 2.20 - 1.98 (m, 4H). |
| 24 | ¹H NMR (300 MHz, Chloroform-*d*) δ 8.19 (s, 1H), 8.07 (dd, *J* = 7.6, 5.2 Hz, 2H), 7.83 (d, *J* = 7.7 Hz, 1H), 7.79 - 7.72 (m, 1H), 7.68 (d, *J* = 5.1 Hz, 1H), 7.62 - 7.58 (m, 1H), 7.19 (t, *J* = 4.4 Hz, 1H), 7.15 (d, *J* = 8.3 Hz, 2H), 6.80 (d, *J* = 8.5 Hz, 2H), 6.71 (d, *J* = 7.7 Hz, 1H), 4.09 (t, *J* = 7.5 Hz, 2H), 3.77 (s, 3H), 3.55 (d, *J* = 12.6 Hz, 1H), 3.38 - 3.24 (m, 2H), 3.01 (t, *J* = 7.5 Hz, 3H), 2.86 (s, 1H), 1.94 (s, 4H), 1.83 (d, *J* = 4.0 Hz, 1H). |
| 25 | ¹H NMR (300 MHz, DMSO-*d₆*) δ 9.82 (s, 1H), 8.24 (d, *J* = 8.2 Hz, 1H), 8.02 (d, *J* = 7.0 Hz, 1H), 7.91 (t, *J* = 6.1 Hz, 2H), 7.79 (t, *J* = 7.6 Hz, 1H), 7.67 (d, *J* = 7.7 Hz, 1H), 7.62 (d, *J* = 3.6 Hz, 1H), 7.21 - 7.06 (m, 4H), 6.79 (d, *J* = 8.3 Hz, 2H), 4.05 (t, *J* = 7.2 Hz, 2H), 3.68 (s, 3H), 2.94 (t, *J* = 7.2 Hz, 2H), 1.90 (d, *J* = 13.2 Hz, 2H), 1.77 (d, *J* = 12.4 Hz, 2H), 1.46 (q, *J* = 13.0, 12.2 Hz, 2H), 1.27 (d, *J* = 19.4 Hz, 4H). |
| 26 | LC/MS : 614.1 |
| 27 | ¹H NMR (300 MHz, Chloroform-a) δ 8.10 - 7.98 (m, 3H), 7.84 - 7.71 (m, 2H), 7.38 (d, *J* = 4.0 Hz, 1H), 7.15 (d, *J* = 8.5 Hz, 2H), 7.02 (d, *J* = 4.0 Hz, 1H), 6.84 - 6.77 (m, 2H), 6.71 (d, *J* = 7.7 Hz, 1H), 4.10 (t, *J* = 7.5 Hz, 2H), 3.77 (s, 3H), 3.61 (d, *J* = 12.1 Hz, 1H), 3.39 (d, *J* = 11.8 Hz, 1H), 3.29 - 3.16 (m, 1H), 3.01 (t, *J* = 7.5 Hz, 3H), 2.84 (s, 1H), 1.90 (d, *J* = 39.7 Hz, 4H). |
| 28 | ¹H NMR (300 MHz, Chloroform-*d*) δ 8.18 (s, 1H), 8.08 (dd, *J* = 7.6, 4.5 Hz, 2H), 7.88 (d, *J* = 7.6 Hz, 1H), 7.76 (t, *J* = 7.7 Hz, 1H), 7.71 - 7.65 (m, 1H), 7.60 (d, *J* = 3.6 Hz, 1H), 7.19 (t, *J* = 4.4 Hz, 1H), 6.93 (d, *J* = 7.7 Hz, 1H), 4.01 (t, *J* = 6.7 Hz, 2H), 3.56 (d, *J* = 11.8 Hz, 1H), 3.42 (t, *J* = 6.0 Hz, 2H), 3.33 (s, 5H), 3.01 (t, *J* = 10.2 Hz, 1H), 2.87 (s, 1H), 2.04 (p, *J* = 6.5 Hz, 2H), 1.94 (s, 3H), 1.83 (d, *J* = 3.7 Hz, 1H). |
| 29 | ¹H NMR (300 MHz, Chloroform-*d*) δ 8.18 (s, 1H), 8.03 (dd, *J* = 7.6, 3.4 Hz, 2H), 7.81 (d, *J* = 7.7 Hz, 1H), 7.70 (t, *J* = 7.6 Hz, 1H), 7.37 (d, *J* = 4.0 Hz, 1H), 7.01 (d, *J* = 4.0 Hz, 1H), 6.89 (d, *J* = 7.7 Hz, 1H), 3.99 (t, *J* = 6.7 Hz, 2H), 3.75 - 3.62 (m, 1H), 3.41 (t, *J* = 6.0 Hz, 3H), 3.33 (s, 3H), 3.15 (t, *J* = 10.2 Hz, 1H), 2.86 (d, *J* = 9.9 Hz, 2H), 2.10 - 1.79 (m, 6H). |
| 30 | ¹H NMR (300 MHz, Chloroform-*d*) δ 8.89 (s, 1H), 8.12 (d, *J* = 7.0 Hz, 1H), 8.06 (dd, *J* = 8.0, 1.5 Hz, 2H), 7.80 (dd, *J* = 8.3, 7.0 Hz, 1H), 7.53 (d, *J* = 4.0 Hz, 1H), 7.03 (d, *J* = 4.1 Hz, 1H), 6.98 (d, *J* = 7.7 Hz, 1H), 4.77 (d, *J* = 5.5 Hz, 1H), 4.19 - 4.09 (m, 1H), 4.04 (t, *J* = 6.7 Hz, 2H), 3.45 (t, *J* = 6.0 Hz, 2H), 3.36 (s, 3H), 3.35 - 3.24 (m, 1H), 2.48 (d, *J* = 13.8 Hz, 1H), 2.14 - 2.02 (m, 2H), 1.76 - 1.60 (m, 2H), 1.40 (tt, *J* = 12.3, 5.9 Hz, 2H). |
| 31 | ¹H NMR (300 MHz, Chloroform-*d*) δ 8.14 (dd, *J* = 10.6, 6.8 Hz, 1H), 7.98 - 7.72 (m, 2H), 7.54 (ddd, *J* = 12.5, 5.0, 1.3 Hz, 1H), 7.46 - 7.40 (m, 1H), 7.37 - 7.30 (m, 1H), 7.15 - 7.00 (m, 2H), 6.95 (d, *J* = 7.5 Hz, 1H), 4.30 - 3.97 (m, 3H), 3.90 - 3.80 (m, 1H), 3.78 - 3.68 (m, 1H), 3.67 - 3.55 (m, 1H), 3.49 (qd, *J* = 5.5, 4.8, 2.0 Hz, 4H), 3.36 (d, *J* = 3.3 Hz, 3H), 3.26 (d, *J* = 4.3 Hz, 3H), 2.45 (d, *J* = 5.0 Hz, 1H), 2.37 - 2.27 (m, 1H), 2.24 - 2.17 (m, 1H), 2.09 (p, *J* = 6.6 Hz, 2H), 1.61 (s, 2H), 0.92 - 0.66 (m, 6H). |
| 32 | ¹H NMR (300 MHz, Chloroform-*d*) δ 8.09 (d, *J* = 6.9 Hz, 1H), 7.89 (d, *J* = 8.1 Hz, 1H), 7.82 - 7.75 (m, 1H), 7.63 - 7.54 (m, 2H), 7.44 - 7.33 (m, 1H), 7.06 (d, *J* = 7.5 Hz, 1H), 6.95 - 6.91 (m, 1H), 4.87 - 4.81 (m, 1H), 4.08 - 3.98 (m, 3H), 3.88 (td, *J* = 11.9, 3.1 Hz, 1H), 3.78 - 3.65 (m, 2H), 3.57 - 3.42 (m, 5H), 3.39 - 3.32 (m, 5H), 3.26 (d, *J* = 1.5 Hz, 3H), 2.06 (h, *J* = 6.7 Hz, 3H), 1.76 - 1.52 (m, 6H), 0.91 - 0.66 (m, 5H). |
| 33 | ¹H NMR (300 MHz, Chloroform-*d*) δ 8.09 (d, *J* = 7.0 Hz, 1H), 7.98 (d, *J* = 6.8 Hz, 1H), 7.88 - 7.71 (m, 3H), 6.94 (d, *J* = 7.7 Hz, 1H), 4.01 (t, *J* = 6.7 Hz, 2H), 3.85 (d, *J* = 12.4 Hz, 1H), 3.65 (d, *J* = 13.1 Hz, 1H), 3.42 (t, *J* = 5.9 Hz, 2H), 3.33 (s, 3H), 3.16 - 3.04 (m, 1H), 2.84 (s, 1H), 2.16 - 1.79 (m, 6H). |
| 34 | ¹H NMR (300 MHz, Chloroform-*d*) δ 8.36 (s, 1H), 8.16 (d, *J* = 8.3 Hz, 1H), 8.08 (d, *J* = 7.0 Hz, 1H), 7.94 (d, *J* = 7.7 Hz, 1H), 7.83 - 7.73 (m, 1H), 7.60 (d, *J* = 9.0 Hz, 2H), 6.92 (dd, *J* = 13.4, 7.9 Hz, 2H), 4.71 (t, *J* = 8.8 Hz, 2H), 4.01 (t, *J* = 6.7 Hz, 2H), 3.42 (t, *J* = 5.9 Hz, 2H), 3.31 (d, *J* = 12.1 Hz, 7H), 3.11 (s, 2H), 2.84 (s, 1H), 2.11 - 1.72 (m, 7H). |
| 35 | ¹H NMR (300 MHz, Chloroform-*d*) δ 8.03 (dd, *J* = 15.6, 7.5 Hz, 3H), 7.82 (d, *J* = 7.7 Hz, 1H), 7.73 (dd, *J* = 8.3, 7.0 Hz, 1H), 6.92 (d, *J* = 7.7 Hz, 1H), 4.00 (t, *J* = 6.8 Hz, 2H), 3.72 (dd, *J* = 11.9, 3.7 Hz, 1H), 3.49 (d, *J* = 10.9 Hz, 1H), 3.41 (t, *J* = 5.9 Hz, 2H), 3.33 (s, 3H), 3.23 (dd, *J* = 12.0, 8.6 Hz, 1H), 2.94 (t, *J* = 9.7 Hz, 1H), 2.83 (dt, *J* = 8.6, 4.5 Hz, 1H), 2.68 (s, 3H), 2.44 (s, 3H), 2.11 - 1.75 (m, 6H). |
| 36 | ¹H NMR (300 MHz, Chloroform-*d*) δ 8.49 (s, 1H), 8.06 (dd, *J* = 12.5, 7.6 Hz, 2H), 7.83 (d, *J* = 7.7 Hz, 1H), 7.71 (t, *J* = 7.6 Hz, 1H), 6.90 (d, *J* = 7.7 Hz, 1H), 3.99 (t, *J* = 6.8 Hz, 2H), 3.55 (d, *J* = 11.2 Hz, 1H), 3.41 (t, *J* = 5.9 Hz, 2H), 3.32 (s, 5H), 3.01 (t, *J* = 9.6 Hz, 1H), 2.88 (s, 1H), 2.47 (s, 6H), 2.09 - 1.86 (m, 6H). |
| 37 | ¹H NMR (300 MHz, Chloroform-*d*) δ 9.08 (s, 1H), 8.26 - 8.11 (m, 3H), 7.89 - 7.77 (m, 2H), 7.72 (ddd, *J* = 9.7, 4.4, 1.3 Hz, 2H), 7.19 (dd, *J* = 5.0, 3.8 Hz, 1H), 5.33 (p, *J* = 6.3 Hz, 1H), 4.79 (d, *J* = 5.6 Hz, 1H), 4.17 (d, *J* = 14.5 Hz, 1H), 3.33 - 3.17 (m, 1H), 2.44 (s, 1H), 1.62 (d, *J* = 3.4 Hz, 2H), 1.52 (s, 3H), 1.49 (s, 3H), 1.29 (dd, *J* = 14.6, 6.4 Hz, 4H). |
| 38 | ¹H NMR (300 MHz, Chloroform-*d*) δ 8.52 (s, 1H), 8.15 - 8.04 (m, 2H), 7.95 (dd, *J* = 13.9, 8.0 Hz, 2H), 7.83 - 7.71 (m, 1H), 6.95 (d, *J* = 7.7 Hz, 1H), 4.80 (d, *J* = 5.3 Hz, 1H), 4.15 - 3.96 (m, 3H), 3.42 (t, *J* = 5.9 Hz, 2H), 3.34 (s, 3H), 2.47 (d, *J* = 13.2 Hz, 1H), 2.06 (q, *J* = 6.4 Hz, 2H), 1.75 (d, *J* = 12.0 Hz, 3H), 1.43 (s, 2H). |
| 39 | ¹H NMR (300 MHz, Chloroform-*d*) δ 9.11 (s, 1H), 8.14 - 8.02 (m, 3H), 7.84 - 7.68 (m, 3H), 6.94 (dd, *J* = 11.8, 8.0 Hz, 2H), 4.72 (t, *J* = 8.8 Hz, 3H), 4.16 - 3.95 (m, 3H), 3.43 (t, *J* = 5.9 Hz, 2H), 3.38 - 3.12 (m, 6H), 2.40 (d, *J* = 13.7 Hz, 1H), 2.05 (p, *J* = 6.4 Hz, 2H), 1.73 - 1.46 (m, 6H), 1.25 (s, 3H). |
| 40 | ¹H NMR (300 MHz, Chloroform-*d*) δ 8.91 (s, 1H), 8.13 - 7.99 (m, 3H), 7.79 (dd, *J* = 8.3, 7.0 Hz, 1H), 6.96 (d, *J* = 7.7 Hz, 1H), 4.74 (d, *J* = 5.4 Hz, 1H), 4.08 - 3.87 (m, 3H), 3.43 (t, *J* = 6.0 Hz, 2H), 3.34 (s, 4H), 2.74 (s, 3H), 2.50 (s, 4H), 2.05 (p, *J* = 6.4 Hz, 2H), 1.83 - 1.61 (m, 3H), 1.55 - 1.18 (m, 4H). |
| 41 | ¹H NMR (300 MHz, Chloroform-*d*) δ 9.97 (s, 1H), 9.18 (s, 1H), 8.19 - 8.01 (m, 3H), 7.78 (dd, *J* = 8.3, 7.0 Hz, 1H), 6.96 (d, *J* = 7.7 Hz, 1H), 4.74 (d, *J* = 5.4 Hz, 1H), 4.09 - 3.88 (m, 3H), 3.43 (t, *J* = 6.0 Hz, 2H), 3.34 (s, 3H), 3.22 (t, *J* = 13.7 Hz, 1H), 2.54 (s, 6H), 2.06 (p, *J* = 6.4 Hz, 2H), 1.72 - 1.23 (m, 9H). |
| 42 | ¹H NMR (300 MHz, Chloroform-*d*) δ 9.09 (s, 1H), 8.16 - 7.98 (m, 3H), 7.78 (dd, *J* = 8.3, 6.9 Hz, 1H), 7.61 - 7.52 (m, 1H), 7.38 (d, *J* = 2.1 Hz, 1H), 6.98 (dd, *J* = 12.4, 8.1 Hz, 2H), 4.72 (d, *J* = 5.5 Hz, 1H), 4.17 - 3.90 (m, 9H), 3.43 (t, *J* = 5.9 Hz, 2H), 3.34 (s, 3H), 3.20 (t, *J* = 13.4 Hz, 1H), 2.40 (d, *J* = 13.4 Hz, 1H), 2.05 (t, *j* = 6.3 Hz, 2H), 1.53 (s, 2H), 1.23 (d, *J* = 16.3 Hz, 3H). |
| 43 | ¹H NMR (300 MHz, Chloroform-*d*) δ 9.00 (d, *J* = 8.6 Hz, 2H), 8.08 (td, *J* = 8.8, 7.9, 5.5 Hz, 3H), 7.84 - 7.73 (m, 1H), 6.96 (d, *J* = 7.7 Hz, 1H), 4.80 (d, *j* = 5.4 Hz, 1H), 4.12 (d, *J* = 14.5 Hz, 1H), 4.02 (t, *J* = 6.7 Hz, 2H), 3.43 (t, *J* = 5.9 Hz, 2H), 3.34 (s, 4H), 2.63 (s, 3H), 2.55 - 2.43 (m, 1H), 2.32 (s, 3H), 2.06 (q, *J* = 6.4 Hz, 2H), 1.65 (s, 2H), 1.56 (s, 1H), 1.39 (d, *J* = 13.1 Hz, 2H). |
| 44 | ¹H NMR (300 MHz, Chloroform-*d*) δ 8.95 (s, 1H), 8.15 - 7.99 (m, 3H), 7.87 - 7.71 (m, 3H), 7.51 (d, *J* = 7.6 Hz, 1H), 6.95 (d, *J* = 7.7 Hz, 1H), 4.88 (d, *J* = 5.3 Hz, 1H), 4.20 (d, *J* = 14.7 Hz, 1H), 4.02 (t, *J* = 6.8 Hz, 2H), 3.43 (t, *J* = 5.9 Hz, 2H), 3.37 - 3.22 (m, 4H), 2.77 (d, *J* = 0.9 Hz, 3H), 2.46 (d, *J* = 13.8 Hz, 1H), 2.05 (t, *J* = 6.3 Hz, 2H), 1.62 (d, *J* = 13.5 Hz, 3H), 1.54 - 1.48 (m, 1H), 1.30 (d, *J* = 24.4 Hz, 4H). |
| 45 | ¹H NMR (300 MHz, Chloroform-*d*) δ 9.01 (s, 2H), 8.19 - 8.03 (m, 6H), 7.86 - 7.77 (m, 2H), 7.75 - 7.66 (m, 4H), 7.18 (d, *J* = 8.9 Hz, 3H), 6.96 (d, *J* = 7.7 Hz, 2H), 4.79 (d, *J* = 5.8 Hz, 2H), 4.34 (s, 1H), 4.19 (s, 2H), 4.11 (t, *J* = 6.3 Hz, 4H), 3.62 (s, 4H), 3.24 (t, *J* = 13.5 Hz, 3H), 3.02 (s, 2H), 2.42 (d, *J* = 13.8 Hz, 3H), 2.02 - 1.92 (m, 5H), 1.28 (s, 10H). |
| 46 | ¹H NMR (300 MHz, Chloroform-*d*) δ 8.31 (s, 1H), 8.12 (dd, *J* = 19.8, 7.7 Hz, 2H), 7.92 (d, *J* = 7.7 Hz, 1H), 7.78 (t, *J* = 7.7 Hz, 1H), 7.42 (d, *J* = 8.6 Hz, 1H), 7.25 (s, 1H), 6.97 (dd, *J* = 15.2, 8.1 Hz, 2H), 4.01 (t, *J* = 6.7 Hz, 2H), 3.95 (d, *J* = 7.9 Hz, 6H), 3.42 (t, *J =* 6.0 Hz, 2H), 3.37 (s, 1H), 3.33 (s, 3H), 3.13 (s, 2H), 2.84 (s, 1H), 2.06 (q, *J* = 6.4 Hz, 2H), 1.89 (t, *J* = 19.1 Hz, 5H). |
| 47 | ¹H NMR (300 MHz, DMSO-*d₆*) δ 12.64 (s, 1H), 10.12 (s, 1H), 8.27 (d, *J* = 8.3 Hz, 1H), 8.07 (d, *J* = 6.9 Hz, 1H), 7.88 - 7.79 (m, 1H), 7.71 (d, *J* = 7.7 Hz, 1H), 7.12 (d, *J* = 7.7 Hz, 1H), 4.03 (d, *J* = 7.1 Hz, 1H), 3.92 (t, *J* = 6.9 Hz, 2H), 3.81 (d, *J* = 11.0 Hz, 1H), 3.63 (d, *J* = 11.4 Hz, 1H), 3.21 (s, 2H), 1.97 - 1.85 (m, 3H), 1.65 - 1.46 (m, 2H). |
| 48 | ¹H NMR (300 MHz, Chloroform-*d*) δ 8.06 (t, *J* = 6.7 Hz, 3H), 7.89 - 7.70 (m, 4H), 7.48 (dd, *J* = 8.7, 1.9 Hz, 1H), 6.92 (d, *J* = 7.6 Hz, 1H), 4.01 (t, *J* = 6.7 Hz, 2H), 3.76 (d, *J* = 12.2 Hz, 1H), 3.53 (d, *J* = 11.6 Hz, 1H), 3.42 (t, *J* = 5.8 Hz, 2H), 3.38 (s, 1H), 3.33 (s, 3H), 3.12 (d, *J* = 9.1 Hz, 1H), 2.85 (s, 1H), 2.72 (s, 3H), 2.05 (q, *J* = 6.3 Hz, 2H), 1.90 (d, *J* = 40.4 Hz, 4H). |
| 49 | ¹H NMR (300 MHz, Chloroform-*d*) δ 8.40 (s, 1H), 8.14 (d, *J* = 8.3 Hz, 1H), 8.06 (d, *J* = 7.0 Hz, 1H), 7.90 (d, *J* = 7.7 Hz, 1H), 7.75 (dd, *J* = 8.3, 7.0 Hz, 1H), 7.33 - 7.27 (m, 2H), 7.00 (d, *J* = 8.4 Hz, 1H), 6.92 (d, *J* = 7.7 Hz, 1H), 4.38 - 4.27 (m, 4H), 4.00 (t, *J* = 6.7 Hz, 2H), 3.42 (t, *J* = 5.9 Hz, 3H), 3.33 (s, 3H), 3.26 (dd, *J* = 21.3, 9.6 Hz, 2H), 3.01 (s, 1H), 2.86 (s, 1H), 2.04 (p, *J* = 6.4 Hz, 2H), 1.91 (s, 4H). |
| 50 | ¹H NMR (300 MHz, Chloroform-*d*) δ 8.06 (d, *J* = 7.0 Hz, 1H), 7.91 (d, *J* = 8.3 Hz, 1H), 7.83 (s, 1H), 7.75 - 7.64 (m, 2H), 6.91 (d, *J* = 7.7 Hz, 1H), 4.00 (t, *J* = 6.8 Hz, 2H), 3.72 (dd, *J* = 13.0, 3.7 Hz, 1H), 3.42 (t, *J* = 5.9 Hz, 3H), 3.33 (s, 4H), 3.13 (d, *J* = 8.0 Hz, 1H), 2.93 (d, *J* = 4.5 Hz, 2H), 2.89 - 2.76 (m, 1H), 2.49 (d, *J* = 3.7 Hz, 6H), 2.04 (d, *J* = 10.9 Hz, 7H), 1.85 (s, 2H), 1.59 (s, 3H), 1.46 (d, *J* = 6.2 Hz, 6H), 1.26 (s, 1H). |
| 51 | ¹H NMR (300 MHz, Chloroform-*d*) δ 8.97 (s, 1H), 8.25 - 7.99 (m, 3H), 7.82 - 7.65 (m, 3H), 7.18 (dd, *J* = 5.0, 3.7 Hz, 1H), 6.96 (d, *J* = 7.7 Hz, 1H), 4.84 - 4.73 (m, 1H), 4.22 - 4.07 (m, 1H), 4.04 (t, *J* = 6.9 Hz, 2H), 3.25 (t, *J* = 15.0 Hz, 2H), 2.58 (t, *J* = 7.2 Hz, 1H), 2.42 (d, *J* = 13.7 Hz, 1H), 2.14 - 2.02 (m, 4H), 1.55 - 1.45 (m, 1H), 1.33 - 1.21 (m, 5H). |
| 52 | ¹H NMR (300 MHz, Chloroform-*d*) δ 9.00 (s, 1H), 8.17 - 7.99 (m, 3H), 7.81 (dd, *J* = 8.4, 6.9 Hz, 1H), 7.77 - 7.65 (m, 2H), 7.18 (dd, *J* = 5.0, 3.7 Hz, 1H), 6.96 (d, *J* = 7.7 Hz, 1H), 4.79 (d, *J* = 5.5 Hz, 1H), 4.19 (brs, 1H), 4.13 (t, *J* = 6.6 Hz, 2H), 3.25 (t, *J* = 12.5 Hz, 1H), 3.18 - 3.06 (m, 2H), 2.92 (s, 3H), 2.46 - 2.31 (m, 2H), 2.03 (d, *J* = 7.0 Hz, 1H), 1.42 - 1.15 (m, 5H). |
| 53 | ¹H NMR (300 MHz, MeOD) δ 8.51 (s, 1H), 8.20 (m, 2H), 8.01 (m, 2H), 7.78 (m, 1H), 7.42 (d, *J* = 8.6 Hz, 1H), 7.25 (s, 1H), 6.97 (dd, *J* = 15.2, 8.1 Hz, 2H), 4.01 (t, *J* = 6.7 Hz, 2H), 3.95 (d, *J* = 7.9 Hz, 6H), 3.42 (t, *J* = 6.0 Hz, 2H), 3.37 (s, 1H), 3.33 (s, 3H), 3.13 (s, 2H), 2.84 (s, 1H), 2.06 (q, *J* = 6.4 Hz, 2H), 1.89 (t, *J* = 19.1 Hz, 5H). |
| 54 | ¹H NMR (300 MHz, MeOD) δ 8.70 (s, 1H), 8.18 (m, 2H), 7.99 (m, 2H), 7.78 (m, 1H), 7.42 (m, 1H), 7.25 (s, 1H), 6.97 (m, 2H), 4.00 (t, *J* = 6.7 Hz, 2H), 3.90 (d, *J* = 7.9 Hz, 6H), 3.41 (t, *J* = 6.0 Hz, 2H), 3.37 (s, 1H), 3.30 (s, 3H), 3.08 (s, 2H), 2.82 (s, 1H), 2.16 (q, *J* = 6.4 Hz, 2H), 1.80 (t, *J* = 19.1 Hz, 5H). |
| 55 | LC/MS: 599.1 |
| 56 | ¹H NMR (300 MHz, Chloroform-*d*) δ 8.53 (s, 1H), 8.10 (dd, *J* = 19.8, 7.7 Hz, 2H), 8.00 (d, *J* = 7.7 Hz, 1H), 7.82 (d, *J* = 19.8 Hz, 1H), 7.67 (t, *J* = 7.7 Hz, 1H), 7.35 (m, 1H), 7.19 (s, 1H), 6.93 (m, 2H), 6.09 (s, 2H), 3.98 (t, *J* = 6.7 Hz, 2H), 3.60 (m, 1H), 3.42 (t, *J* = 6.0 Hz, 2H), 3.35 (m, 3H), 3.10 (m, 1H), 2.91 (m, 2H), 2.14 (m, 6H). |
| 57 | ¹H NMR (300 MHz, Chloroform-*d*) δ 8.96 (s, 1H), 8.15 - 8.00 (m, 3H), 7.85 - 7.62 (m, 3H), 7.25 - 7.14 (m, 1H), 6.94 (d, *J* = 7.7 Hz, 1H), 5.11 - 4.98 (m, 1H), 4.79 (d, *J* = 5.8 Hz, 1H), 4.17 (d, *J* = 14.6 Hz, 1H), 4.10 - 3.96 (m, 2H), 3.60 - 3.48 (m, 2H), 3.32 (s, 3H), 3.18 - 3.06 (m, 2H), 2.92 (s, 3H), 2.58 - 2.39 (m, 2H), 1.19 (s, 3H). |
| 58 | ¹H NMR (300 MHz, Chloroform-*d*) δ 8.96 (s, 1H), 8.12 - 8.01 (m, 3H), 7.82 - 7.67 (m, 3H), 7.21 - 7.14 (m, 1H), 6.94 (d, *J* = 7.7 Hz, 1H), 4.79 (d, *J* = 5.7 Hz, 1H), 4.31 - 4.17 (m, 2H), 4.05 - 3.93 (m, 2H), 3.29 (s, 3H), 3.19 (s, 3H), 2.42 (d, *J* = 13.4 Hz, 2H), 1.99 - 1.80 (m, 6H), 1.18 (s, 3H). |
| 59 | ¹H NMR (300 MHz, Chloroform-*d*) δ 8.18 (s, 1H), 8.03 (dd, *J* = 7.6, 3.6 Hz, 2H), 7.82 (d, *J* = 7.7 Hz, 1H), 7.71 (dd, *J* = 8.3, 7.0 Hz, 1H), 7.33 (d, *J* = 4.0 Hz, 1H), 7.15 (d, *J* = 4.0 Hz, 1H), 6.90 (d, *J* = 7.7 Hz, 1H), 3.99 (t, *J* = 6.8 Hz, 2H), 3.73 - 3.62 (m, 1H), 3.41 (t, *J* = 5.9 Hz, 3H), 3.32 (s, 3H), 3.21 - 3.09 (m, 1H), 2.94 - 2.81 (m, 2H), 2.06 - 1.75 (m, 6H). |
| 60 | ¹H NMR (300 MHz, Chloroform-*d*) δ 8.87 (s, 1H), 8.09 (d, *J* = 7.0 Hz, 1H), 8.03 (d, *J* = 8.0 Hz, 2H), 7.78 (dd, *J* = 8.3, 7.0 Hz, 1H), 7.47 (d, *J* = 4.0 Hz, 1H), 7.14 (s, 1H), 6.96 (d, *J* = 7.7 Hz, 1H), 4.75 (d, *J* = 5.5 Hz, 1H), 4.17 - 4.07 (m, 1H), 4.02 (t, *J* = 6.8 Hz, 2H), 3.42 (t, *J* = 5.9 Hz, 2H), 3.34 (s, 3H), 3.26 (t, *J* = 13.0 Hz, 1H), 2.45 (d, *J* = 13.5 Hz, 1H), 2.12 - 1.97 (m, 2H), 1.73 - 1.58 (m, 3H), 1.53 (s, 1H), 1.37 (dt, *J* = 12.3, 6.6 Hz, 2H), 1.29 - 1.23 (m, 1H). |
| 61 | ¹H NMR (300 MHz, Chloroform-*d*) δ 8.11 - 7.96 (m, 3H), 7.79 (d, *J* = 7.7 Hz, 1H), 7.71 (dd, *J* = 8.3, 7.0 Hz, 1H), 7.36 (s, 1H), 6.90 (d, *J* = 7.7 Hz, 1H), 3.99 (t, *J* = 6.8 Hz, 2H), 3.75 (d, *J* = 9.8 Hz, 1H), 3.52 (d, *J* = 11.7 Hz, 1H), 3.41 (t, *J* = 5.9 Hz, 2H), 3.33 (s, 3H), 3.18 - 3.05 (m, 1H), 2.84 (t, *J* = 9.7 Hz, 2H), 2.09 - 1.76 (m, 6H). |
| 62 | ¹H NMR (300 MHz, Chloroform-*d*) δ 9.11 (s, 1H), 8.28 (d, *J* = 8.0 Hz, 1H), 8.06 (d, *J* = 7.0 Hz, 1H), 8.02 - 7.93 (m, 2H), 7.89 (d, *J* = 8.2 Hz, 1H), 7.81 - 7.68 (m, 2H), 6.91 (d, *J* = 7.7 Hz, 1H), 4.00 (t, *J* = 6.8 Hz, 2H), 3.86 (d, *J* = 11.2 Hz, 1H), 3.64 (d, *J* = 11.4 Hz, 1H), 3.42 (t, *J* = 5.9 Hz, 2H), 3.33 (s, 3H), 3.10 - 2.97 (m, 1H), 2.79 (q, *J* = 10.5 Hz, 2H), 2.08 - 1.78 (m, 6H). |
| 63 | ¹H NMR (300 MHz, Chloroform-*d*) δ 8.57 (s, 1H), 8.09 (d, *J* = 8.2 Hz, 1H), 8.00 (d, *J* = 6.9 Hz, 1H), 7.80 (d, *J* = 7.7 Hz, 1H), 7.68 (dd, *J* = 8.2, 7.0 Hz, 1H), 7.51 (dd, *J* = 11.4, 1.4 Hz, 2H), 6.87 (d, *J* = 7.7 Hz, 1H), 3.98 (t, *J* = 6.7 Hz, 2H), 3.77 (s, 3H), 3.75 - 3.66 (m, 1H), 3.55 - 3.44 (m, 1H), 3.41 (t, *J* = 6.0 Hz, 2H), 3.32 (s, 3H), 3.14 (t, *J* = 9.9 Hz, 1H), 2.88 (s, 1H), 2.02 (p, *J* = 6.5 Hz, 3H), 1.97 - 1.89 (m, 3H), 1.85 (d, *J* = 5.0 Hz, 1H). |
| 64 | ¹H NMR (500 MHz, Chloroform-*d*) δ 8.90 (s, 1H), 8.14 (s, 1H), 8.04 (dd, *J* = 14.5, 7.6 Hz, 2H), 7.81 (d, *J* = 7.7 Hz, 1H), 7.72 (t, *J* = 7.6 Hz, 1H), 6.91 (d, *J* = 7.7 Hz, 1H), 4.00 (t, *J* = 6.8 Hz, 2H), 3.77 (d, *J* = 11.0 Hz, 1H), 3.55 (d, *J* = 11.6 Hz, 1H), 3.42 (t, *J* = 6.0 Hz, 2H), 3.33 (s, 3H), 3.22 (t, *J* = 10.4 Hz, 1H), 2.95 (t, *J* = 10.7 Hz, 1H), 2.87 (dt, *J* = 8.7, 4.6 Hz, 1H), 2.76 (s, 3H), 2.03 (p, *J* = 6.1 Hz, 3H), 1.94 (t, *J* = 11.0 Hz, 2H), 1.82 (dd, *J* = 9.9, 3.6 Hz, 1H). |
| 65 | ¹H NMR (300 MHz, Chloroform-*d*) δ 8.32 (s, 1H), 8.09 (dd, *J* = 15.1, 7.6 Hz, 2H), 7.90 (d, *J* = 7.7 Hz, 1H), 7.75 (t, *J* = 7.6 Hz, 1H), 7.24 (s, 1H), 6.93 (d, *J* = 7.7 Hz, 1H), 4.01 (t, *J* = 6.7 Hz, 2H), 3.50 (d, *J* = 11.5 Hz, 1H), 3.42 (t, *J* = 6.0 Hz, 2H), 3.33 (s, 5H), 3.11 - 2.99 (m, 1H), 2.93 - 2.84 (m, 1H), 2.79 (d, *J* = 6.2 Hz, 2H), 2.64 (t, *J* = 5.8 Hz, 2H), 2.04 (p, *J* = 6.4 Hz, 2H), 1.98 - 1.74 (m, 8H). |
| 66 | ¹H NMR (300 MHz, Chloroform-*d*)) δ 8.10 (s, 1H), 7.99 (dd, *J* = 13.5, 7.6 Hz, 2H), 7.74 (d, *J* = 7.7 Hz, 1H), 7.68 (dd, *J* = 8.3, 7.0 Hz, 1H), 6.88 (d, *J* = 7.7 Hz, 1H), 4.08 - 3.92 (m, 3H), 3.79 (d, *J* = 12.2 Hz, 1H), 3.41 (t, *J* = 5.9 Hz, 2H), 3.32 (s, 4H), 3.04 (t, *J* = 11.7 Hz, 1H), 2.84 (dt, *J* = 10.0, 5.5 Hz, 1H), 2.18 - 2.06 (m, 1H), 2.02 (t, *J* = 6.3 Hz, 2H), 1.98 - 1.86 (m, 2H), 1.80 (t, *J* = 11.7 Hz, 1H). |
| 67 | ¹H NMR (300 MHz, Chloroform-*d*) δ 10.45 (s, 1H), 8.26 (d, *J* = 8.3 Hz, 1H), 8.08 (d, *J* = 6.9 Hz, 1H), 7.90 (d, *J* = 7.7 Hz, 1H), 7.72 (dd, *J* = 8.3, 7.0 Hz, 1H), 7.57 (d, *J* = 1.4 Hz, 1H), 7.31 (d, *J* = 1.3 Hz, 1H), 6.97 (d, *J* = 7.7 Hz, 1H), 5.01 (s, 1H), 4.02 (t, *J* = 6.8 Hz, 2H), 3.88 (d, *J* = 14.2 Hz, 1H), 3.75 (s, 3H), 3.43 (t, *J* = 6.0 Hz, 2H), 3.34 (s, 3H), 3.09 (t, *J* = 12.2 Hz, 1H), 2.61 (d, *J* = 11.5 Hz, 1H), 2.05 (p, *J* = 6.3 Hz, 2H), 1.86 - 1.61 (m, 5H), 1.26 (s, 2H). |
| 68 | ¹H NMR (300 MHz, Chloroform-*d*) δ 8.11 - 7.97 (m, 3H), 7.92 (d, *J* = 4.6 Hz, 1H), 7.79 (d, *J* = 7.7 Hz, 1H), 7.72 (t, *J* = 7.6 Hz, 1H), 7.07 (d, *J* = 4.5 Hz, 1H), 6.90 (d, *J* = 7.7 Hz, 1H), 3.99 (t, *J* = 6.8 Hz, 2H), 3.85 (dd, *J* = 12.2, 3.9 Hz, 1H), 3.63 (d, *J* = 12.2 Hz, 1H), 3.41 (t, *J* = 5.9 Hz, 2H), 3.33 (s, 4H), 3.06 (t, *J* = 10.1 Hz, 1H), 2.83 (t, *J* = 4.4 Hz, 1H), 1.99 (dt, *J* = 25.9, 8.6 Hz, 5H), 1.86 - 1.69 (m, 1H). |
| 69 | ¹H NMR (300 MHz, Chloroform-*d*) δ 8.32 (s, 1H), 8.29 - 8.21 (m, 2H), 8.04 (t, *J* = 7.2 Hz, 2H), 7.71 (ddd, *J* = 15.2, 12.2, 7.4 Hz, 3H), 6.89 (d, *J* = 7.6 Hz, 1H), 3.99 (t, *J* = 6.7 Hz, 3H), 3.76 (d, *J* = 12.4 Hz, 1H), 3.41 (t, *J* = 5.9 Hz, 3H), 3.33 (s, 3H), 3.06 (t, *J* = 10.2 Hz, 1H), 2.94 - 2.83 (m, 1H), 2.09 - 1.74 (m, 6H). |
| 70 | ¹H NMR (300 MHz, Chloroform-*d*) δ 8.10 (s, 1H), 7.99 (dd, *J* = 13.5, 7.6 Hz, 2H), 7.74 (d, *J* = 7.7 Hz, 1H), 7.68 (dd, *J* = 8.3, 7.0 Hz, 1H), 6.88 (d, *J* = 7.7 Hz, 1H), 4.08 - 3.92 (m, 3H), 3.79 (d, *J* = 12.2 Hz, 1H), 3.41 (t, *J* = 5.9 Hz, 2H), 3.32 (s, 4H), 3.04 (t, *J* = 11.7 Hz, 1H), 2.84 (dt, *J* = 10.0, 5.5 Hz, 1H), 2.18 - 2.06 (m, 1H), 2.02 (t, *J* = 6.3 Hz, 2H), 1.98 - 1.86 (m, 2H), 1.80 (t, *J* = 11.7 Hz, 1H). |
| 71 | ¹H NMR (300 MHz, Chloroform-*d*) δ 8.26 (s, 1H), 8.00 (dd, *J* = 7.6, 2.5 Hz, 2H), 7.76 (d, *J* = 7.7 Hz, 1H), 7.66 (dd, *J* = 8.3, 7.0 Hz, 1H), 7.05 (s, 1H), 6.87 (d, *J* = 7.7 Hz, 1H), 3.97 (t, *J* = 6.8 Hz, 2H), 3.88 (dd, *J* = 12.1, 3.7 Hz, 1H), 3.65 (d, *J* = 12.0 Hz, 1H), 3.40 (t, *J* = 5.9 Hz, 2H), 3.32 (s, 3H), 3.21 (dd, *J* = 12.1, 9.2 Hz, 1H), 3.00 - 2.81 (m, 2H), 2.14 - 1.74 (m, 6H). |
| 72 | ¹H NMR (300 MHz, Chloroform-*d*) δ 8.14 (d, *J* = 8.2 Hz, 1H), 8.02 (d, *J* = 7.0 Hz, 1H), 7.79 - 7.65 (m, 3H), 7.56 (d, *J* = 1.2 Hz, 1H), 6.90 (d, *J* = 7.7 Hz, 1H), 3.97 (t, *J* = 6.8 Hz, 3H), 3.59 (d, *J* = 12.5 Hz, 1H), 3.40 (t, *J* = 5.9 Hz, 2H), 3.30 (s, 3H), 3.12 (q, *J* = 9.8, 8.7 Hz, 1H), 3.01 - 2.78 (m, 2H), 2.07 - 1.70 (m, 6H). |
| 73 | ¹H NMR (300 MHz, Chloroform-*d*) δ 8.11 (d, *J* = 8.1 Hz, 1H), 8.07 - 7.95 (m, 2H), 7.80 - 7.64 (m, 2H), 6.92 (d, *J* = 7.6 Hz, 1H), 4.06 - 3.90 (m, 3H), 3.66 (d, *J* = 12.6 Hz, 1H), 3.37 (dd, *J* = 13.7, 7.6 Hz, 3H), 3.28 (s, 3H), 3.18 (t, *J* = 11.5 Hz, 1H), 2.09 - 1.51 (m, 8H). |
| 74 | ¹H NMR (300 MHz, Chloroform-*d*)) δ 8.71 (d, *J* = 2.2 Hz, 1H), 8.28 (d, *J* = 2.2 Hz, 1H), 8.07 (d, *J* = 7.0 Hz, 1H), 7.99 (d, *J* = 8.3 Hz, 1H), 7.89 (s, 1H), 7.83 - 7.71 (m, 2H), 6.93 (d, *J* = 7.7 Hz, 1H), 4.00 (t, *J* = 6.8 Hz, 2H), 3.81 (d, *J* = 11.2 Hz, 1H), 3.59 (d, *J* = 11.5 Hz, 1H), 3.42 (t, *J* = 6.0 Hz, 2H), 3.33 (s, 3H), 3.15 - 3.04 (m, 1H), 2.82 (s, 2H), 2.04 (p, *J* = 6.6 Hz, 3H), 1.90 (dd, *J* = 23.1, 10.9 Hz, 3H). |
| 75 | ¹H NMR (500 MHz, Chloroform-o) δ 9.01 (d, *J* = 4.1 Hz, 1H), 8.58 (s, 1H), 8.49 (d, *J* = 7.4 Hz, 1H), 8.22 (d, *J* = 8.3 Hz, 1H), 8.09 (d, *J* = 8.3 Hz, 1H), 8.04 (d, *J* = 8.2 Hz, 1H), 8.00 (d, *J* = 7.0 Hz, 1H), 7.75 (d, *J* = 7.7 Hz, 1H), 7.63 (q, *J* = 7.1 Hz, 2H), 7.49 (dd, *J* = 8.6, 4.2 Hz, 1H), 6.88 (d, *J* = 7.6 Hz, 1H), 3.98 (d, *J* = 7.1 Hz, 3H), 3.72 (d, *J* = 12.2 Hz, 1H), 3.59 (t, *J* = 10.6 Hz, 1H), 3.41 (t, *J* = 6.0 Hz, 2H), 3.32 (d, *J* = 1.7 Hz, 3H), 3.28 (s, 1H), 2.92 (s, 1H), 2.03 (t, *J* = 6.4 Hz, 2H), 1.99 (d, *J* = 6.1 Hz, 2H), 1.88 (s, 1H), 1.81 (s, 1H). |
| 76 | ¹H NMR (300 MHz, Chloroform-*d*) δ 8.38 (s, 1H), 8.33 - 8.25 (m, 1H), 8.22 (s, 1H), 8.04 (dd, *J* = 13.0, 7.6 Hz, 2H), 7.95 - 7.88 (m, 1H), 7.81 (d, *J* = 7.7 Hz, 1H), 7.67 (dd, *J* = 8.5, 6.9 Hz, 1H), 7.48 (tt, *J* = 7.3, 5.5 Hz, 2H), 6.88 (d, *J* = 7.7 Hz, 1H), 3.99 (t, *J* = 6.8 Hz, 2H), 3.74 (d, *J* = 12.3 Hz, 1H), 3.52 (dd, *J* = 13.0, 6.0 Hz, 1H), 3.41 (t, *J* = 5.9 Hz, 2H), 3.32 (s, 4H), 3.00 (t, *J* = 10.0 Hz, 1H), 2.87 (d, *J* = 3.9 Hz, 1H), 2.03 (p, *J* = 6.5 Hz, 2H), 1.97 - 1.73 (m, 4H). |
| 77 | ¹H NMR (300 MHz, Chloroform-*d*) δ 8.12 (s, 1H), 8.06 (dd, *J* = 7.6, 6.2 Hz, 2H), 7.84 (d, *J* = 7.7 Hz, 1H), 7.72 (d, *J* = 12.6 Hz, 3H), 6.92 (d, *J* = 7.7 Hz, 1H), 4.00 (t, *J* = 6.8 Hz, 2H), 3.63 (d, *J* = 12.0 Hz, 1H), 3.42 (t, *J* = 5.9 Hz, 3H), 3.33 (s, 3H), 3.22 - 3.08 (m, 1H), 2.87 (d, *J* = 10.6 Hz, 2H), 2.11 - 1.87 (m, 6H). |
| 78 | ¹H NMR (300 MHz, Chloroform-*d*) δ 8.83 (s, 1H), 8.14 - 7.95 (m, 4H), 7.79 (dd, *J* = 8.3, 7.0 Hz, 1H), 7.14 (d, *J* = 4.5 Hz, 1H), 6.96 (d, *J* = 7.7 Hz, 1H), 4.83 (d, *J* = 5.5 Hz, 1H), 4.12 (d, *J* = 7.2 Hz, 1H), 4.02 (t, *J* = 6.8 Hz, 2H), 3.44 (d, *J* = 6.0 Hz, 2H), 3.34 (s, 4H), 2.48 (d, *J* = 13.5 Hz, 1H), 2.10 - 1.99 (m, 2H), 1.64 (d, *J* = 17.1 Hz, 3H), 1.47 - 1.20 (m, 4H). |
| 79 | ¹H NMR (300 MHz, Chloroform-*d*) δ 9.37 (s, 1H), 8.45 (dd, *J* = 7.0, 1.0 Hz, 1H), 8.35 - 8.28 (m, 1H), 8.07 (dd, *J* = 13.0, 7.6 Hz, 2H), 7.94 (d, *J* = 7.7 Hz, 1H), 7.77 (ddd, *J* = 13.8, 8.5, 7.0 Hz, 2H), 6.96 (d, *J* = 7.7 Hz, 1H), 5.24 (d, *J* = 5.4 Hz, 1H), 4.19 - 3.97 (m, 3H), 3.43 (t, *J* = 6.0 Hz, 2H), 3.34 (s, 3H), 3.32 - 3.19 (m, 1H), 2.47 (d, *J* = 13.5 Hz, 1H), 2.17 - 1.95 (m, 3H), 1.63 (s, 1H), 1.55 (s, 1H), 1.27 (q, *J* = 9.9, 7.0 Hz, 4H). |
| 80 | ¹H NMR (300 MHz, Chloroform-*d*) δ 8.75 (s, 1H), 8.09 (d, *J* = 7.0 Hz, 1H), 8.01 (d, *J* = 8.0 Hz, 2H), 7.78 (dd, *J* = 8.3, 7.0 Hz, 1H), 7.50 (s, 1H), 6.96 (d, *J* = 7.7 Hz, 1H), 4.75 (d, *J* = 5.5 Hz, 1H), 4.16 - 4.06 (m, 1H), 4.02 (t, *J* = 6.8 Hz, 2H), 3.42 (t, *J* = 6.0 Hz, 2H), 3.34 (s, 4H), 2.47 (d, *J* = 13.7 Hz, 1H), 2.12 - 1.98 (m, 3H), 1.67 (d, *J* = 16.7 Hz, 3H), 1.53 (d, *J* = 1.5 Hz, 1H), 1.43 (td, *J* = 12.6, 5.6 Hz, 2H), 1.26 (t, *J* = 7.1 Hz, 2H). |
| 81 | ¹H NMR (300 MHz, Chloroform-*d*) δ 8.92 (s, 1H), 8.89 (s, 1H), 8.13 - 7.99 (m, 3H), 7.84 - 7.74 (m, 1H), 6.96 (d, *J* = 7.8 Hz, 1H), 4.81 (d, *J* = 5.6 Hz, 1H), 4.13 (d, *J* = 14.2 Hz, 1H), 4.02 (t, *J* = 6.8 Hz, 2H), 3.43 (t, *J* = 5.9 Hz, 2H), 3.34 (s, 4H), 2.81 (s, 3H), 2.49 (d, *J* = 13.4 Hz, 1H), 2.11 - 1.98 (m, 3H), 1.65 (d, *J* = 14.4 Hz, 3H), 1.53 (s, 1H), 1.41 - 1.29 (m, 3H), 1.18 (s, 1H), 0.87 (p, *J* = 8.4, 7.6 Hz, 2H). |
| 82 | ¹H NMR (300 MHz, Chloroform-*d*) δ 9.24 (s, 1H), 8.72 (s, 1H), 8.41 (d, *J* = 7.3 Hz, 1H), 8.15 - 7.87 (m, 4H), 7.84 - 7.76 (m, 1H), 6.96 (d, *J* = 7.7 Hz, 1H), 4.80 (d, *J* = 5.6 Hz, 1H), 4.11 (d, *J* = 11.2 Hz, 1H), 4.02 (t, *J* = 6.7 Hz, 2H), 3.38 (d, *J* = 27.0 Hz, 6H), 2.44 (d, *J* = 13.8 Hz, 1H), 2.05 (p, *J* = 6.3 Hz, 2H), 1.68 (d, *J* = 8.9 Hz, 3H), 1.53 (s, 1H), 1.26 (s, 4H). |
| 83 | ¹H NMR (300 MHz, Chloroform-*d*) δ 9.07 (s, 1H), 8.12 - 8.04 (m, 3H), 7.78 (dd, *J* = 8.3, 7.0 Hz, 1H), 7.50 - 7.40 (m, 2H), 7.03 (d, *J* = 8.4 Hz, 1H), 6.96 (d, *J* = 7.7 Hz, 1H), 4.71 (d, *J* = 5.5 Hz, 1H), 4.34 (q, *J* = 5.3 Hz, 4H), 4.16 - 3.94 (m, 4H), 3.42 (t, *J* = 6.0 Hz, 2H), 3.34 (s, 3H), 3.18 (s, 1H), 2.40 (d, *J* = 13.7 Hz, 1H), 2.12 - 1.98 (m, 3H), 1.61 (s, 1H), 1.34 - 1.21 (m, 5H). |
| 84 | ¹H NMR (300 MHz, Chloroform-*d*) δ 9.01 (s, 1H), 8.12 - 8.02 (m, 3H), 7.77 (dd, *J* = 8.3, 7.1 Hz, 1H), 7.38 (s, 1H), 6.95 (d, *J* = 7.7 Hz, 1H), 4.75 (d, *J* = 5.4 Hz, 1H), 4.12 (d, *J* = 14.2 Hz, 1H), 4.02 (t, *J* = 6.7 Hz, 2H), 3.42 (t, *J* = 6.0 Hz, 2H), 3.33 (s, 3H), 3.21 (t, *J* = 12.2 Hz, 1H), 2.81 (t, *J* = 5.9 Hz, 2H), 2.64 (t, *J* = 6.0 Hz, 2H), 2.43 (d, *J* = 13.2 Hz, 1H), 2.05 (p, *J* = 6.4 Hz, 2H), 1.84 (dd, *J* = 11.5, 6.7 Hz, 4H), 1.67 (s, 2H), 1.51 - 1.33 (m, 3H), 1.25 (d, *J* = 8.5 Hz, 2H). |
| 85 | ¹H NMR (300 MHz, Chloroform-*d*) δ 8.95 (s, 1H), 8.16 - 8.00 (m, 5H), 7.84 - 7.72 (m, 2H), 7.53 (d, *J* = 8.7 Hz, 1H), 6.96 (d, *J* = 7.7 Hz, 1H), 6.58 (d, *J* = 9.6 Hz, 1H), 4.75 (d, *J* = 5.5 Hz, 1H), 4.12 (d, *J* = 14.1 Hz, 1H), 4.02 (t, *J* = 6.8 Hz, 2H), 3.43 (t, *J* = 6.0 Hz, 2H), 3.34 (d, *J* = 0.8 Hz, 4H), 2.41 (d, *J* = 13.7 Hz, 1H), 2.05 (p, *J* = 6.4 Hz, 2H), 1.62 (s, 2H), 1.21 (s, 2H). |
| 86 | ¹H NMR (300 MHz, Chloroform-*d*) δ 8.91 (s, 1H), 8.13 - 7.86 (m, 4H), 7.79 - 7.70 (m, 1H), 6.99 - 6.88 (m, 1H), 4.75 (dd, *J* = 12.3, 5.2 Hz, 1H), 4.01 (t, *J* = 6.8 Hz, 3H), 3.43 (t, *J* = 5.9 Hz, 2H), 3.37 - 3.19 (m, 4H), 2.42 (d, *J* = 13.7 Hz, 1H), 2.04 (t, *J* = 6.4 Hz, 2H), 1.62 (d, *J* = 16.4 Hz, 3H), 1.26 (d, *J* = 9.5 Hz, 2H). |
| 87 | ¹H NMR (300 MHz, Chloroform-*d*) δ 9.91 (s, 1H), 8.81 (dd, *J* = 4.3, 1.8 Hz, 1H), 8.69 (dd, *J* = 7.4, 1.5 Hz, 1H), 8.26 (dd, *J* = 8.4, 1.8 Hz, 1H), 8.14 (dd, *J* = 8.2, 1.4 Hz, 1H), 8.03 (d, *J* = 7.0 Hz, 1H), 7.86 (d, *J* = 7.7 Hz, 1H), 7.81 - 7.67 (m, 2H), 7.58 (dd, *J* = 8.3, 7.0 Hz, 1H), 7.40 (dd, *J* = 8.3, 4.3 Hz, 1H), 6.95 (d, *J* = 7.7 Hz, 1H), 5.39 (d, *J* = 4.5 Hz, 1H), 4.01 (t, *J* = 6.8 Hz, 3H), 3.43 (t, *J* = 6.0 Hz, 2H), 3.34 (s, 3H), 3.28 - 3.09 (m, 1H), 2.52 (d, *J* = 11.4 Hz, 1H), 2.05 (p, *J* = 6.4 Hz, 2H), 1.66 - 1.37 (m, 5H). |
| 88 | 1H NMR (300 MHz, Chloroform-*d*) δ 8.83 (d, *J* = 2.2 Hz, 1H), 8.78 (s, 1H), 8.39 (d, *J* = 2.2 Hz, 1H), 8.07 (d, *J* = 7.0 Hz, 1H), 8.00 (d, *J* = 8.2 Hz, 1H), 7.90 (d, *J* = 7.7 Hz, 1H), 7.75 (dd, *J* = 8.3, 7.0 Hz, 1H), 6.94 (d, *J* = 7.7 Hz, 1H), 4.83 (d, *J* = 5.5 Hz, 1H), 4.02 (t, *J* = 6.8 Hz, 3H), 3.44 (t, *J* = 6.0 Hz, 2H), 3.35 (s, 3H), 2.44 (d, *J* = 13.6 Hz, 1H), 2.06 (p, *J* = 6.4 Hz, 2H), 1.80 - 1.57 (m, 4H), 1.50 - 1.30 (m, 2H). |
| 89 | ¹H NMR (300 MHz, Chloroform-*d*) δ 8.87 (s, 1H), 8.13 - 8.00 (m, 3H), 7.78 (dd, *J* = 8.3, 7.0 Hz, 1H), 7.21 (s, 1H), 6.96 (d, *J* = 7.8 Hz, 1H), 4.83 (d, *J* = 5.4 Hz, 1H), 4.11 (d, *J* = 14.9 Hz, 1H), 4.02 (t, *J* = 6.8 Hz, 2H), 3.43 (t, *J* = 6.0 Hz, 2H), 3.34 (s, 4H), 2.51 (d, *J* = 13.8 Hz, 1H), 2.05 (p, *J* = 6.5 Hz, 2H), 1.77 - 1.61 (m, 3H), 1.42 (t, *J* = 6.3 Hz, 2H). |
| 90 | ¹H NMR (300 MHz, Chloroform-*d*) δ 8.05 (d, *J* = 6.9 Hz, 1H), 7.86 (d, *J* = 8.3 Hz, 1H), 7.72 (dd, *J* = 7.6, 4.9 Hz, 2H), 7.64 (d, *J* = 5.0 Hz, 1H), 7.57 (d, *J* = 3.7 Hz, 1H), 7.52 (s, 1H), 7.20 - 7.13 (m, 1H), 6.91 (d, *J* = 7.7 Hz, 1H), 3.99 (t, *J* = 6.8 Hz, 2H), 3.88 (d, *J* = 11.8 Hz, 2H), 3.41 (t, *J* = 5.9 Hz, 2H), 3.32 (s, 3H), 2.70 - 2.56 (m, 2H), 2.44 (dt, *J* = 10.6, 6.0 Hz, 1H), 2.21 - 1.97 (m, 6H). |
| 91 | ¹H NMR (300 MHz, DMSO-*d*₆) δ 9.94 (s, 1H), 8.22 (d, *J* = 8.3 Hz, 1H), 8.06 (d, *J* = 6.9 Hz, 1H), 7.81 (dd, *J* = 8.3, 7.0 Hz, 1H), 7.70 (d, *J* = 7.7 Hz, 1H), 7.61 (d, *J* = 4.1 Hz, 1H), 7.40 (d, *J* = 4.1 Hz, 1H), 7.12 (d, *J* = 7.7 Hz, 1H), 3.92 (t, *J* = 6.9 Hz, 2H), 3.68 (d, *J* = 11.8 Hz, 2H), 3.36 (d, *J* = 6.2 Hz, 2H), 3.21 (s, 3H), 2.58 (t, *J* = 11.4 Hz, 3H), 2.03 (d, *J* = 13.2 Hz, 2H), 1.91 (p, *J* = 6.5 Hz, 2H), 1.85 - 1.67 (m, 2H). |
| 92 | ¹H NMR (300 MHz, Chloroform-*d*) δ 8.06 (d, *J* = 6.9 Hz, 1H), 7.86 (d, *J* = 8.2 Hz, 1H), 7.78 - 7.68 (m, 2H), 7.46 (s, 1H), 7.30 (d, *J* = 4.3 Hz, 1H), 6.98 (d, *J* = 8.4 Hz, 1H), 6.92 (d, *J* = 7.7 Hz, 1H), 4.39 - 4.26 (m, 4H), 4.00 (t, *J* = 6.8 Hz, 2H), 3.82 (d, *J* = 11.6 Hz, 2H), 3.41 (t, *J* = 6.0 Hz, 2H), 3.32 (s, 3H), 2.57 (t, *J* = 11.3 Hz, 2H), 2.40 (d, *J* = 11.7 Hz, 1H), 2.16 - 1.93 (m, 6H). |
| 93 | 1H NMR (300 MHz, Chloroform-d) δ 8.07 (d, *J* = 6.9 Hz, 1H), 7.94 (d, *J* = 4.5 Hz, 1H), 7.87 (d, *J* = 8.3 Hz, 1H), 7.79 - 7.68 (m, 2H), 7.49 (s, 1H), 7.08 (d, *J* = 4.6 Hz, 1H), 6.93 (d, *J* = 7.7 Hz, 1H), 3.98 (q, *J* = 10.1, 8.5 Hz, 4H), 3.41 (t, *J* = 5.9 Hz, 2H), 3.32 (s, 3H), 2.89 (t, *J* = 11.6 Hz, 2H), 2.49 (d, *J* = 10.8 Hz, 1H), 2.22 - 1.95 (m, 6H). |
| 94 | ¹H NMR (300 MHz, Chloroform-*d*) δ 8.53 (d, *J* = 2.1 Hz, 1H), 8.07 - 7.94 (m, 3H), 7.81 - 7.66 (m, 2H), 7.19 (d, *J* = 8.7 Hz, 1H), 6.90 (d, *J* = 7.7 Hz, 1H), 4.72 (d, *J* = 5.3 Hz, 1H), 3.94 (t, *J* = 6.7 Hz, 3H), 3.27 (s, 3H), 3.18 (d, *J* = 7.3 Hz, 3H), 2.26 (d, *J* = 13.4 Hz, 1H), 1.98 (t, *J* = 6.4 Hz, 2H), 1.63 - 1.22 (m, 6H). |
| 95 | ¹H NMR (300 MHz, Chloroform-o) δ 9.04 (s, 1H), 8.37 (s, 1H), 8.28 - 8.19 (m, 1H), 8.06 (d, *J* = 7.0 Hz, 1H), 7.96 (ddd, *J* = 9.6, 7.5, 1.7 Hz, 3H), 7.72 (dd, *J* = 8.3, 7.0 Hz, 1H), 7.59 - 7.44 (m, 2H), 6.92 (d, *J* = 7.7 Hz, 1H), 4.84 (d, *J* = 5.4 Hz, 1H), 4.27 - 4.13 (m, 1H), 4.00 (t, *J* = 6.8 Hz, 2H), 3.42 (t, *J* = 6.0 Hz, 2H), 3.33 (s, 3H), 3.31 - 3.20 (m, 1H), 2.38 (d, *J* = 13.6 Hz, 1H), 2.04 (p, *J* = 6.4 Hz, 2H), 1.52 (dd, *J* = 10.1, 3.1 Hz, 3H), 1.21 - 1.01 (m, 2H). |
| 96 | ¹H NMR (300 MHz, Chloroform-*d*) δ 8.06 (d, *J* = 6.9 Hz, 1H), 7.86 (d, *J* = 8.2 Hz, 1H), 7.78 - 7.67 (m, 2H), 7.59 (d, *J* = 10.3 Hz, 2H), 7.48 (s, 1H), 6.89 (dd, *J* = 12.8, 8.0 Hz, 2H), 4.69 (t, *J* = 8.8 Hz, 2H), 3.99 (t, *J* = 6.8 Hz, 2H), 3.81 (d, *J* = 11.5 Hz, 2H), 3.41 (t, *J* = 6.0 Hz, 2H), 3.32 (s, 5H), 2.55 (t, *J* = 10.9 Hz, 2H), 2.39 (d, *J* = 11.0 Hz, 1H), 2.19 - 1.94 (m, 6H). |
| 97 | ¹H NMR (300 MHz, Chloroform-*d*) δ 8.05 (d, *J* = 6.9 Hz, 1H), 7.85 (d, *J* = 8.3 Hz, 1H), 7.71 (q, *J* = 7.6 Hz, 2H), 7.52 (s, 1H), 7.40 (dd, *J* = 8.4, 2.1 Hz, 1H), 7.24 (d, *J* = 2.1 Hz, 1H), 6.97 (d, *J* = 8.4 Hz, 1H), 6.91 (d, *J* = 7.7 Hz, 1H), 4.04 - 3.91 (m, 8H), 3.84 (d, *J* = 11.7 Hz, 2H), 3.41 (t, *J* = 5.9 Hz, 2H), 3.32 (s, 3H), 2.55 (t, *J* = 11.0 Hz, 2H), 2.47 - 2.34 (m, 1H), 2.03 (td, *J* = 14.9, 12.7, 9.0 Hz, 6H). |
| 98 | ¹H NMR (300 MHz, Chloroform-*d*) δ 8.28 (d, *J* = 7.8 Hz, 1H), 8.21 (s, 1H), 8.04 (d, *J* = 6.9 Hz, 1H), 7.92 (d, *J* = 7.6 Hz, 1H), 7.82 (d, *J* = 8.3 Hz, 1H), 7.75 - 7.65 (m, 2H), 7.48 (dd, *J* = 14.5, 7.4 Hz, 3H), 6.90 (d, *J* = 7.6 Hz, 1H), 3.98 (t, *J* = 6.7 Hz, 4H), 3.40 (t, *J* = 5.9 Hz, 2H), 3.32 (s, 3H), 2.72 (t, *J* = 10.8 Hz, 2H), 2.47 - 2.33 (m, 1H), 2.03 (td, *J* = 14.6, 12.4, 8.1 Hz, 6H). |
| 99 | LC/MS : 592.0 |
| 100 | LC/MS : 582.1 |
| 101 | LC/MS : 544.1 |
| 102 | LC/MS : 592.0 |
| 103 | LC/MS : 582.1 |
| 104 | LC/MS : 544.1 |
| 105 | ¹H NMR (300 MHz, Chloroform-*d*) δ 8.16 (s, 1H), 8.09 - 8.01 (m, 2H), 7.98 - 7.87 (m, 2H), 7.81 (d, *J* = 7.7 Hz, 1H), 7.75 (dd, *J* = 8.8, 4.8 Hz, 2H), 7.49 (d, *J* = 8.6 Hz, 1H), 6.91 (d, *J* = 7.7 Hz, 1H), 6.55 (d, *J* = 9.6 Hz, 1H), 3.99 (t, *J* = 6.8 Hz, 2H), 3.72 (d, *J* = 11.4 Hz, 1H), 3.49 (d, *J* = 10.8 Hz, 1H), 3.41 (t, *J* = 5.9 Hz, 2H), 3.33 (s, 3H), 3.07 (t, *J* = 10.2 Hz, 1H), 2.81 (dd, *J* = 21.3, 9.5 Hz, 2H), 2.08 - 1.76 (m, 6H). |
| 106 | ¹H NMR (300 MHz, Chloroform-*d*) δ 9.01 (s, 1H), 8.14 (t, *J* = 6.5 HZ, 2H), 8.06 (d, *J* = 7.7 Hz, 1H), 7.84 (t, *J* = 7.7 Hz, 1H), 7.78 - 7.69 (m, 3H), 7.21 (s, 1H), 6.97 (d, *J* = 7.7 Hz, 1H), 4.81 (s, 1H), 4.32 (t, *J* = 6.1 Hz, 1H), 4.19 (d, *J* = 14.2 Hz, 1H), 3.84 (d, *J* = 11.9 Hz, 2H), 3.28 (t, *J* = 13.8 Hz, 2H), 3.05 (s, 3H), 2.80 (s, 3H), 2.68 (t, *J* = 11.9 Hz, 2H), 2.45 (d, *J* = 13.7 Hz, 1H), 1.87 (d, *J* = 13.5 Hz, 2H), 1.81 - 1.72 (m, 2H), 1.46 - 1.24 (m, 5H). |
| 107 | ¹H NMR (300 MHz, Chloroform-o) δ 9.00 - 8.96 (m, 1H), 8.09 (t, *J* = 8.3 Hz, 2H), 8.04 - 8.00 (m, 1H), 7.78 (d, *J* = 10.5 Hz, 3H), 7.17 (d, *J* = 8.2 Hz, 1H), 6.96 (d, *J* = 7.7 Hz, 1H), 4.74 (d, *J* = 5.5 Hz, 1H), 4.10 (d, *J* = 15.2 Hz, 1H), 4.03 (t, *J* = 6.7 Hz, 2H), 3.44 (t, *J* = 5.9 Hz, 2H), 3.34 (d, *J* = 1.6 Hz, 4H), 3.27 (t, *J* = 13.6 Hz, 1H), 2.39 (d, *J* = 13.8 Hz, 1H), 2.08 - 2.03 (m, 2H), 1.59 - 1.53 (m, 2H), 1.33 - 1.14 (m, 3H). |
| 108 | ¹H NMR (300 MHz, Chloroform-*d*) δ 9.18 (s, 1H), 9.07 (s, 1H), 9.00 (d, *J* = 9.1 Hz, 1H), 8.44 (d, *J* = 8.6 Hz, 1H), 8.12 - 8.04 (m, 3H), 7.77 (t, *J* = 7.9 Hz, 1H), 7.66 (d, *J* = 9.4 Hz, 1H), 7.15 (d, *J* = 8.4 Hz, 1H), 6.96 (d, *J* = 7.6 Hz, 1H), 4.76 (d, *J* = 5.0 Hz, 1H), 4.22 (s, 3H), 4.09 - 3.99 (m, 3H), 3.43 (t, *J* = 5.9 Hz, 2H), 3.34 (d, *J* = 1.5 Hz, 3H), 3.23 (s, 1H), 2.41 (d, *J* = 12.0 Hz, 1H), 2.11 - 2.02 (m, 2H), 1.25 (s, 1H), 1.09 (s, 2H). |
| 109 | ¹H NMR (300 MHz, Methanol-*d₄*) δ 8.45 (d, *J* = 2.1 Hz, 1H), 8.16 (dd, *J* = 8.3, 3.4 Hz, 1H), 8.07 (d, *J* = 7.1 Hz, 1H), 7.96 (dd, *J* = 8.8, 2.1 Hz, 1H), 7.78 (t, *J* = 7.7 Hz, 1H), 7.69 (d, *J* = 7.6 Hz, 1H), 7.32 (d, *J* = 8.7 Hz, 1H), 7.02 (d, *J* = 7.7 Hz, 1H), 4.07 - 3.90 (m, 3H), 3.76 (d, *J* = 11.4 Hz, 1H), 3.45 (t, *J* = 6.0 Hz, 2H), 3.33 (s, 3H), 2.89 (d, *J* = 10.5 Hz, 1H), 2.67 (t, *J* = 11.0 Hz, 1H), 2.51 - 2.38 (m, 1H), 2.16 - 1.74 (m, 6H). |
| 110 | ¹H NMR (300 MHz, Chloroform-*d*) δ 8.96 (s, 1H), 8.13 - 7.99 (m, 3H), 7.79 (ddd, *J* = 8.3, 4.4, 2.5 Hz, 2H), 7.67 (d, *J* = 1.8 Hz, 1H), 7.29 (s, 1H), 6.96 (d, *J* = 7.7 Hz, 1H), 4.76 (d, *J* = 5.5 Hz, 1H), 4.15 - 3.99 (m, 3H), 3.44 (t, *J* = 6.0 Hz, 2H), 3.35 (s, 4H), 2.44 (d, *J* = 13.8 Hz, 1H), 2.07 (p, *J* = 6.4 Hz, 2H), 1.63 (d, *J* = 13.7 Hz, 3H), 1.35 - 1.17 (m, 2H). |
| 111 | ¹H NMR (300 MHz, DMSO-*d₆*) δ 9.93 (s, 1H), 8.22 (d, *J* = 8.3 Hz, 1H), 8.06 (d, *J* = 6.7 Hz, 1H), 7.81 (dd, *J* = 8.3, 7.0 Hz, 1H), 7.70 (d, *J* = 7.7 Hz, 1H), 7.56 (d, *J* = 4.0 Hz, 1H), 7.49 (d, *J* = 4.0 Hz, 1H), 7.12 (d, *J* = 7.7 Hz, 1H), 3.92 (t, *J* = 6.9 Hz, 2H), 3.68 (d, *J* = 11.5 Hz, 2H), 3.36 (d, *J* = 6.2 Hz, 2H), 3.21 (s, 3H), 2.59 (d, *J* = 12.1 Hz, 3H), 2.03 (d, *J* = 13.3 Hz, 2H), 1.91 (p, *J* = 6.5 Hz, 2H), 1.77 (q, *J* = 10.2 Hz, 2H). |
| 112 | ¹H NMR (300 MHz, Chloroform-*d*) δ 9.07 (d, *J* = 3.8 Hz, 1H), 8.50 (d, *J* = 7.3 Hz, 1H), 8.25 (d, *J* = 8.4 Hz, 1H), 8.05 (dd, *J* = 7.5, 3.5 Hz, 2H), 7.87 (d, *J* = 8.3 Hz, 1H), 7.78 - 7.58 (m, 3H), 7.57 - 7.45 (m, 2H), 6.90 (d, *J* = 7.7 Hz, 1H), 4.23 (d, *J* = 12.9 Hz, 2H), 3.99 (t, *J* = 6.8 Hz, 2H), 3.41 (t, *J* = 6.0 Hz, 2H), 3.32 (s, 3H), 3.04 (t, *J* = 12.3 Hz, 2H), 2.48 (t, *J* = 11.5 Hz, 1H), 2.13 - 1.88 (m, 6H). |
| 113 | ¹H NMR (300 MHz, DMSO-*d₆*) δ 9.98 (s, 1H), 8.26 - 8.13 (m, 2H), 8.06 (d, *J* = 7.0 Hz, 1H), 7.87 - 7.77 (m, 1H), 7.68 (d, *J* = 7.7 Hz, 1H), 7.12 (d, *J* = 7.7 Hz, 1H), 3.92 (t, *J* = 6.9 Hz, 2H), 3.75 (d, *J* = 12.0 Hz, 2H), 3.35 (t, *J* = 6.1 Hz, 2H), 3.21 (s, 2H), 3.12 (t, *J* = 11.9 Hz, 1H), 2.73 (t, *J* = 11.4 Hz, 2H), 2.62 (d, *J* = 11.1 Hz, 1H), 2.04 (s, 2H), 1.96 - 1.86 (m, 2H), 1.78 (q, *J* = 10.9 Hz, 2H). |
| 114 | ¹H NMR (300 MHz, Chloroform-*d*) δ 8.06 (d, *J* = 6.9 Hz, 1H), 7.88 (d, *J* = 8.2 Hz, 1H), 7.79 - 7.69 (m, 2H), 7.51 (s, 1H), 7.07 (s, 1H), 6.93 (d, *J* = 7.7 Hz, 1H), 4.04 - 3.89 (m, 4H), 3.41 (t, *J* = 6.0 Hz, 2H), 3.33 (s, 3H), 2.87 (t, *J* = 11.5 Hz, 2H), 2.53 (t, *J* = 10.6 Hz, 1H), 2.20 - 1.95 (m, 6H). |
| 115 | ¹H NMR (300 MHz, Chloroform-*d*) δ 8.25 (d, *J* = 8.7 Hz, 2H), 8.05 (d, *J* = 7.0 Hz, 1H), 7.85 (d, *J* = 8.3 Hz, 1H), 7.71 (d, *J* = 7.8 Hz, 3H), 7.50 (s, 1H), 6.90 (d, *J* = 7.7 Hz, 1H), 4.16 (d, *J* = 12.7 Hz, 2H), 3.98 (t, *J* = 6.8 Hz, 2H), 3.40 (t, *J* = 5.9 Hz, 2H), 3.32 (s, 3H), 2.97 (t, *J* = 11.8 Hz, 2H), 2.48 (s, 1H), 2.20 - 1.87 (m, 6H). |
| 116 | ¹H NMR (300 MHz, Chloroform-*d*) δ 8.37 (s, 1H), 8.03 (dd, *J* = 7.7, 2.5 Hz, 2H), 7.76 - 7.63 (m, 2H), 7.48 (d, *J* = 15.9 Hz, 2H), 6.89 (d, *J* = 7.6 Hz, 1H), 4.02 - 3.86 (m, 4H), 3.76 (s, 3H), 3.40 (t, *J* = 6.0 Hz, 2H), 3.31 (s, 3H), 2.83 (t, *J* = 11.4 Hz, 2H), 2.59 (d, *J* = 11.4 Hz, 1H), 2.10 - 1.96 (m, 6H). |
| 117 | ¹H NMR (300 MHz, Chloroform-o) δ 8.97 (s, 1H), 8.12 - 8.02 (m, 3H), 7.80 (dd, *J* = 8.3, 7.0 Hz, 1H), 7.75 - 7.67 (m, 2H), 7.18 (dd, *J* = 5.0, 3.8 Hz, 1H), 6.93 (d, *J* = 7.7 Hz, 1H), 4.79 (d, *J* = 5.5 Hz, 1H), 4.16 (d, *J* = 15.7 Hz, 1H), 4.00 (t, *J* = 7.1 Hz, 2H), 3.31 - 3.23 (m, 3H), 2.88 (s, 3H), 2.81 (s, 3H), 2.42 (d, *J* = 13.8 Hz, 1H), 2.15 - 2.08 (m, 2H), 1.63 (d, *J* = 11.8 Hz, 2H), 1.37 - 1.23 (m, 4H). |
| 118 | ¹H NMR (400 MHz, Chloroform-*d*) δ 8.97 (s, 1H), 8.62 (ddd, *J* = 4.9, 1.8, 1.0 Hz, 1H), 8.10 - 8.04 (m, 3H), 7.80 - 7.69 (m, 4H), 7.56 (d, *J* = 4.0 Hz, 1H), 7.29 (ddd, *J* = 7.5, 4.9, 1.2 Hz, 2H), 6.95 (d, *J* = 7.7 Hz, 1H), 4.83 (d, *J* = 5.5 Hz, 1H), 4.19 (d, *J* = 14.2 Hz, 1H), 4.02 (t, *J* = 6.8 Hz, 2H), 3.43 (t, *J* = 6.0 Hz, 2H), 3.34 (s, 3H), 3.27 (t, *J* = 12.4 Hz, 1H), 2.44 (d, *J* = 13.7 Hz, 1H), 2.09 - 2.01 (m, 3H), 1.69 - 1.58 (m, 4H), 1.49 - 1.39 (m, 3H), 0.92 - 0.82 (m, 3H). |
| 119 | ¹H NMR (400 MHz, Chloroform-a) δ 8.60 (dt, *J* = 4.7, 1.4 Hz, 1H), 8.19 (s, 1H), 8.09 (dd, *J* = 12.0, 7.6 Hz, 2H), 7.90 (d, *J* = 7.7 Hz, 1H), 7.80 - 7.74 (m, 2H), 7.71 (d, *J* = 7.9 Hz, 1H), 7.57 (q, *J =* 4.0 Hz, 2H), 7.29 - 7.27 (m, 1H), 6.93 (d, *J* = 7.7 Hz, 1H), 4.01 (t, *J* = 6.7 Hz, 2H), 3.58 (d, *J* = 11.5 Hz, 1H), 3.42 (t, *J* = 6.0 Hz, 2H), 3.40 - 3.36 (m, 1H), 3.33 (s, 3H), 3.14 (s, 1H), 2.87 (s, 1H), 2.04 (q, *J* = 6.5 Hz, 2H), 1.96 (d, *J* = 21.5 Hz, 3H), 1.88 - 1.80 (m, 1H). |
| 120 | ¹H NMR (400 MHz, Chloroform-*d*) δ 8.61 (d, *J* = 4.8 Hz, 1H), 8.05 (d, *J* = 6.9 Hz, 1H), 7.86 (d, *J* = 8.2 Hz, 1H), 7.81 - 7.67 (m, 4H), 7.55 (s, 2H), 7.43 (s, 1H), 7.29 (s, 1H), 6.91 (d, *J* = 7.7 Hz, 1H), 3.99 (t, *J* = 6.8 Hz, 2H), 3.92 (d, *J* = 11.4 Hz, 2H), 3.41 (t, *J* = 5.9 Hz, 2H), 3.32 (s, 3H), 2.71 (t, *J* = 10.7 Hz, 2H), 2.42 (d, *J* = 10.4 Hz, 1H), 2.19 - 2.10 (m, 3H), 2.05 - 2.00 (m, 2H). |
| 121 | ¹H NMR (300 MHz, DMSO-*d₆*) δ 10.15 (s, 1H), 8.76 (s, 1H), 8.24 (d, *J* = 8.3 Hz, 1H), 8.07 (d, *J* = 6.9 Hz, 1H), 7.84 - 7.80 (m, 1H), 7.66 (d, *J* = 7.7 Hz, 1H), 7.13 (d, *J* = 7.8 Hz, 1H), 3.92 (t, *J* = 7.0 Hz, 3H), 3.77 (d, *J* = 12.0 Hz, 1H), 3.21 (s, 3H), 3.02 (d, *J* = 7.7 Hz, 4H), 2.86 (d, *J* = 7.9 Hz, 2H), 2.77 - 2.72 (m, 1H), 1.91 (dd, *J* = 7.9, 5.1 Hz, 3H), 1.85 - 1.72 (m, 2H), 1.56 (d, *J* = 8.8 Hz, 2H). |
| 122 | ¹H NMR (300 MHz, Chloroform-*d*) δ 8.55 (s, 1H), 8.19 (d, *J* = 8.3 Hz, 1H), 8.07 (d, *J* = 7.0 Hz, 1H), 7.97 - 7.88 (m, 1H), 7.75 (dd, *J* = 8.3, 7.0 Hz, 1H), 7.61 (dd, *J* = 8.5, 2.4 Hz, 1H), 7.56 (s, 1H), 6.94 (dd, *J* = 9.0, 6.8 Hz, 2H), 4.20 (dd, *J* = 5.7, 3.8 Hz, 2H), 4.01 (t, *J* = 6.7 Hz, 2H), 3.81 (dd, *J* = 5.7, 3.7 Hz, 2H), 3.47 (s, 3H), 3.42 (t, *J* = 5.9 Hz, 3H), 3.33 (s, 3H), 3.15 (s, 1H), 3.03 (s, 1H), 2.89 (s, 1H), 2.29 (s, 3H), 2.07 - 2.01 (m, 2H), 1.93 (d, *J* = 8.1 Hz, 4H). |
| 123 | ¹H NMR (300 MHz, Chloroform-*d*) δ 8.46 (s, 1H), 8.20 (d, *J* = 8.3 Hz, 1H), 8.10 (d, *J* = 7.0 Hz, 1H), 7.96 (d, *J* = 7.7 Hz, 1H), 7.84 - 7.76 (m, 1H), 7.64 (d, *J* = 9.3 Hz, 1H), 7.58 (s, 1H), 6.95 (dd, *J* = 8.2, 3.5 Hz, 2H), 4.18 (t, *J* = 5.7 Hz, 2H), 4.04 (t, *J* = 6.7 Hz, 2H), 3.44 (t, *J* = 6.0 Hz, 3H), 3.36 (s, 3H), 3.12 (s, 2H), 2.84 (t, *J* = 5.7 Hz, 3H), 2.41 (s, 6H), 2.30 (s, 3H), 2.07 (t, *J* = 6.4 Hz, 2H), 1.87 (d, *J* = 15.0 Hz, 4H). |
| 124 | ¹H NMR (300 MHz, Chloroform-*d*) δ 8.64 (s, 1H), 8.11 (d, *J* = 6.9 Hz, 1H), 8.01 (dd, *J* = 7.9, 5.7 Hz, 2H), 7.77 (dd, *J* = 8.2, 7.0 Hz, 1H), 7.71 - 7.63 (m, 1H), 7.54 (dd, *J* = 7.3, 1.6 Hz, 1H), 7.51 - 7.43 (m, 1H), 6.94 (d, *J* = 7.7 Hz, 1H), 4.09 (t, *J* = 6.1 Hz, 2H), 3.61 (s, 3H), 3.53 (q, *J* = 5.6 Hz, 2H), 2.87 (d, *J* = 15.9 Hz, 1H), 2.12 (d, *J* = 11.1 Hz, 1H), 2.00 - 1.92 (m, 2H), 1.68 (s, 2H), 1.49 - 1.40 (m, 2H), 1.25 (s, 4H). |
| 125 | ¹H NMR (300 MHz, Chloroform-*d*) δ 8.07 (d, *J* = 8.3 Hz, 1H), 8.00 (d, *J* = 6.9 Hz, 1H), 7.77 (d, *J* = 7.4 Hz, 2H), 7.58 (d, *J* = 10.1 Hz, 4H), 6.87 (d, *J* = 7.6 Hz, 1H), 6.64 (d, *J* = 8.2 Hz, 1H), 4.85 (s, 1H), 4.73 (t, *J* = 8.9 Hz, 1H), 4.55 (t, J = 8.8 Hz, 3H), 3.42 (t, *J* = 5.9 Hz, 2H), 3.33 (s, 3H), 2.69 (d, *J* = 10.2 Hz, 3H), 2.50 (d, *J* = 7.4 Hz, 2H), 2.04 (d, *J* = 6.1 Hz, 2H). |
| 126 | ¹H NMR (300 MHz, Chloroform-*d*) δ 8.07 (d, *J* = 6.8 Hz, 1H), 8.05 - 7.98 (m, 1H), 7.83 - 7.76 (m, 1H), 7.18 (d, *J* = 3.8 Hz, 1H), 7.10 (d, *J* = 3.9 Hz, 1H), 7.00 (s, 1H), 6.96 (s, 1H), 6.89 (d, *J* = 3.9 Hz, 1H), 4.00 (t, *J* = 6.6 Hz, 2H), 3.42 (t, *J* = 6.0 Hz, 2H), 3.33 (s, 3H), 2.67 (t, *J* = 10.4 Hz, 2H), 2.49 (t, *J* = 6.6 Hz, 2H), 2.08 - 2.00 (m, 2H). |
| 127 | ¹H NMR (300 MHz, Chloroform-*d*) δ 8.10 (ddd, *J* = 6.7, 3.5, 1.5 Hz, 1H), 7.77 - 7.65 (m, 2H), 7.24 (dt, *J* = 5.4, 1.9 Hz, 3H), 7.19 - 7.12 (m, 2H), 6.94 (dd, *J* = 7.5, 5.3 Hz, 1H), 6.84 (dd, *J* = 7.5, 1.6 Hz, 1H), 5.80 (d, *J* = 38.6 Hz, 1H), 5.43 (dd, *J* = 19.5, 13.8 Hz, 1H), 4.50 (dd, *J* = 16.3, 13.8 Hz, 1H), 4.00 (t, *J* = 6.9 Hz, 2H), 3.44 (td, *J* = 6.0, 1.6 Hz, 2H), 3.32 (s, 3H), 2.63 (s, 1H), 2.43 - 2.30 (m, 1H), 2.16 - 2.08 (m, 1H), 2.07 - 2.01 (m, 2H). |
| 128 | ¹H NMR (300 MHz, Chloroform-*d*) δ 8.10 (d, *J* = 6.6 Hz, 1H), 7.78 - 7.69 (m, 2H), 7.66 (d, *J* = 7.8 Hz, 1H), 7.07 (dd, *J* = 8.7, 2.8 Hz, 2H), 6.91 (d, *J* = 7.4 Hz, 1H), 6.85 (d, *J* = 7.5 Hz, 1H), 6.76 (d, *J* = 8.7 Hz, 2H), 5.37 (d, *J* = 16.1 Hz, 1H), 4.43 (d, *J* = 13.3 Hz, 1H), 4.00 (t, *J* = 6.9 Hz, 2H), 3.78 (s, 3H), 3.45 (t, *J* = 6.0 Hz, 2H), 3.33 (s, 3H), 2.43 - 2.30 (m, 2H), 2.15 - 2.10 (m, 1H), 2.05 (s, 2H). |
| 129 | ¹H NMR (300 MHz, Chloroform-*d*) δ 8.49 (d, *J* = 2.6 Hz, 1H), 8.13 (q, *J* = 3.6, 2.6 Hz, 2H), 7.91 (d, *J* = 8.4 Hz, 1H), 7.82 - 7.76 (m, 2H), 7.76 - 7.64 (m, 3H), 6.99 - 6.92 (m, 1H), 6.86 (d, *J* = 7.5 Hz, 1H), 6.46 (dd, *J* = 2.7, 1.6 Hz, 1H), 5.92 (d, *J* = 44.1 Hz, 1H), 5.56 - 5.41 (m, 1H), 4.55 - 4.42 (m, 1H), 4.00 (t, *J* = 6.9 Hz, 2H), 3.44 (t, *J* = 5.9 Hz, 2H), 3.32 (s, 3H), 2.52 - 2.27 (m, 2H), 2.16 - 2.09 (m, 1H), 2.04 (t, *J* = 6.4 Hz, 2H). |
| 130 | ¹H NMR (300 MHz, Chloroform-*d*) δ 8.57 (s, 1H), 8.16 (d, *J* = 8.2 Hz, 1H), 8.05 (d, *J* = 7.0 Hz, 1H), 7.90 (d, *J* = 7.6 Hz, 1H), 7.73 (dd, *J* = 8.3, 1.3 Hz, 1H), 7.63 - 7.55 (m, 2H), 6.96 (d, *J* = 7.7 Hz, 1H), 6.88 (d, *J* = 8.2 Hz, 1H), 4.70 (t, *J* = 8.8 Hz, 3H), 4.07 (t, *J* = 6.6 Hz, 2H), 3.61 (t, *J* = 6.2 Hz, 2H), 3.34 - 3.18 (m, 5H), 2.98 - 2.85 (m, 2H), 2.26 (t, *J* = 6.3 Hz, 2H), 1.96 - 1.85 (m, 3H), 1.84 - 1.76 (m, 1H). |
| 131 | ¹H NMR (400 MHz, DMSO-*d₆*) δ 9.84 (s, 1H), 7.98 (d, *J* = 10.8 Hz, 1H), 7.77 (d, *J* = 10.0 Hz, 2H), 7.52 (t, *J* = 10.8 Hz, 1H), 7.47 - 7.30 (m, 2H), 7.01 (t, *J* = 4.9 Hz, 1H), 6.86 (d, *J* = 10.0 Hz, 1H), 3.77 - 3.45 (m, 3H), 3.32 (d, *J* = 13.5 Hz, 1H), 3.20 (d, *J* = 14.6 Hz, 1H), 2.71 - 2.54 (m, 1H), 2.30 (t, *J* = 14.5 Hz, 1H), 2.23 - 2.10 (m, 3H), 2.03 (t, *J* = 15.1 Hz, 1H), 1.81 - 1.63 (m, 2H), 1.63 - 1.48 (m, 3H), 1.46 - 1.30 (m, 3H), 1.28 - 1.10 (m, 1H), 1.00 - 0.79 (m, 3H). |
| 132 | ¹H NMR (400 MHz, DMSO-*d₆*) δ 9.85 (s, 1H), 7.98 (d, *J* = 11.0 Hz, 1H), 7.77 (d, *J* = 9.2 Hz, 1H), 7.55 (t, *J* = 9.8 Hz, 1H), 7.41 (d, *J* = 10.2 Hz, 1H), 7.30 (d, *J* = 5.4 Hz, 1H), 7.10 (d, *J* = 5.4 Hz, 1H), 6.87 (d, *J* = 10.2 Hz, 1H), 3.63 (t, *J* = 9.1 Hz, 1H), 3.50 (d, *J* = 11.5 Hz, 1H), 3.30 (d, *J* = 15.0 Hz, 1H), 3.20 (d, *J* = 15.5 Hz, 1H), 2.68 - 2.55 (m, 1H), 2.37 - 2.23 (m, 3H), 2.23 - 2.09 (m, 4H), 1.79 - 1.65 (m, 1H), 1.57 (d, *J* = 15.2 Hz, 1H), 1.44 - 1.30 (m, 2H), 1.30 - 1.15 (m, 2H), 1.06 - 0.78 (m, 4H). |
| 133 | ¹H NMR (400 MHz, Chloroform-*d*) δ 8.53 (s, 1H), 8.18 (d, *J* = 8.4 Hz, 1H), 8.06 (d, *J* = 7.2 Hz, 1H), 7.91 (d, *J* = 8.0 Hz, 1H), 7.76 (d, *J* = 7.2, 8.2 Hz, 1H), 7.63 - 7.56 (m, 2H), 6.90 (d, *J* = 8.0 Hz, 1H), 6.85 (d, *J* = 7.6 Hz, 1H), 4.71 (t, *J* = 8.8 Hz, 2H), 3.98 (t, *J* = 6.8 Hz, 2H), 3.78 (d, *J* = 12.0 Hz, 2H), 3.40 - 3.25 (m, 4H), 3.18 - 3.01 (m, 2H), 2.92 - 2.84 (m, 1H), 2.73 (s, 3H), 2.60 (t, *J* = 11.6 Hz, 2H), 2.01 - 1.81 (m, 6H), 1.45 - 1.23 (m, 5H). |
| 134 | ¹H NMR (400 MHz, Chloroform-*d*) δ 9.10 (s, 1H), 8.13 - 8.03 (m, 3H), 7.79 (t, *J* = 7.6 Hz, 1H), 7.48 - 7.40 (m, 2H), 7.03 (d, *J* = 8.4 Hz, 1H), 6.88 (d, *J* = 7.6 Hz, 1H), 4.71 (d, *J* = 5.2 Hz, 1H), 4.34 (dd, *J* = 4.8, 12.4 Hz, 4H), 4.00 (d, *J* = 14.8 Hz, 1H), 3.99 (t, *J* = 6.8 Hz, 2H), 3.78 (d, *J* = 12.0 Hz, 2H), 3.23 - 3.13 (m, 1H), 2.73 (s, 3H), 2.60 (t, *J* = 11.2 Hz, 2H), 2.39 (d, *J* = 13.2 Hz, 1H), 1.95 (d, *J* = 10.4 Hz, 2H), 1.77 (q, *J* = 6.4 Hz, 2H), 1.61 (s, 3H), 1.44 - 1.37 (m, 2H), 1.29 - 1.21 (m, 3H). |
| 135 | ¹H NMR (400 MHz, DMSO-*d*₆) δ 10.09 (s, 1H), 8.58 (s, 1H), 8.21 - 8.08 (m, 3H), 8.06 (d, *J* = 6.8 Hz, 1H), 7.81 (dd, *J* = 7.2, 8.4 Hz, 1H), 7.52 - 7.42 (m, 2H), 7.39 (d, *J* = 7.6 Hz, 1H), 7.10 (d, *J* = 7.6 Hz, 1H), 5.00 (d, *J* = 5.2 Hz, 1H), 3.99 - 3.81 (m, 3H), 3.72 (t, *J* = 10.0 Hz, 1H), 3.51 (d, *J* = 11.6 Hz, 2H), 2.81 (s, 3H), 2.61 (t, *J* = 10.8 Hz, 2H), 2.15 (d, *J* = 10.8 Hz, 1H), 1.86 (d, *J* = 12.4 Hz, 2H), 1.75 - 1.52 (m, 5H), 1.46 - 1.11 (m, 5H). |
| 136 | ¹H NMR (400 MHz, DMSO-*d₆*) δ 10.13 (s, 1H), 8.27 (d, *J* = 8.4 Hz, 1H), 8.07 (dd, *J* = 3.2, 3.6 Hz, 2H), 7.83 (d, *J* = 7.2 Hz, 1H), 7.70 (d, *J* = 7.6 Hz, 1H), 7.26 (d, *J* = 3.6 Hz, 1H), 7.17 (d, *J* = 7.6 Hz, 1H), 6.80 - 6.73 (m, 1H), 3.93 (t, *J* = 6.8 Hz, 2H), 3.86 (d, *J* = 10.4 Hz, 1H), 3.65 (d, *J* = 11.2 Hz, 1H), 3.51 (d, *J* = 12.0 Hz, 2H), 2.92 - 2.80 (m, 1H), 2.71 (t, *J* = 11.2 Hz, 1H), 2.61 (t, *J* = 12.0 Hz, 3H), 2.08 (s, 3H), 2.07 - 2.01 (m, 1H), 1.87 (d, *J* = 10.8 Hz, 1H), 1.69 (d, *J* = 6.8 Hz, 2H), 1.59 - 1.49 (m, 2H), 1.41 - 1.19 (m, 3H). |
| 137 | ¹H NMR (400 MHz, DMSO-*d₆*) δ 10.12 (s, 1H), 8.29 (d, *J* = 8.0 Hz, 1H), 8.07 (d, *J* = 7.2 Hz, 1H), 7.83 (dd, *J* = 7.6, 8.0 Hz, 1H), 7.80 - 7.71 (m, 4H), 7.71 - 7.64 (m, 2H), 7.12 (d, *J* = 8.0Hz, 1H), 3.92 (t, *J* = 6.8Hz, 2H), 3.84 (d, *J* = 10.8 Hz, 1H), 3.65 (d, *J* = 12.4 Hz, 1H), 3.36 (t, *J* = 6.0 Hz, 2H), 3.21 (s, 3H), 2.93 - 2.83 (m, 1H), 2.38 (d, *J* = 10.8 Hz, 1H), 2.24 (d, *J* = 10.8 Hz, 1H), 2.03 - 1.96 (m, 1H), 1.91 (quintet, *J*=6.4Hz, 2H), 1.88 - 1.80 (m, 1H), 1.65 - 1.51 (m, 1H), 1.51 - 1.35 (m, 1H). |
| 138 | ¹H NMR (400 MHz, Chloroform-*d*) δ 9.67 (s, 1H), 8.13 - 8.05 (m, 3H), 7.97 (d, *J* = 7.2 Hz, 2H), 7.82 - 7.77 (m, 1H), 7.71 - 7.56 (m, 3H), 6.96 (d, *J* = 8.0 Hz, 1H), 4.74 (d, *J* = 5.6 Hz, 1H), 4.13 (d, *J* = 14.0 Hz, 1H), 4.02 (t, *J* = 6.8 Hz, 2H), 3.43 (t, *J* = 6.0 Hz, 2H), 3.34 (s, 3H), 3.26 - 3.16 (m, 1H), 2.38 (d, *J* = 13.2 Hz, 1H), 2.10 - 2.01 (m, 2H), 1.54 - 1.49(m, 1H), 1.29 - 1.08 (m, 4H). |
| 139 | ¹H NMR (400 MHz, Chloroform-*d*) δ 8.92 (s, 1H), 8.13 - 7.98 (m, 3H), 7.80 (t, *J* = 8.0 Hz, 1H), 7.65 (s, 1H), 7.18 (d, *J* = 3.2 Hz, 1H), 6.89 (d, *J* = 7.6 Hz, 1H), 6.63 - 6.58 (m, 1H), 4.77 (d, *J* = 6.0 Hz, 1H), 4.16 - 4.08 (m, 2H), 4.30 - 3.95 (m, 2H), 3.84 - 3.73 (m, 2H), 3.30 - 3.12 (m, 1H), 2.74 (s, 3H), 2.65 - 2.56 (m, 2H), 2.49 - 2.42 (m, 1H), 2.04 (s, 1H), 1.99 - 1.92 (m, 2H), 1.82 - 1.74 (m, 2H), 1.71 - 1.66 (m, 1H), 4.66 - 1.58 (m, 2H), 1.45 - 1.21 (m, 7H). |
| 140 | ¹H NMR (400 MHz, Chloroform-*d*) δ 9.27 (s, 1H), 8.13 - 8.05 (m, 2H), 7.96 (d, *J* = 8.4 Hz, 1H), 7.80 - 7.72 (m, 1H), 6.90 (d, *J* = 7.6 Hz, 1H), 4.03 - 3.95 (m, 2H), 3.79 (d, *J* = 12.4 Hz, 2H), 3.66 (t, *J* = 13.6 Hz, 2H), 3.23 (d, *J* = 10.8 Hz, 1H), 3.01 - 2.95 (m, 1H), 2.78 - 2.72 (m, 4H), 2.65 (s, 3H), 2.62 - 2.56 (m, 2H), 2.52 - 2.44 (m, 1H), 2.41 - 2.35 (m, 2H), 2.19 - 2.10 (m, 3H), 2.06 - 2.00 (m, 1H), 1.99 - 1.91 (m, 2H), 1.83 - 1.67 (m, 9H), 1.45 - 1.36 (m, 3H), 1.29 - 1.23 (m, 3H). |
| 141 | ¹H NMR (400 MHz, DMSO-*d₆*) δ 10.23 (s, 1H), 8.25 (d, *J* = 8.0 Hz, 1H), 8.08 (d, *J* = 7.2 Hz, 1H), 7.84 (t, *J* = 7.6 Hz, 1H), 7.73 (s, 1H), 7.18 (d, *J* = 8.0Hz, 1H), 3.94 (t, *J* = 6.4Hz, 2H), 3.51 (d, *J* = 10.8 Hz, 3H), 3.32 (s, 3H), 3.31 - 3.26 (m, 2H), 3.13 - 3.04 (m, 4H), 2.88 - 2.81 (m, 2H), 2.81 (s, 3H), 2.62 (t, *J* = 12.0 Hz, 3H), 1.87 (d, *J* = 11.5 Hz, 2H), 1.81 - 1.61 (m, 5H), 1.27 - 1.13 (m, 8H). |
| 142 | ¹H NMR (400 MHz, Chloroform-*d*) δ 8.56 (s, 1H), 8.18 (d, *J* = 8.4 Hz, 1H), 8.06 (d, *J* = 6.8 Hz, 1H), 7.93 (d, *J* = 7.6 Hz, 1H), 7.75 (t, *J* = 7.2 Hz, 1H), 7.57 (d, *J* = 8.4Hz, 2H), 6.92 (d, *J* = 7.6 Hz, 1H), 6.72 (d, *J* = 8.8 Hz, 2H), 4.20 (br s, 2H), 4.01 (t, *J* = 6.8 Hz, 2H), 3.42 (d, *J* = 6.0 Hz, 2H), 3.33 (s, 3H), 3.16 - 3.00 (m, 2H), 2.91 - 2.79 (m, 1H), 2.04 - 1.73 (m, 6H), 1.30 - 1.20 (m, 2H). |
| 143 | ¹H NMR (400 MHz, Chloroform-*d*) δ 8.23 (s, 1H), 8.05 (d, *J* = 8.0 Hz, 2H), 7.79 (d, *J* = 7.6 Hz, 1H), 7.73(t, *J* = 7.6 Hz, 1H), 6.83 (d, *J* = 7.6 Hz, 1H), 3.96 (t, *J* = 6.8 Hz, 2H), 3.83 - 3.69 (m, 3H), 3.56 - 3.37 (m, 2H), 3.19 - 3.09 (m, 1H), 2.86 (s, 3H), 2.73 (s, 3H), 2.59 (t, *J* = 12.0 Hz, 2H), 2.11 - 2.00 (m, 3H), 1.99 - 1.87 (m, 3H), 1.85 - 1.70 (m, 3H), 1.45 - 1.29 (m, 3H). |
| 144 | ¹H NMR (400 MHz, Chloroform-*d*) δ 8.51 (s, 1H), 8.37 (s, 1H), 8.15 (d, *J* = 8.0 Hz, 1H), 8.08 - 7.91 (m, 4H), 7.86 (d, *J* = 7.2 Hz, 1H), 7.81 - 7.60 (m, 4H), 6.83 (d, *J* = 7.2 Hz, 1H), 4.02 - 3.90 (m, 2H), 3.77 (d, *J* = 10.4 Hz, 2H), 3.65 - 3.55 (m, 1H), 3.40 - 3.25 (m, 2H), 3.07 - 2.97 (m, 1H), 2.91 (s, 1H), 2.73 (s, 3H), 2.65 - 2.55 (m, 2H), 1.97 - 1.86 (m, 4H), 1.80 - 1.71 (m, 2H), 1.64 (s, 1H), 1.45 - 1.24 (m, 3H). |
| 145 | ¹H NMR (400 MHz, Chloroform-*d*) δ 9.16 (s, 1H), 8.15 - 8.04 (m, 3H), 7.77 (d, *J* = 8.0 Hz, 2H), 7.71 (d, *J* = 8.4 Hz, 1H), 6.95 (d, *J* = 7.6 Hz, 1H), 6.76 (d, *J* = 8.8 Hz, 2H), 4.21 (br s, 2H), 4.10 - 4.05 (m, 1H), 4.02 (t, *J* = 6.8 Hz, 2H), 3.43 (t, *J* = 6.0 Hz, 1H), 3.33 (s, 3H), 3.23 - 3.11 (m, 1H), 2.38 (d, *J* = 14.0 Hz, 1H), 2.11 - 1.98 (m, 3H), 1.33 - 1.16 (m, 5H). |
| 146 | ¹H NMR (400 MHz, Chloroform-*d*) δ 8.56 (s, 1H), 8.06 (d, *J* = 10.8 Hz, 1H), 8.01 (d, *J* = 9.2 Hz, 1H), 7.82 (d, *J* = 10.4 Hz, 1H), 7.74 - 7.63 (m, 2H), 7.59 (s, 1H), 7.19 (t, *J* = 5.6 Hz, 1H), 6.92 (d, *J* = 10.0 Hz, 1H), 4.19 (t, *J* = 9.2 Hz, 2H), 3.65 (s, 3H), 3.64 - 3.53 (m, 2H), 3.50 - 3.31 (m, 1H), 3.17 (t, *J* = 9.6 Hz, 1H), 2.99 - 2.84 (m, 3H), 2.81 (t, *J* = 9.6 Hz, 2H), 1.92 - 1.75 (m, 2H). |
| 147 | ¹H NMR (400 MHz, Chloroform-*d*) δ 8.97 (s, 1H), 8.13 - 8.02 (m, 3H), 7.81 - 7.76 (m, 1H), 7.74 - 7.68 (m, 2H), 7.20 - 7.16 (m, 1H), 6.99 (d, *J* = 7.6 Hz, 1H), 4.81 - 4.76 (m, 1H), 4.27 - 4.09 (m, 4H), 3.66 (s, 2H), 3.29 - 3.20 (m, 1H), 2.87 - 2.80 (m, 2H), 2.42 (d, *J* = 14.0 Hz, 1H), 1.37 - 1.22 (m, 5H). |
| 148 | ¹H NMR (400 MHz, Chloroform-*d*) δ 10.31 (br s, 1H), 8.08 (d, *J* = 6.8 Hz, 1H), 7.98 (d, *J* = 7.6 Hz, 1H), 7.92 (d, *J* = 8.4 Hz, 1H), 7.84 (d, *J* = 7.6 Hz, 2H), 7.74 (t, *J* = 7.2 Hz, 1H), 7.65 (t, *J* = 7.6 Hz, 1H), 7.53 (d, *J* = 7.6 Hz, 2H), 6.75 (d, *J* = 7.6 Hz, 1H), 4.01 (t, *J* = 6.8 Hz, 2H), 3.42 (t, *J* = 6.0 Hz, 2H), 3.38 - 3.27 (m, 5H), 3.24 - 3.15 (m, 1H), 3.12 - 2.95 (m, 3H), 2.80 - 2.74 (m, 1H), 2.50 (d, *J* = 9.2 Hz, 1H), 2.26 (t, *J* = 9.6 Hz, 1H), 2.17 - 2.09 (m, 1H), 2.04 (quintet, *J* = 6.4 Hz, 2H), 1.97 - 1.88 (m, 1H), 1.79 - 1.67 (m, 2H). |
| 149 | ¹H NMR (400 MHz, Chloroform-*d*) 8.13 (s, 1H), 8.08 - 8.04 (m, 2H), 7.83 (d, *J* = 7.6 Hz, 1H), 7.77 - 7.70 (m, 1H), 7.74 (d, *J* = 7.2 Hz, 1H), 7.51 (s, 1H), 7.25 - 7.22 (m, 1H), 6.91 (d, *J* = 7.6 Hz, 1H), 4.00 (t, *J* = 6.8 Hz, 2H), 3.64 (d, *J* = 11.6 Hz, 1H), 3.42 (t, *J* = 6.0 Hz, 3H), 3.33 (s, 3H), 3.15 - 3.06 (m, 1H), 2.89 - 2.79 (m, 2H), 2.03 (quintet, *J* = 6.4 Hz, 2H), 1.97 - 1.89 (m, 2H), 1.86 - 1.78 (m, 1H), 1.25 (s, 1H). |
| 150 | ¹H NMR (400 MHz, Chloroform-*d*) δ 9.39 (s, 1H), 8.08 (d, *J* = 3.2 Hz, 1H), 8.05 (d, *J* = 5.2 Hz, 1H), 7.95 (d, *J* = 10.4 Hz, 1H), 7.84 (d, *J* = 9.6 Hz, 2H), 7.74 (dd, *J* = 9.2, 10.8 Hz, 1H), 7.58 - 7.50 (m, 1H), 7.46 (d, *J* = 9.2Hz, 1H), 7.45 - 7.40 (m, 1H), 6.96 (d, *J* = 10.0 Hz, 1H), 4.02 (t, *J* = 8.8 Hz, 2H), 3.43 (t, *J* = 8.0Hz, 2H), 3.36 (t, *J* = 6.4 Hz, 2H), 3.34 (s, 3H), 3.17 - 3.05 (m, 2H), 3.01 - 2.88 (m, 1H), 2.43 - 2.29 (m, 1H), 2.05 (quintet, *J* = 8.4 Hz, 2H), 2.00 - 1.85 (m, 2H), 1.70 - 1.53 (m, 3H), 1.55 - 1.34 (m, 2H). |
| 151 | ¹H NMR (500 MHz, Chloroform-*d*) δ 8.33 (s, 1H), 8.17 (d, *J* = 10.5 Hz, 1H), 8.10 (d, *J* = 8.5 Hz, 1H), 7.95 - 7.87 (m, 3H), 7.82 - 7.77 (m, 3H), 7.66 - 7.61 (m, 2H), 7.56 - 7.43 (m, 3H), 6.96 (d, *J* = 9.5 Hz, 1H), 4.04 (t, *J* = 8.5 Hz, 2H), 3.61 - 3.51 (m, 1H), 3.45 (t, *J* = 7.5 Hz, 2H), 3.36 (s, 5H), 3.12 - 3.02 (m, 1H), 2.93 - 2.86 (m, 1H), 2.07 (quintet, *J* = 8.0 Hz, 2H), 2.00 - 1.92 (m, 3H), 1.89 - 1.78 (m, 1H). |
| 152 | ¹H NMR (400 MHz, Chloroform-*d*) δ 9.16 (s, 1H), 8.14 - 8.04 (m, 3H), 8.01 (d, *J* = 8.4 Hz, 2H), 7.80 (d, *J* = 8.4 Hz, 2H), 7.78 - 7.74 (m, 1H), 7.64 (d, *J* = 7.2 Hz, 2H), 7.51 (t, *J* = 6.8 Hz, 2H), 7.48 - 7.41 (m, 1H), 6.96 (d, *J* = 7.6 Hz, 1H), 4.79 (d, *J* = 5.2 Hz, 1H), 4.17 (d, *J* = 14.8 Hz, 1H), 4.02 (t, *J* = 6.4 Hz, 2H), 3.43 (d, *J* = 6.0 Hz, 2H), 3.34 (s, 3H), 3.25 (t, *J* = 12.8 Hz, 1H), 2.41 (d, *J* = 13.2 Hz, 1H), 2.05 (quintet, *J* = 6.4 Hz, 1H), 1.60 - 1.51 (m, 2H), 1.33 - 1.15 (m, 3H). |
| 153 | ¹H NMR (400 MHz, Chloroform-*d*) δ 9.09 (s, 1H), 8.12 - 8.03 (m, 3H), 8.01 (d, *J* = 7.6 Hz, 1H), 7.84 (d, *J* = 8.4 Hz, 2H), 7.81 - 7.71 (m, 3H), 6.94 (d, *J* = 8.0 Hz, 1H), 4.71 (d, *J* = 4.8 Hz, 1H), 4.11 (d, *J* = 13.6 Hz, 1H), 4.01 (t, *J* = 6.4 Hz, 2H), 3.42 (t, *J* = 6.0 Hz, 2H), 3.33 (s, 3H), 3.21 (t, *J* = 13.2 Hz, 1H), 2.36 (d, *J* = 13.2 Hz, 1H), 2.22 (s, 3H), 2.04 (quintet, *J* = 6.4 Hz, 2H), 1.64 - 1.46 (m, 2H), 1.28 - 1.11 (m, 3H). |
| 154 | ¹H NMR (400 MHz, Chloroform-*d*) δ 8.33 (s, 1H), 8.11 (d, *J* = 8.0 Hz, 1H), 8.06 (d, *J* = 8.0 Hz, 1H), 7.87 (d, *J* = 7.6 Hz, 1H), 7.78 - 7.68 (m, 4H), 7.56 (s, 1H), 6.92 (d, *J* = 7.6 Hz, 1H), 4.04 - 3.95 (m, 2H), 3.49 - 3.38 (m, 3H), 3.33 (s, 3H), 3.27 - 3.19 (m, 2H), 3.00 - 2.91 (m, 1H), 2.83 (s, 1H), 2.22 (s, 1H), 2.09 - 1.99 (m, 3H), 1.95 - 1.87 (m, 3H), 1.31 - 1.20 (m, 2H). |
| 155 | ¹H NMR (400 MHz, Chloroform-*d*) δ 8.50 (s, 1H), 8.15 (d, *J* = 8.0 Hz, 1H), 7.93 (d, *J* = 7.2 Hz, 2H), 7.78 (d, *J* = 7.2 Hz, 2H), 7.70 (t, *J* = 7.2 Hz, 1H), 7.64 - 7.56 (m, 2H), 7.43 (t, *J* = 7.2 Hz, 1H), 7.34 (t, *J* = 8.0 Hz, 2H), 6.91 (t, *J* = 8.0 Hz, 2H), 4.71 (t, *J* = 8.8 Hz, 2H), 4.31 (t, *J* = 6.8 Hz, 2H), 3.68 (t, *J* = 6.8 Hz, 2H), 3.41 - 3.25 (m, 4H), 3.20 - 3.01 (m, 2H), 2.93 - 2.80 (m, 1H), 1.99 - 1.75 (m, 4H). |
| 156 | ¹H NMR (400 MHz, Chloroform-*d*) 9.14 (s, 1H), 8.13 - 8.06 (m, 2H), 7.98 - 7.94 (m, 1H), 7.83 - 7.70 (m, 5H), 7.46 - 7.41 (m, 1H), 7.38 - 7.31 (m, 2H), 6.97 - 6.88 (m, 2H), 4.78 - 4.67 (m, 3H), 4.32(t, *J* = 7 Hz, 2H), 4.09(d, *J*= 15.2 Hz, 1H), 3.70(t, *J*= 6.8 Hz, 2H), 3.31(t, *J*= 8.8 Hz, 2H), 3.24 - 3.13 (m, 1H), 2.40 (d, *J*= 13.6 Hz, 1H), 1.70 - 1.60 (m, 1H), 1.32 - 1.14 (m, 4H). |
| 157 | ¹H NMR (500 MHz, Chloroform-*d*) δ 8.09 (d, *J* = 9.0 Hz, 1H), 8.05 - 7.98 (m, 2H), 7.94 (s, 1H), 7.80 - 7.72 (m, 2H), 7.58 (d, *J* = 9.0 Hz, 1H), 6.93 (d, *J* = 9.5 Hz, 1H), 4.13 - 4.07 (m, 1H), 4.05 - 4.00 (m, 2H), 3.91 - 3.82 (m, 1H), 3.48 - 3.42 (m, 2H), 3.56 (s, 3H), 3.34 - 3.27 (m, 1H), 3.11 - 3.01 (m, 1H), 2.86 - 2.79 (m, 1H), 2.11 - 2.04 (m, 3H), 1.99 - 1.92 (m, 2H), 1.32 - 1.27 (m, 1H). |
| 158 | ¹H NMR (400 MHz, Chloroform-*d*) δ 8.97 (s, 1H), 8.09 (d, *J* = 4.4 Hz, 1H), 8.06 (t, *J* = 8.0 Hz, 2H), 7.77 (dd, *J* = 7.2, 8.4 Hz, 1H), 7.68 (d, *J* = 4.0 Hz, 1H), 7.64 - 7.59 (m, 2H), 7.47 - 7.40 (m, 3H), 7.32 (d, *J* = 4.0 Hz, 1H), 6.95 (d, *J* = 7.6 Hz, 1H), 4.82 (d, *J* = 5.2 Hz, 1H), 4.22 - 4.13 (m, 1H), 4.02 (t, *J* = 6.8 Hz, 2H), 3.43 (t, *J* = 6.0 Hz, 2H), 3.34 (s, 3H), 3.32 - 3.23 (m, 1H), 2.45 (d, *J* = 14.4 Hz, 1H), 2.05 (quintet, *J* = 6.4 Hz, 2H), 1.73 - 1.57 (m, 3H), 1.46 - 1.35 (m, 2H). |
| 159 | ¹H NMR (400 MHz, Chloroform-o) δ 8.97 (s, 1H), 8.10 - 8.02 (m, 3H), 7.77 (dd, *J* = 7.2, 8.4 Hz, 1H), 7.68 (d, *J* = 4.0 Hz, 1H), 7.64 - 7.59 (m, 2H), 7.48 - 7.39 (m, 3H), 7.32 (d, *J* = 4.0 Hz, 1H), 6.95 (d, *J* = 7.6 Hz, 1H), 4.82 (d, *J* = 5.2 Hz, 1H), 4.18 (d, *J* = 14.0 Hz, 1H), 4.02 (t, *J* = 6.8 Hz, 2H), 3.43 (t, *J* = 6.0 Hz, 2H), 3.34 (s, 3H), 3.31 - 3.22 (m, 1H), 2.45 (d, *J* = 14.4 Hz, 1H), 2.08 - 2.01 (m, 2H), 1.68 - 1.57 (m, 3H), 1.48 - 1.37 (m, 2H). |
| 160 | ¹H NMR (500 MHz, Chloroform-*d*) δ 8.93 (s, 1H), 8.14 - 8.03 (m, 3H), 7.84 - 7.77 (m, 1H), 7.66 (dd, *J* = 1.0, 2.5 Hz, 1H), 7.20 (dd, *J* = 1.0, 4.0 Hz, 1H), 6.99 (d, *J* = 9.5 Hz, 1H), 6.63 (d, *J* = 2.0, 4.5 Hz, 1H), 4.05 (t, *J* = 8.5 Hz, 2H), 3.45 (t, *J* = 7.5 Hz, 2H), 3.36 (s, 4H), 3.30 - 3.21 (m, 1H), 2.54 - 2.47 (m, 1H), 2.08 (quintet, *J* = 8.0 Hz, 2H), 1.76 - 1.62 (m, 3H), 1.42 - 1.26 (m, 3H). |
| 161 | ¹H NMR (400 MHz, Chloroform-*d*) δ 8.20 (s, 1H), 8.07 (dd, *J* = 7.2, 8.8 Hz, 2H), 7.86 (d, *J* = 7.6 Hz, 1H), 7.84 (dd, *J* = 5.2, 8.4 Hz, 2H), 7.74 (dd, *J* = 7.2, 8.0 Hz, 1H), 7.30 - 7.20 (m, 2H), 6.92 (d, *J* = 7.6 Hz, 1H), 4.00 (t, *J* = 6.8 Hz, 2H), 3.56 (d, *J* = 11.6 Hz, 1H), 3.42 (t, *J* = 6.0 Hz, 2H), 3.33 (s, 3H), 3.17 (dd, *J* = 9.6, 10.0 Hz, 1H), 2.96 - 2.79 (m, 2H), 2.09 - 2.00 (m, 3H), 1.97 - 1.77 (m, 4H). |
| 162 | ¹H NMR (400 MHz, Chloroform-*d*) δ 8.97 (s, 1H), 8.06 (dd, *J* = 7.2, 8.4 Hz, 2H), 8.01 (d, *J* = 7.6 Hz, 1H), 7.70 - 7.71 (m, 2H), 7.67 - 7.62 (m, 1H), 7.58 (td, *J* = 5.2, 2.8 Hz, 1H), 7.34 (dt, *J* = 2.0, 8.0 Hz, 1H), 6.93 (d, *J* = 7.6 Hz, 1H), 4.74 (d, *J* = 5.2 Hz, 1H), 4.14 - 4.06 (m, 1H), 4.01 (t, *J* = 6.4 Hz, 2H), 3.42 (t, *J* = 6.0 Hz, 2H), 3.33 (s, 3H), 3.30 - 3.20 (m, 1H), 2.39 (d, *J* = 13.6 Hz, 1H), 2.08 - 2.00 (m, 2H), 1.74 (s, 1H), 1.56 (s, 1H), 1.28 - 1.13 (m, 3H). |
| 163 | ¹H NMR (400 MHz, Chloroform-*d*) δ 8.77 (s, 1H), 7.96 (d, *J* = 8.0 Hz, 1H), 7.89 (d, *J* = 6.4 Hz, 1H), 7.65 (d, *J* = 7.6 Hz, 1H), 7.58 - 7.42 (m, 4H), 7.34 - 7.27 (m, 1H), 6.77 (d, *J* = 7.6 Hz, 1H), 3.97 - 3.80 (m, 3H), 3.59 (d, *J* = 9.2 Hz, 1H), 3.41 - 3.25 (m, 5H), 2.97 - 2.81 (m, 3H), 2.60 - 2.52 (m, 1H), 2.09 - 1.78 (m, 5H). |
| 164 | ¹H NMR (500 MHz, Chloroform-*d*) δ 8.32 (s, 1H), 8.15 (d, *J* = 10.5 Hz, 1H), 8.09 (d, *J* = 8.5 Hz, 1H), 7.94 - 7.86 (m, 3H), 7.81 - 7.72 (m, 3H), 7.63 - 7.57 (m, 2H), 7.24 - 7.18 (m, 2H), 6.96 (d, *J* = 9.5 Hz, 1H), 4.03 (t, *J* = 8.5 Hz, 2H), 3.64 - 3.56 (m, 1H), 3.45 (t, *J* = 3.0 Hz, 2H), 3.38 - 3.34 (m, 4H), 3.32 - 3.24 (m, 1H), 3.05 - 2.96 (m, 1H), 2.95 - 2.86 (m, 1H), 2.07 (quintet, *J*= 6.4 Hz, 2H), 2.00 - 1.91 (m, 3H), 1.90 - 1.80 (m, 1H). |
| 165 | ¹H NMR (400 MHz, Chloroform-*d*) δ 8.46 (s, 1H), 7.96 (d, *J* = 7.2 Hz, 1H), 7.95 (d, *J* = 5.2 Hz, 1H), 7.71 (d, *J* = 7.6 Hz, 1H), 7.67 - 7.55 (m, 3H), 6.83 (d, *J* = 8.0 Hz, 2H), 4.65 (t, *J* = 8.4 Hz, 2H), 3.95 (t, *J* = 6.0 Hz, 2H), 3.72 (t, *J* = 8.0 Hz, 1H), 3.77 - 3.64 (m, 1H), 3.48 - 3.31 (m, 6H), 3.30 (s, 3H), 3.23 (t, *J* = 8.4 Hz, 2H), 2.31 - 2.16 (m, 2H), 2.07 - 1.93 (m, 2H). |
| 166 | ¹H NMR (400 MHz, Chloroform-*d*) δ 8.53 (s, 1H), 7.95 - 7.76 (m, 4H), 7.59 (d, *J* = 7.2 Hz, 1H), 7.51 (t, *J* = 7.6 Hz, 1H), 7.17 (t, *J* = 8.4 Hz, 2H), 6.77 (d, *J* = 7.2 Hz, 1H), 3.91 (t, *J* = 6.0 Hz, 2H), 3.73 (t, *J* = 8.4 Hz, 1H), 3.47 - 3.41 (m, 1H), 3.39 - 3.31 (m, 4H), 3.28 (s, 3H), 2.29 - 2.13 (m, 2H), 2.11 - 1.89 (m, 3H). |
| 167 | ¹H NMR (400 MHz, Chloroform-*d*) δ 8.96 (s, 1H), 8.12 - 7.96 (m, 3H), 7.69 (t, *J* = 7.6 Hz, 1H), 6.91 (d, *J* = 8.0 Hz, 1H), 4.99 (d, *J* = 4.4 Hz, 1H), 3.98 (t, *J* = 6.8 Hz, 2H), 3.39 (t, *J* = 6.0 Hz, 2H), 3.35 - 3.33 (m, 1H), 3.31 (s, 3H), 3.26 - 3.23 (m, 1H), 2.06 - 1.97 (m, 3H), 1.77 - 1.59 (m, 5H) 1.53 (s, 9H). |
| 168 | ¹H NMR (400 MHz, Chloroform-*d*) δ 10.41 (s, 1H), 8.09 (d, *J* = 8.4 Hz, 1H), 7.83 (d, *J* = 6.8 Hz, 1H), 7.58 - 7.43 (m, 2H), 6.67 (d, *J* = 7.6 Hz, 1H), 4.28 (d, *J* = 11.6 Hz, 1H), 3.82 (t, *J* = 6.8 Hz, 2H), 3.34 - 3.21 (m, 3H), 3.23 (s, 3H), 2.94 - 2.86 (m, 1H), 2.20 (d, *J* = 12.0 Hz, 1H), 1.87 (quintet, *J* = 6.0 Hz, 2H), 1.83 - 1.58 (m, 4H), 1.54 - 1.43 (m, 1H). |
| 169 | ¹H NMR (400 MHz, Chloroform-*d*) δ 9.07 (s, 1H), 8.13 - 8.03 (m, 3H), 8.00 (d, *J* = 8.4 Hz, 2H), 7.80 - 7.76 (m, 1H), 7.75 (d, *J* = 8.4 Hz, 2H), 7.60 (dd, *J* = 5.6, 8.4 Hz, 2H), 7.19 (t, *J* = 8.4 Hz, 2H), 6.95 (d, *J* = 8.0 Hz, 1H), 4.77 (d, *J* = 5.2 Hz, 1H), 4.16 (d, *J* = 14.4 Hz, 1H), 4.02 (t, *J* = 6.8 Hz, 2H), 3.43 (t, *J* = 5.6 Hz, 2H), 3.33 (s, 3H), 3.25 (t, *J* = 12.8 Hz, 1H), 2.41 (d, *J* = 13.2 Hz, 1H), 2.09 - 2.01 (m, 2H), 1.68 - 1.51 (m, 3H), 1.29 - 1.17 (m, 2H). |

### <Experimental Example 1> Measurement of YAP-TEAD binding inhibition effect

A Luciferase assay which allows an inhibition degree of YAP-TEAD, previously reported in Cancers 2018, 10(5), 140, was used to measure an in vitro binding inhibition rate of YAP-TEAD.

HEK293T cells were purchased from American Type Culture Collection and incubated in DMEM media containing 10% FBS, L-glutamine, and penicillin/streptomycin. The HEK293T cells were seeded in a 24 well plate at a density of 1×10⁵, and coated with polyethyleneamine (10 µg/mL). These cells were transfected with 8XGTIIC-luciferase (Addgene reference 34615) which is TEAD luciferase reporter plasmid and control β-galactosidase plasmid CMV-β-Gal using a lipofectamine 2000 reagent (Life Technologies, Inc.) according to the manufacturer's manual. Thereafter, the compound of the present invention was treated at a concentration of 10 µM for 24 hours, the cells were lysed using a Reporter lysis buffer (Promega, France), luciferase activity was read with a Mithras LB940 plate reader, and then normalization was performed with β-galactosidase. The results are shown in the following Table 3.

**[Table 3]**

| Compound | Inhibition(%) | Compound | Inhibition(%) |
|---|---|---|---|
| 3 | 63 | 75 | 93 |
| 13 | 50 | 76 | 87 |
| 15 | 81 | 78 | 53 |
| 16 | 77 | 79 | 71 |
| 17 | 78 | 80 | 77 |
| 20 | 65 | 83 | 92 |
| 21 | 59 | 84 | 83 |
| 22 | 78 | 106 | 99.2 |
| 24 | 61 | 117 | 85 |
| 25 | 61 | 118 | 84 |
| 26 | 100 | 119 | 68 |
| 28 | 81 | 122 | 68 |
| 29 | 87 | 123 | 54 |
| 30 | 56 | 131 | 78 |
| 34 | 87 | 133 | 59 |
| 35 | 56 | 134 | 62 |
| 36 | 54 | 136 | 80 |
| 39 | 66 | 137 | 87 |
| 42 | 68 | 138 | 79 |
| 43 | 50 | 139 | 98 |
| 48 | 58 | 145 | 53 |
| 49 | 51 | 149 | 59 |
| 50 | 63 | 151 | 57 |
| 55 | 50 | 152 | 77 |
| 56 | 63 | 153 | 55 |
| 58 | 65 | 155 | 66 |
| 59 | 87 | 156 | 64 |
| 60 | 67 | 158 | 61 |
| 64 | 76 | 159 | 84 |
| 66 | 59 | 160 | 53 |
| 68 | 81 | 161 | 88 |
| 69 | 94 | 162 | 74 |
| 71 | 91 | | |

From the results of Table 3, it was confirmed that the compounds of the present invention showed an inhibition rate of 50% or more at a concentration of 10 µM, and due to the structural characteristic of the compounds of the present invention, TEAD-dependent transcription by inhibition of YAP-TEAD binding was able to be effectively inhibited. Therefore, the compounds of the present invention may inhibit the expression of cyr61, CTGF, PD-L1, and the like which play an important role in the cancer occurrence process among main target genes of TEAD-dependent transcription, thereby showing anticancer activity.

### <Experimental Example 2> Measurement of anticancer activity by MTT assay

HT-29 which is a commercially available colorectal cancer cell line was treated with trypsin-EDTA, incubated, and seeded in a 96 well plate. After an isothermal treatment for 24 hours, each cell was treated with a candidate compound (compound of the present invention) so that a final concentration was 0-2 µM. The treated cells were incubated for further 72 hours and cell viability was measured by an ATP detection method (CellTiter-Glo LuminescentCell Viability Assay, Promega).

In order to confirm the anticancer effect of the compounds according to the present invention, a proliferation assay for HT29 cells which are a colorectal cancer cell line was performed by the above method, and IC₅₀ values as a result are listed in the following Table 4:

**[Table 4]**

| Compound | Proliferation Assay (IC₅₀, µM) | Compound | Proliferation Assay (IC₅₀, µM) |
|---|---|---|---|
| 5 | 7.1 | 58 | 10.2 |
| 6 | 7.29 | 59 | 7.6 |
| 9 | 25.28 | 60 | 12.5 |
| 12 | 30.71 | 61 | 5.6 |
| 15 | 2.99 | 64 | 24.7 |
| 16 | 4.24 | 65 | 17.5 |
| 17 | 6.91 | 66 | 8.7 |
| 18 | 26.39 | 68 | 10.9 |
| 20 | 32.3 | 69 | 16.8 |
| 21 | 34.2 | 75 | 16.8 |
| 29 | 12.2 | 78 | 30.1 |
| 30 | 22 | 79 | 14 |
| 34 | 13.3 | 80 | 5.6 |
| 38 | 2.2 | 81 | 45.4 |
| 39 | 20.3 | 82 | 30.6 |
| 42 | 44.5 | 83 | 19.4 |
| 43 | 35 | 84 | 8.2 |
| 45 | 40.4 | 117 | 12 |
| 47 | 29.2 | 118 | 2.9 |
| 56 | 9.8 | 122 | 6.5 |

From the results of Table 4, the compounds of the present invention showed a IC₅₀ value of 45.4 µM or less, specifically 2.2 to 45.4 µM, and the compounds of the present invention were confirmed to inhibit cell proliferation of HT29 cells which are a colorectal cancer cell line by binding inhibition of YAP-TEAD.

### <Experimental Example 3> Anticancer activity evaluation experiment using an AOM/DSS orthotopic syngeneic mouse model

In order to measure an anticancer effect in the state of immunity existing in a large intestine, a conventionally known AOM/DSS model was used to measure anticancer activity *(*J. Vis. Exp. (67), e4100 10.3791/4100*,* Cell Death & Dis. 2018, 9, 153*.).*

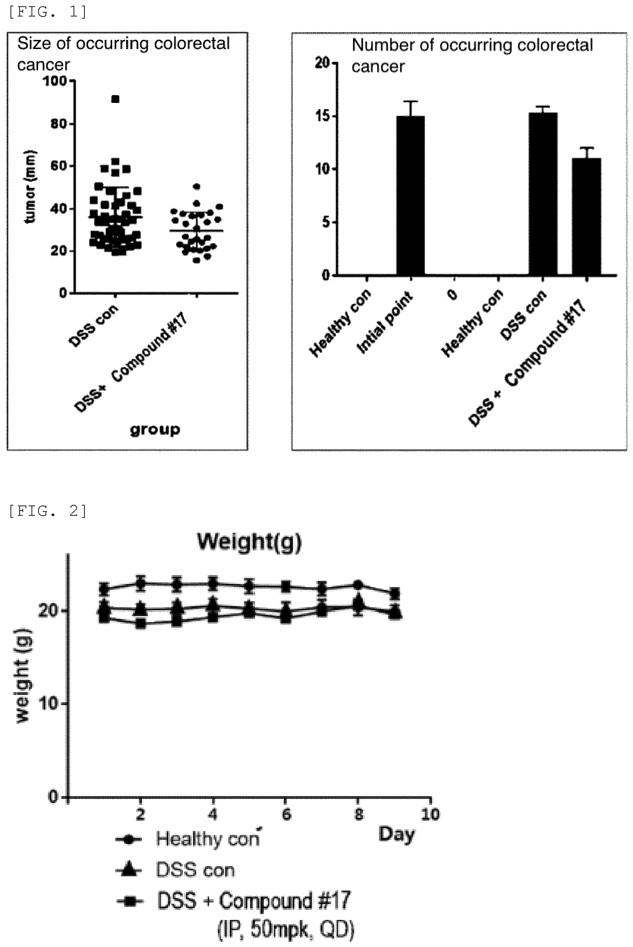

A B6 mouse was adapted to an animal room for one week and then azoxymethane (AOM) was injected intraperitoneally at 10 mg/kg. After one week, the B6 mouse was supplied with a 2.5% dextran sulfate sodium (DSS) solution for one week and alternatively supplied with fresh water for two weeks, which was repeated three time, thereby causing colorectal cancer. After colorectal cancer was caused, the YAP-TEAD inhibition compound 17 of the present invention was injected intraperitoneally at 50 mg/kg, five times a week, for three weeks. After the dosing period was over, an intestine tissue was surgically isolated and the number and size of cancer tissue in the intestine tissue were measured and quantitatively analyzed, and the results are illustrated in FIGS. 1 to 3.

The results of observing the size and number of tumors following the administration of the compound of the present invention are shown in FIG. 1, and it was confirmed therefrom that when the compound of the present invention was administered, a degree of an increase in tumor size was significantly decreased for the size and the number as compared with the case in which the compound of the present invention was not administered. In addition, a normal weight increase was observed in all individuals during the experiment, and as a result of an autopsy after the experiment, nothing unusual was observed in each tissue and organ.

In addition, in order to conform potential toxicity of the compound of the present invention, the weight in the entire section of drug treatment was measured and the results are shown in FIG. 2, and the weight of the group treated with only DSS was decreased as compared with the group treated with no DSS, but any further weight loss by the administration of the compound of the present invention was not observed.

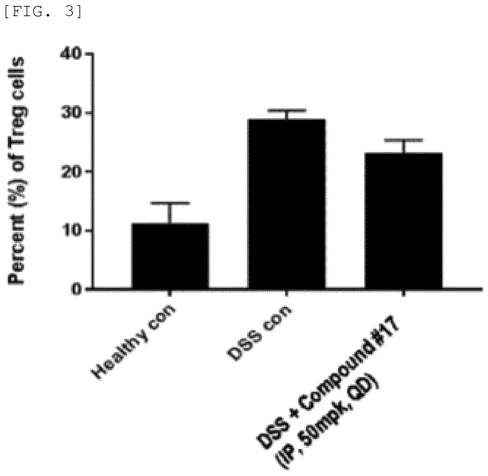

In addition, in order to confirm a ratio of Treg cells which play an important role in immunosuppression in cancer tissues, FACS analysis was performed. The method was as follows: a cancer tissue in an intestinal tissue was cut and made into a single cell using collagenase, antibody staining for labels CD4 and Fop3 was performed, and then FACS analysis was performed, and the results are shown in FIG. 3.

Treg cells which are known to play an important role in immunosuppression in Tumor microenvironment were greatly increased in an AOM/DSS-induced colorectal cancer tissue. However, in the group to which the compound 17 of the present invention was administered intraperitoneally for three weeks, Treg cell decrease was confirmed. Accordingly, it was seen that the compound of the present invention decreases immunosuppression by Treg overexpression in the process of colorectal cancer generation to increase immune anticancer activity.

## Claims

1. A compound represented by the following Chemical Formula 1, a prodrug thereof, a hydrate thereof, a solvate thereof, or a pharmaceutically acceptable salt thereof: 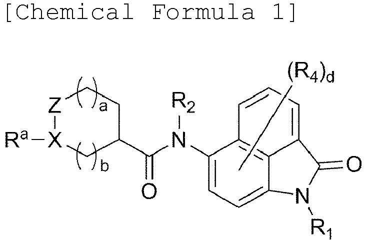 wherein
R^{a} is hydrogen or -L-R₃;
R₁ is hydrogen or -(CH₂)ₙ-R;
R is C1-C10alkoxy, C6-C20aryloxy, hydroxy, C1-C10alkylcarbonyloxy, C6-C20arylcarbonyloxy, cyano, halogen, nitro, C1-C10alkylsulfanyl, C6-C20arylsulfanyl, sulfanyl, - NR^{a1}R^{a2}, C1-C10alkoxyC1-C10alkyl, C6-C20aryloxyC1-C10alkyl, C1-C10alkoxyC1-C10alkoxy, C1-C10alkoxyC1-C10alkoxyC1-C10alkyl, C1-C10alkylsulfonyl, C6-C20arylsulfonyl, C1-C10alkylC6-C20arylsulfonyl, C6-C20arylC1-C10alkylsulfonyl, sulfo, C6-C20aryl, C3-C20cycloalkyl, carbamoyl, C1-C10alkylaminocarbonyl, C6-C20arylaminocarbonyl, sulfamoyl, C1-C10alkylaminosulfonyl, C6-C20arylaminosulfonyl, C1-C10alkylcarbonyl, C6-C20arylcarbonyl, C1-C10alkoxycarbonyl, C6-C20aryloxycarbonyl, carboxyl, formyl, C2-C20heteroaryl, or C3-C20heterocycloalkyl, and the aryl, heteroaryl, and heterocycloalkyl of R may be further substituted by one or more substituents selected from the group consisting of C1-C10alkyl, halogen, nitro, cyano, hydroxy, C1-C10alkoxy, C6-C20aryloxy, C1-C10alkylsulfanyl, haloC1-C10alkylsulfanyl, C6-C20arylsulfanyl, diC1-C10alkylamino, C1-C10alkylsulfonyl, C6-C20arylsulfonyl, C1-C10alkylC6-C20arylsulfonyl, C6-C20arylC1-C10alkylsulfonyl, C3-C20cycloalkyl, carboxyl, sulfo, formyl, carbamoyl, sulfamoyl, amino, C1-C10alkylcarbonyl, C6-C20arylcarbonyl, C1-C10alkoxycarbonyl, C6-C20aryloxycarbonyl, C1-C10alkylcarbonyloxy, C6-C20arylcarbonyloxy, C2-C20heteroaryl, and C3-C20heterocycloalkyl;
R^{a1} and R^{a2} are independently of each other hydrogen, C1-C10alkyl, C1-C10alkylsulfonyl, C6-C20arylsulfonyl, C1-C10alkylC6-C20arylsulfonyl, C6-C20arylC1-C10alkylsulfonyl, sulfo, C6-C20aryl, C3-C20cycloalkyl, or sulfamoyl;
n is an integer of 1 to 10;
R₂ is hydrogen, C1-C10alkyl, C1-C10alkoxyC1-C10alkyl, C3-C20cycloalkyl, C3-C20cycloalkyloxyC1-C10alkyl, C6-C20aryl, or C6-C20aryloxyC1-C10alkyl;
X is CH or N;
Z is -CH₂- or -CO-;
L is -SO₂-, -SO₂-L-, -NHCO-, -CONH-, C1-C10 alkylene, or -CO-;
L' is -NH- or 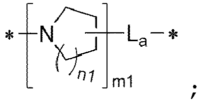
Lₐ is C1-C10alkylene;
n1 is an integer of 1 to 3;
m 1 is an integer of 0 or 1;
with a proviso that (i) when X is CH, L is necessarily -SO₂-NH-, and
(ii) when L is C1-C10 alkylene, a case in which R₃ is C1-C10alkyl, C1-C10alkoxy, or C6-C20aryloxy is excluded;
R₃ is C1-C10alkyl, C1-C10alkoxy, C6-C20aryloxy, C3-C20heterocycloalkyl, C6-C20aryl, or C2-C20heteroaryl, and the heterocycloalkyl, aryl, and heteroaryl of R₃ may be further substituted by one or more substituents selected from the group consisting of C1-C10alkyl, halogen, haloC1-C10alkyl, nitro, cyano, C1-C10alkylcarbonylamino, C6-C20arylcarbonylamino, amino, C1-C10alkoxy, haloC1-C10alkoxy, C1-C10alkoxyC1-C10alkoxy, C6-C20aryloxy, hydroxy, C1-C10alkylcarbonyl, haloC1-C10alkylcarbonyl, C6-C20arylcarbonyl, C1-C10alkoxycarbonyl, C6-C20aryloxycarbonyl, carboxyl, formyl, sulfanyl, C1-C10alkylsulfanyl, C6-C20arylsulfanyl, diC1-C10alkylaminoC1-C10alkoxy, dihydroxyaminosulfanyl, C1-C10alkylsulfonyl, C6-C20arylsulfonyl, C1-C10alkylC6-C20arylsulfonyl, C6-C20arylC1-C10alkylsulfonyl, sulfo, carbamoyl, C1-C10alkylaminocarbonyl, C6-C20arylaminocarbonyl, sulfamoyl, C1-C10alkylaminosulfonyl, C6-C20arylaminosulfonyl, C6-C20aryl, C2-C20heteroaryl, C1-C10alkylcarbonyloxy, C6-C20arylcarbonyloxy, and C3-C20heterocycloalkyl;
R₄ is halogen, haloC1-C10alkyl, cyano, C1-C10alkyl, C6-C20aryl, C2-C20heteroaryl, C1-C10alkoxy, C6-C20aryloxy, C2-C20heteroaryloxy, haloC1-C10alkoxy, hydroxy, amino, or aminoC1-C10alkyl;
d is an integer of 0 to 5, and when d is an integer of 2 or more, R₄ may be the same as or different from each other;
a and b are independently of each other an integer of 0, 1, or 2, and a+b is an integer of 1, 2 or 3; and
the heteroaryl and the heterocycloalkyl contains one or more heteroatoms selected from nitrogen, oxygen, and sulfur.

2. The compound, the prodrug thereof, the hydrate thereof, the solvate thereof, or the pharmaceutically acceptable salt thereof of claim 1, wherein the compound is represented by the following Chemical Formula 2: 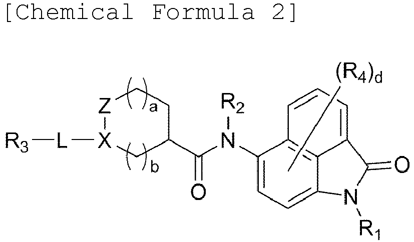 wherein R₁, R₂, X, Z, L, R₃, R₄, a, b, and d are as defined in Chemical Formula 1 of claim 1.

3. The compound, the prodrug thereof, the hydrate thereof, the solvate thereof, or the pharmaceutically acceptable salt thereof of claim 2, wherein the compound is represented by the following Chemical Formula 3, 4, 5, 6, or 7: 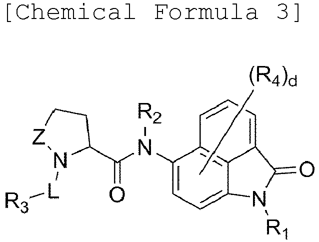 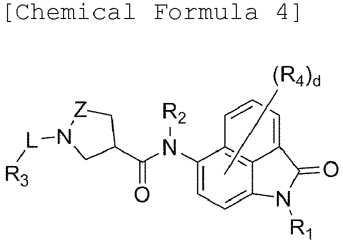 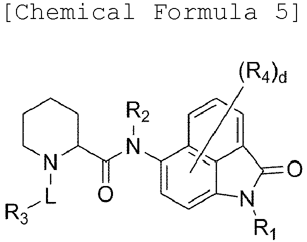 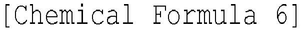 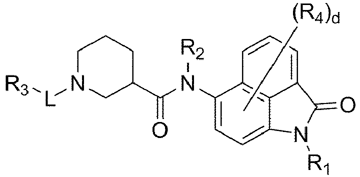 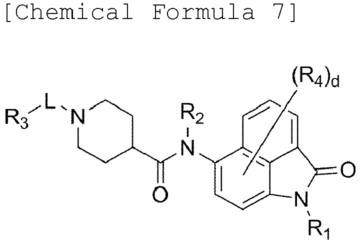 wherein R₁, R₂, Z, L, R₃, R₄, and d are as defined in Chemical Formula 2 of claim 2.

4. The compound, the prodrug thereof, the hydrate thereof, the solvate thereof, or the pharmaceutically acceptable salt thereof of claim 1, wherein the compound is represented by the following Chemical Formula 8, 9, or 10: 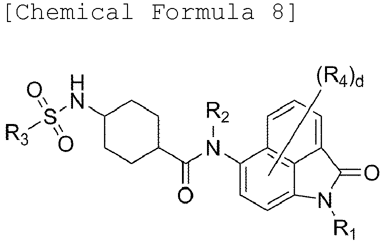 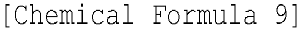 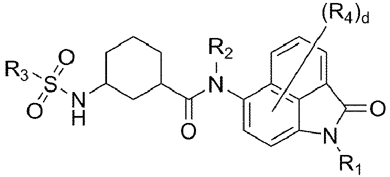 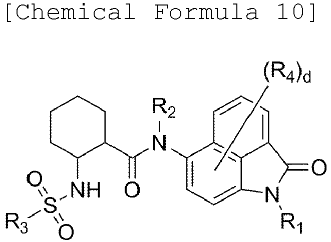 wherein R₁, R₂, R₃, R₄, and d are as defined in Chemical Formula 2 of claim 2.

5. The compound, the prodrug thereof, the hydrate thereof, the solvate thereof, or the pharmaceutically acceptable salt thereof of claim 3, wherein the compound is represented by any one selected from the following Chemical Formulae 11 to 25: 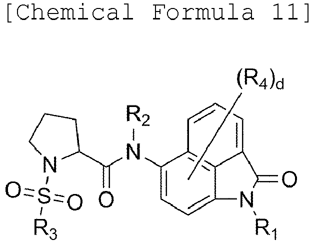 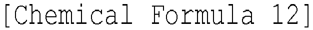 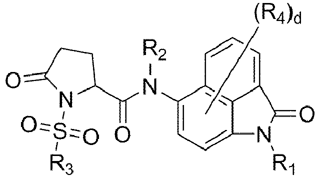 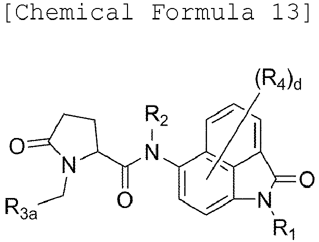 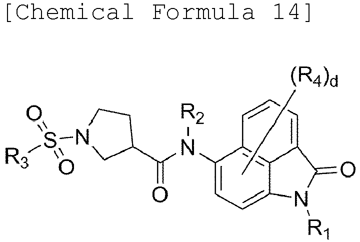 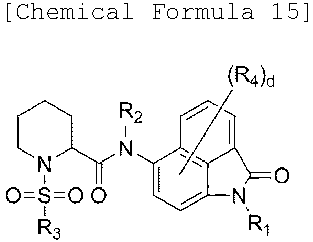 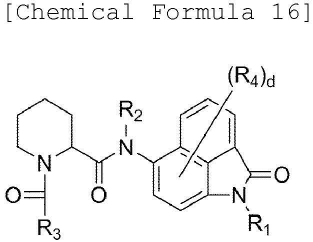 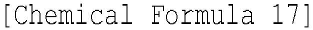 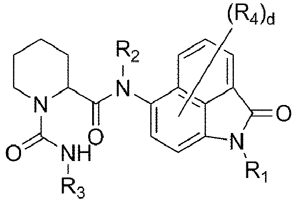 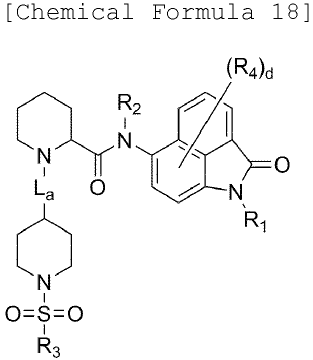 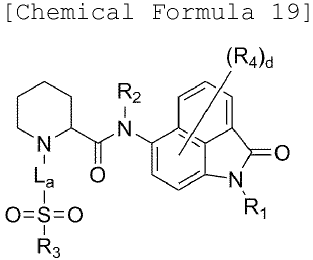 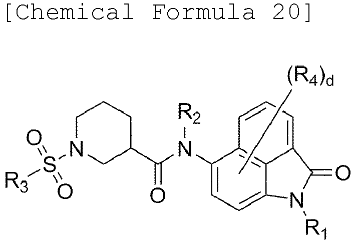 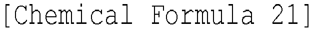 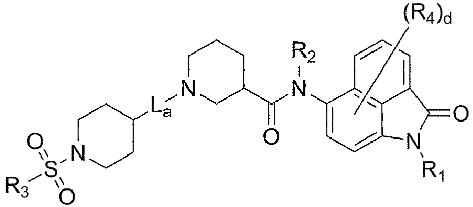 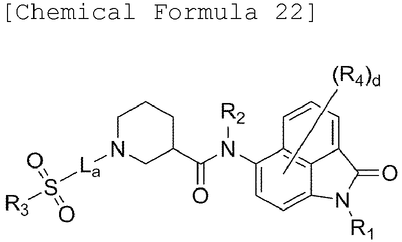 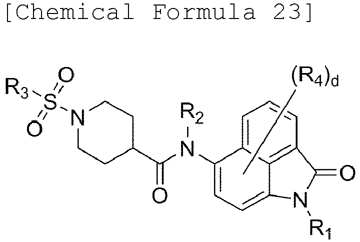 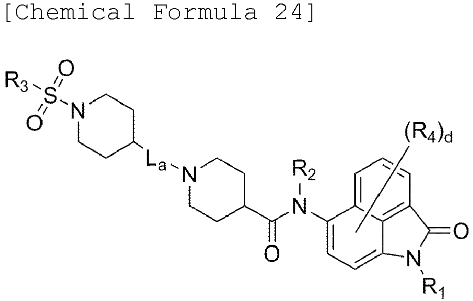 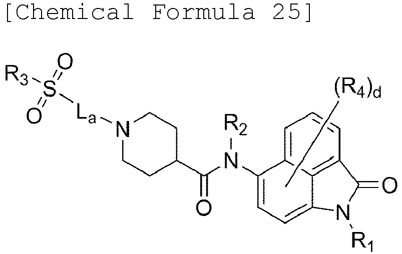
wherein R₁, R₂, R₃, R₄, and d are as defined in Chemical Formula 2 of claim 2;
R₃ₐ is C3-C20heterocycloalkyl, C6-C20aryl, or C2-C20heteroaryl, and the heterocycloalkyl, aryl, and heteroaryl of R₃ₐ may be further substituted by one or more substituents selected from the group consisting of C1-C10alkyl, halogen, haloC1-C10alkyl, nitro, cyano, C1-C10alkylcarbonylamino, C6-C20arylcarbonylamino, amino, C1-C10alkoxy, haloC1-C10alkoxy, C1-C10alkoxyC1-C10alkoxy, C6-C20aryloxy, hydroxy, C1-C10alkylcarbonyl, haloC1-C10alkylcarbonyl, C6-C20arylcarbonyl, carboxyl, formyl, sulfanyl, C1-C10alkylsulfanyl, C6-C20arylsulfanyl, diC1-C10alkylaminoC1-C10alkoxy, dihydroxyaminosulfanyl, C1-C10alkylsulfonyl, C6-C20arylsulfonyl, sulfo, carbamoyl, C1-C10alkylaminocarbonyl, C6-C20arylaminocarbonyl, sulfamoyl, C1-C10alkylaminosulfonyl, C6-C20arylaminosulfonyl, C6-C20aryl, C2-C20heteroaryl, and C3-C20heterocycloalkyl; and
Lₐ is C1-C5alkylene.

6. The compound, the prodrug thereof, the hydrate thereof, the solvate thereof, or the pharmaceutically acceptable salt thereof of claim 4 or 5, wherein
R₁ is hydrogen or - (CH₂)ₙ-R;
R is C1-C6alkoxy, C6-C12aryloxy, hydroxy, cyano, halogen, nitro, C1-C6alkylsulfanyl, C6-C12arylsulfanyl, sulfanyl, -NR^{a1}R^{a2}, C1-C6alkoxyC1-C6alkyl, C6-C12aryloxyC1-C6alkyl, C1-C6alkoxyC1-C6alkoxy, C1-C6alkoxyC1-C6alkoxyC1-C6alkyl, C1-C6alkylsulfonyl, C6-C12arylsulfonyl, C1-C6alkylC6-C12arylsulfonyl, C6-C12arylC1-C6alkylsulfonyl, sulfo, C6-C12aryl, C1-C6alkoxycarbonyl, C6-C12aryloxycarbonyl, carboxyl, formyl, C2-C12heteroaryl, or C3-C12heterocycloalkyl, and the aryl, heteroaryl, and heterocycloalkyl of R may be further substituted by one or more substituents selected from the group consisting of C1-C6alkyl, halogen, nitro, cyano, hydroxy, C1-C6alkoxy, C6-C12aryloxy, C1-C6alkylsulfonyl, C6-C12arylsulfonyl, C1-C6alkylC6-C12arylsulfonyl, C6-C12arylC1-C6alkylsulfonyl, carboxyl, sulfo, formyl, carbamoyl, sulfamoyl, and amino;
R^{a1} and R^{a2} are independently of each other hydrogen, C1-C6alkyl, C1-C6alkylsulfonyl, C6-C12arylsulfonyl, C1-C6alkylC6-C12arylsulfonyl, C6-C12arylC1-C6alkylsulfonyl, sulfo, or sulfamoyl;
n is an integer of 1 to 5;
R₂ is hydrogen, C1-C6alkyl, C1-C6alkoxyC1-C6alkyl, or C6-C12aryloxyC1-C6alkyl ;
R₃ is C1-C6alkyl, C1-C6alkoxy, C6-C12aryloxy, C3-C12heterocycloalkyl, C6-C12aryl, or C2-C12heteroaryl;
R₃ₐ is C3-C12heterocycloalkyl, C6-C12aryl, or C2-C12heteroaryl;
the heterocycloalkyl, aryl, and heteroaryl of R₃ and R₃ₐ may be further substituted by one or more substituents selected from the group consisting of C1-C6alkyl, halogen, haloC1-C6alkyl, nitro, cyano, C1-C6alkylcarbonylamino, C6-C12arylcarbonylamino, amino, C1-C6alkoxy, haloC1-C6alkoxy, C1-C6alkoxyC1-C6alkoxy, C6-C12aryloxy, hydroxy, C1-C6alkylcarbonyl, haloC1-C6alkylcarbonyl, C6-C12arylcarbonyl, carboxyl, formyl, sulfanyl, C1-C6alkylsulfanyl, C6-C12arylsulfanyl, diC1-C6alkylaminoC1-C6alkoxy, dihydroxyaminosulfanyl, C1-C6alkylsulfonyl, C6-C12arylsulfonyl, sulfo, carbamoyl, C1-C6alkylaminocarbonyl, C6-C12arylaminocarbonyl, sulfamoyl, C1-C6alkylaminosulfonyl, C6-C12arylaminosulfonyl, C6-C12aryl, C2-C12heteroaryl, and C3-C12heterocycloalkyl;
Lₐ is C1-C5alkylene;
R₄ is halogen, haloC1-C6alkyl, cyano, C1-C6alkyl, C6-C12aryl, C2-C12heteroaryl, C1-C6alkoxy, C6-C12aryloxy, C2-C12heteroaryloxy, haloC1-C6alkoxy,hydroxy, amino, or aminoC1-C6alkyl; and
d is an integer of 0 to 5, and when d is an integer of 2 or more, R₄ may be the same as or different from each other.

7. The compound, the prodrug thereof, the hydrate thereof, the solvate thereof, or the pharmaceutically acceptable salt thereof of claim 6, wherein
R₁ is hydrogen or - (CH₂) ₙ-R;
R is C1-C6alkoxy, hydroxy, cyano, halogen, C1-C6alkylsulfanyl, -NR^{a1}R^{a2}, C1-C6alkoxyC1-C6alkyl, C1-C6alkoxyC1-C6alkoxy, C1-C6alkoxyC1-C6alkoxyC1-C6alkyl, C1-C6alkylsulfonyl, C6-C12arylsulfonyl, C6-C12aryl, C1-C6alkoxycarbonyl, C6-C12aryloxycarbonyl, C2-C12heteroaryl, or C3-C12heterocycloalkyl, and the aryl, heteroaryl, and heterocycloalkyl of R may be further substituted by one or more substituents selected from the group consisting of C1-C6alkyl, halogen, nitro, cyano, hydroxy, C1-C6alkoxy, C6-C12aryloxy, C1-C6alkylsulfonyl, C6-C12arylsulfonyl, C1-C6alkylC6-C12arylsulfonyl, C6-C12arylC1-C6alkylsulfonyl, carboxyl, sulfo, and formyl;
R^{a1} and R^{a2} is independently of each other C1-C6alkyl, C1-C6alkylsulfonyl, or C6-C12arylsulfonyl;
n is an integer of 1 to 3;
R₂ is hydrogen or C1-C6alkoxyC1-C6alkyl;
R₃ is C1-C6alkyl, C1-C6alkoxy, C3-C12heterocycloalkyl, C6-C12aryl, or C2-C12heteroaryl;
R₃ₐ is C3-C12heterocycloalkyl, C6-C12aryl, or C2-C12heteroaryl;
the heterocycloalkyl, aryl, and heteroaryl of R₃ and R₃ₐ may be further substituted by one or more substituents selected from the group consisting of C1-C6alkyl, halogen, haloC1-C6alkyl, nitro, cyano, C1-C6alkylcarbonylamino, C6-C12arylcarbonylamino, amino, C1-C6alkoxy, haloC1-C6alkoxy, C1-C6alkoxyC1-C6alkoxy, hydroxy, C1-C6alkylcarbonyl, haloC1-C6alkylcarbonyl, C6-C12arylcarbonyl, carboxyl, formyl, sulfanyl, C1-C6alkylsulfanyl, C6-C12arylsulfanyl, diC1-C6alkylaminoC1-C6alkoxy, dihydroxyaminosulfanyl, C1-C6alkylsulfonyl, C6-C12arylsulfonyl, sulfo, carbamoyl, sulfamoyl, C6-C12aryl, C2-C12heteroaryl, and C3-C12heterocycloalkyl;
R₄ is halogen, haloC1-C6alkyl, cyano, C1-C6alkyl, C6-C12aryl, C2-C12heteroaryl, C1-C6alkoxy, C6-C12aryloxy, C2-C12heteroaryloxy, haloC1-C6alkoxy,hydroxy, amino, or aminoC1-C6alkyl; and
d is an integer of 0 to 5, and when d is an integer of 2 or more, R₄ may be the same as or different from each other.

8. The compound, the prodrug thereof, the hydrate thereof, the solvate thereof, or the pharmaceutically acceptable salt thereof of claim 7, wherein R₁ is hydrogen.

9. The compound, the prodrug thereof, the hydrate thereof, the solvate thereof, or the pharmaceutically acceptable salt thereof of claim 7, wherein
R₁ is -(CH₂)ₙ-OR₁₁, - (CH₂) ₙ-OH, -(CH₂)ₙ-CN, -(CH₂)ₙ-X₁, - (CH₂)ₙ-SR₁₂, - (CH₂)-NR₁₃R₁₄, - (CH₂) ₙ-NR₁₃-SO₂R₁₅, - (CH₂) ₙ-L₁₋OR₁₁, - (CH₂)ₙ-SO₂R₁₆, or - (CH₂)ₙ-C (=O) OR₁₇, or is selected from the following structures: 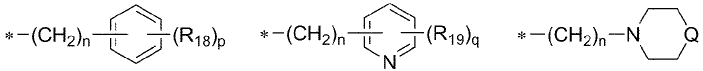 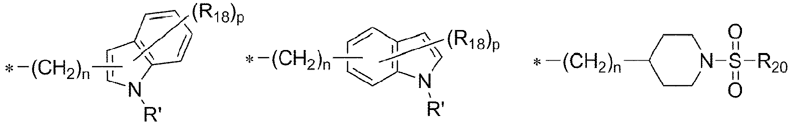
wherein R₁₁ is C1-C4alkyl or C1-C4alkoxyC1-C4alkyl;
X₁ is halogen;
R₁₂ to R₁₄ are independently of each other C1-C4alkyl;
R₁₅, R_{16,} R₁₇, and R₂₀ are independently of one another C1-C4alkyl or C6-C12aryl;
L₁ is C₁-C₄ alkylene;
R₁₈ and R₁₉ are independently of each other C1-C4alkyl, halogen, nitro, cyano, hydroxy, C1-C4alkoxy, C6-C12aryloxy, carboxyl, sulfo, or formyl;
R' is hydrogen or C1-C4alkyl;
Q is CH₂, NH, O, or S;
n is an integer of 1 to 3;
p is an integer of 0 to 5, and when p is an integer of 2 or more, R₁₈ may be the same as or different from each other; and
q is an integer of 0 to 4, and when q is an integer of 2 or more, R₁₉ may be the same or different from each other.

10. The compound, the prodrug thereof, the hydrate thereof, the solvate thereof, or the pharmaceutically acceptable salt thereof of claim 7, wherein
R₂ is hydrogen or C1-C4alkoxyC1-C4alkyl;
R₃ is selected from C1-C4alkyl,C1-C4alkoxy, or the following structures: 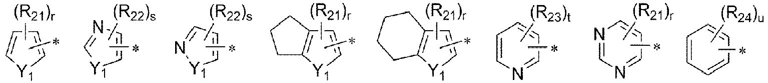 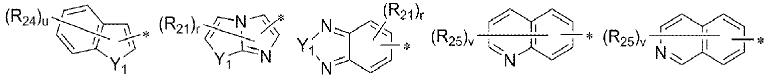 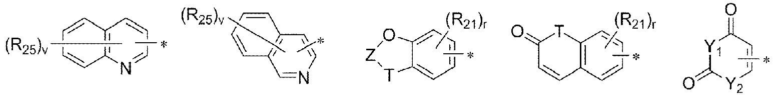 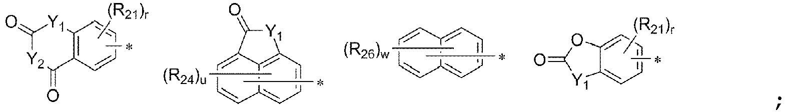
R₂₁ to R₂₆ are independently of each one another C1-C4alkyl, halogen, haloC1-C4alkyl, nitro, cyano, C1-C4alkylcarbonylamino, C6-C12arylcarbonylamino, amino, C1-C4alkoxy, haloC1-C4alkoxy, C1-C4alkoxyC1-C4alkoxy, hydroxy, C1-C4alkylcarbonyl, haloC1-C4alkylcarbonyl, C6-C12arylcarbonyl, carboxyl, formyl, sulfanyl, C1-C4alkylsulfanyl, C6-C12arylsulfanyl, diC1-C4alkylaminoC1-C4alkoxy, dihydroxyaminosulfanyl, C1-C4alkylsulfonyl, C6-C12arylsulfonyl, sulfo, carbamoyl, sulfamoyl, C6-C12aryl, C2-C12heteroaryl, or C3-C12heterocycloalkyl;
Y₁ and Y₂ are independently of each other NR", O, or S;
Z is - (CR₂₇R₂₈)ₓ- ;
R₂₇ and R₂₈ are independently of each other hydrogen, C1-C4alkyl,or halogen;
x is an integer of 1 to 3;
T is CH₂ or O;
R" is hydrogen or C1-C4alkyl;and
r is an integer of 0 to 3, and when r is an integer of 2 or more, R₂₁ may be the same as or different from each other; s is an integer of 0 to 2, and when s is an integer of 2 or more, R₂₂ may be the same as or different from each other; t is an integer of 0 to 4, and when t is an integer of 2 or more, R₂₃ may be the same as or different from each other; u is an integer of 0 to 5, and when u is an integer of 2 or more, R₂₄ may be the same as or different from each other; v is an integer of 0 to 6, and when v is an integer of 2 or more, R₂₅ may be the same as or different from each other; and w is an integer of 0 to 7, and when w is an integer of 2 or more, R₂₆ may be the same as or different from each other;
R₄ is halogen, haloC1-C4alkyl, cyano, C1-C4alkyl,Ar₁, HET₁, C1-C4alkoxy, -O-Ar₁, -O-HET₁, haloC1-C4alkoxy, hydroxy, amino, or aminoC1-C4alkyl;
Ar₁ is phenyl, biphenyl, or naphthyl;
HET₁ is pyrrole, furyl, thienyl, pyrazolyl, isoxazolyl, isothiazolyl, imidazolyl, oxazolyl, thiazolyl, triazolyl, oxadiazolyl, thiadiazolyl, pyridinyl, pyridazinyl, pyrimidyl, pyrazinyl, triazinyl, quinolinyl, isoquinolinyl, indolyl, benzofuranyl, benzothienyl, isoindolyl, indazolyl, benzoisoxazolyl, benzoisothiazolyl, benzoimidazolyl, benzoxazolyl, benzothiazolyl, or benzotriazolyl;
d is an integer of 0 to 5, and when d is an integer of 2 or more, R⁴ may be the same as or different from each other.

11. The compound, the prodrug thereof, the hydrate thereof, the solvate thereof, or the pharmaceutically acceptable salt thereof of claim 10, wherein
R₃ is C₁-C₄ alkyl or C₁-C₄ alkoxy, or is selected from the following structures: 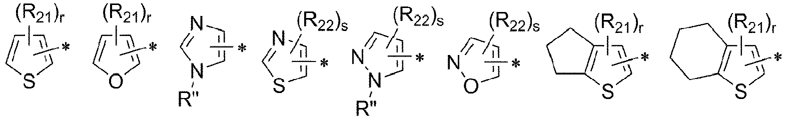 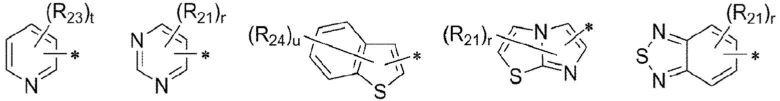 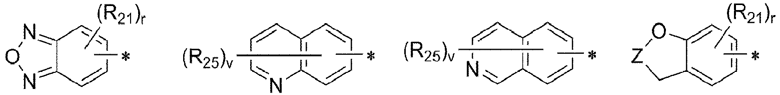 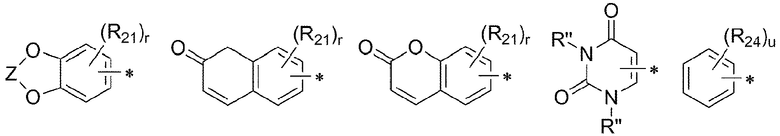 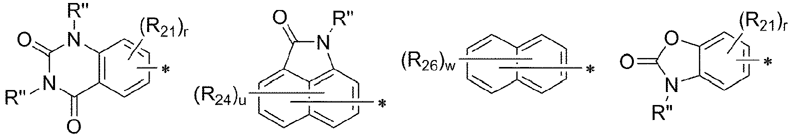
wherein R₂₁ to R₂₆ are independently of one another C1-C4alkyl, halogen, haloC1-C4alkyl, nitro, C1-C4alkylcarbonylamino, amino, C1-C4alkoxy, haloC1-C4alkoxy, C1-C4alkoxyC1-C4alkoxy, hydroxy, haloC1-C4alkylcarbonyl, carboxyl, diC1-C4alkylaminoC1-C4alkoxy, dihydroxyaminosulfanyl, C1-C4alkylsulfonyl, C6-C12aryl, or C2-C12heteroaryl;
Z is - (CR₂₇R₂₈)ₓ-;
R₂₇ and R₂₈ are independently of each other hydrogen, C1-C4alkyl,or halogen;
x is an integer of 1 to 3;
R" is hydrogen or C1-C4alkyl;and
r is an integer of 0 to 3, and when r is an integer of 2 or more, R₂₁ may be the same as or different from each other; s is an integer of 0 to 2, and when s is an integer of 2 or more, R₂₂ may be the same as or different from each other; t is an integer of 0 to 4, and when t is an integer of 2 or more, R₂₃ may be the same as or different from each other; u is an integer of 0 to 5, and when u is an integer of 2 or more, R₂₄ may be the same as or different from each other; v is an integer of 0 to 6, and when v is an integer of 2 or more, R₂₅ may be the same as or different from each other; and w is an integer of 0 to 7, and when w is an integer of 2 or more, R₂₆ may be the same as or different from each other.

12. The compound, the prodrug thereof, the hydrate thereof, the solvate thereof, or the pharmaceutically acceptable salt thereof of claim 1, wherein the compound is any one selected from the following:

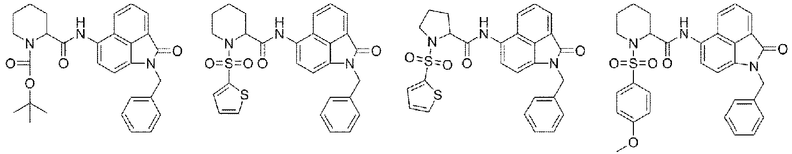

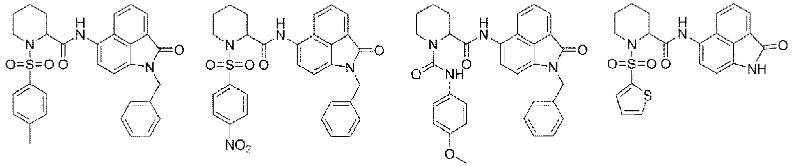

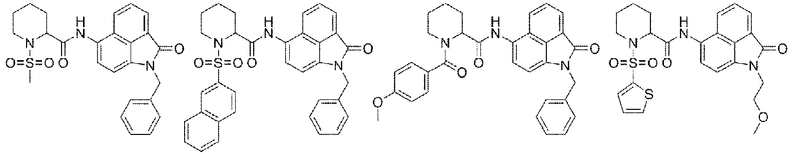

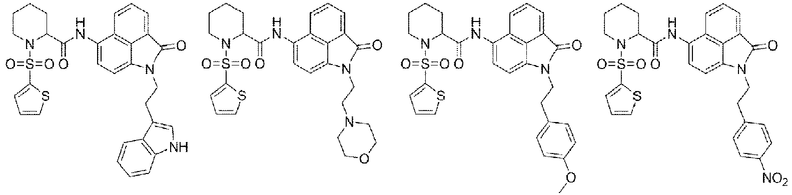

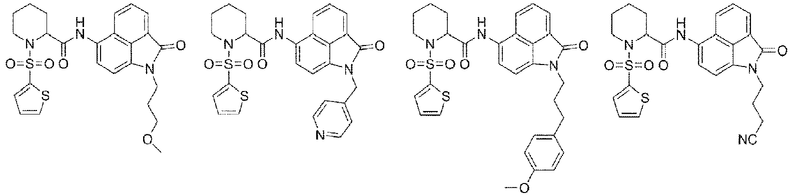

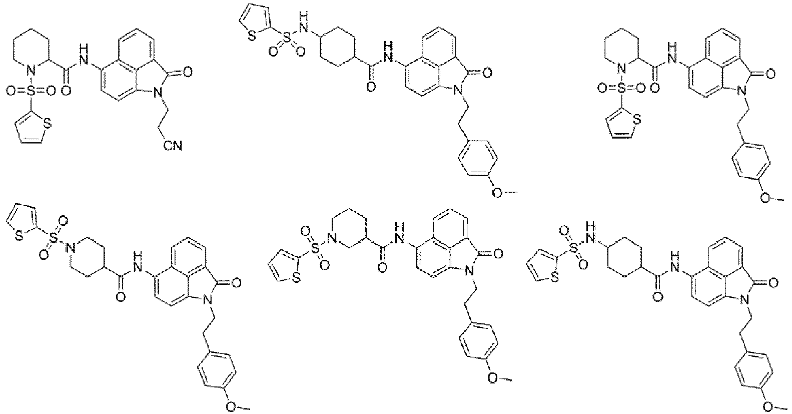

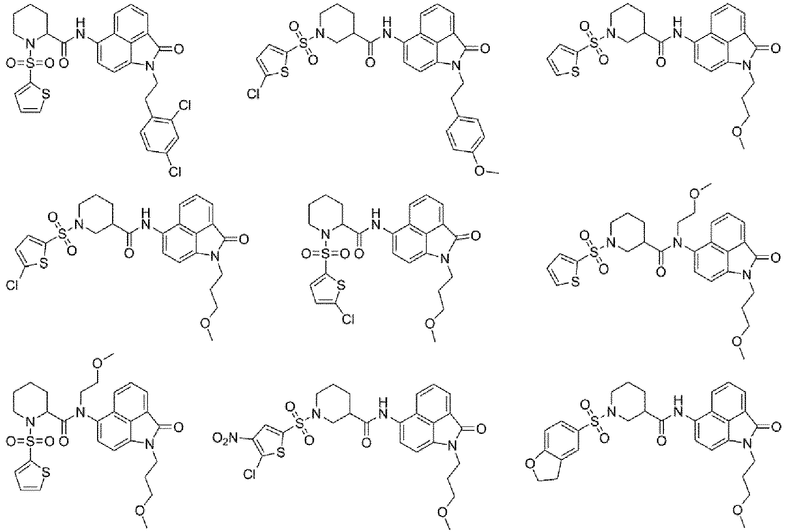

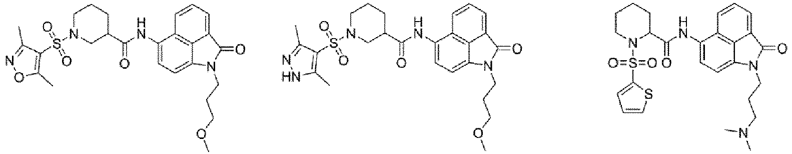

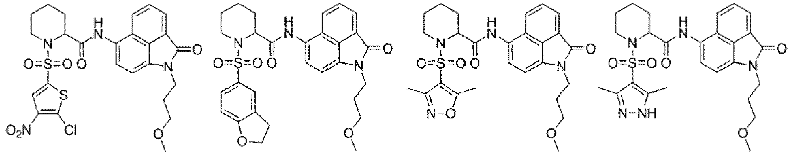

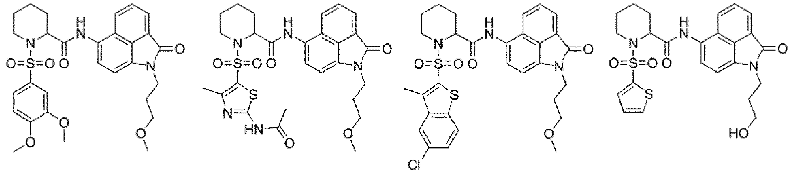

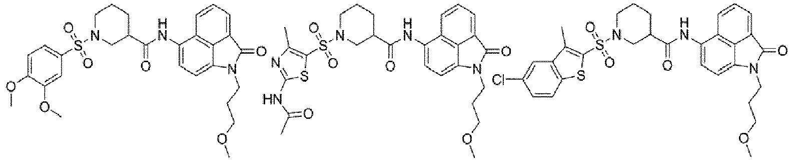

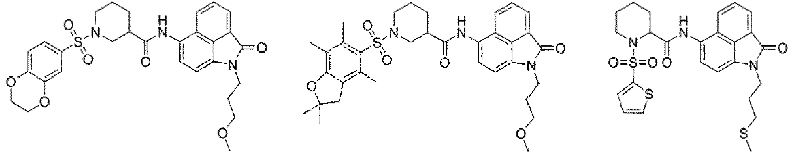

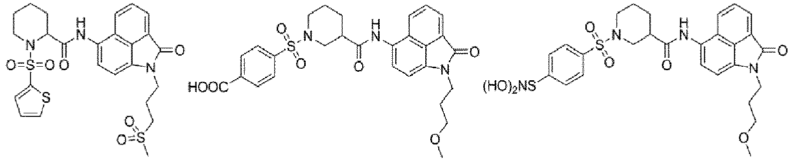

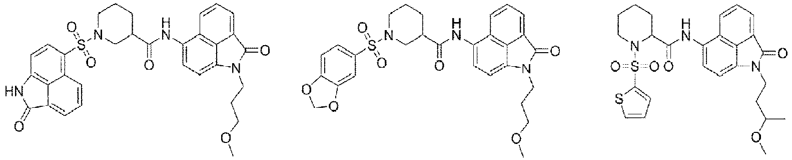

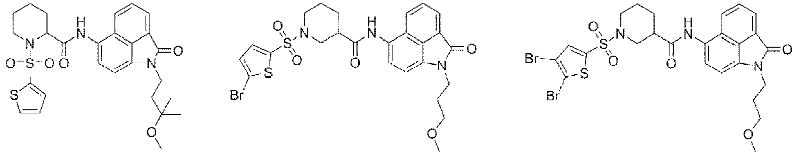

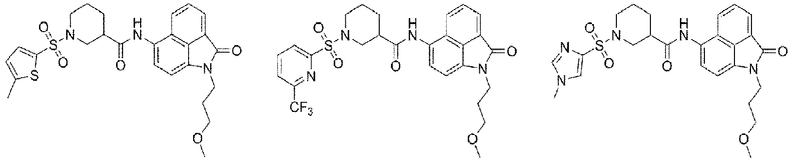

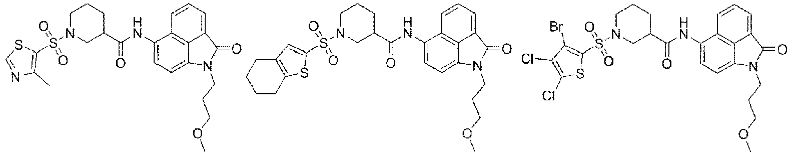

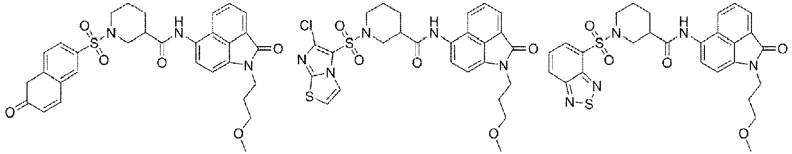

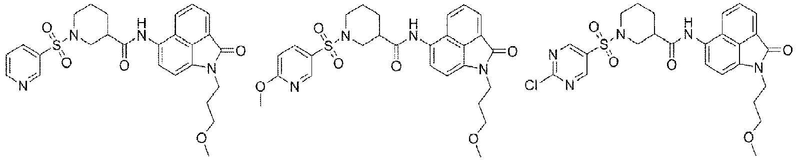

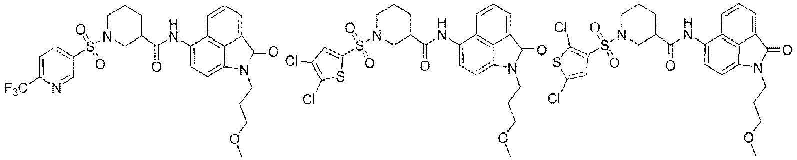

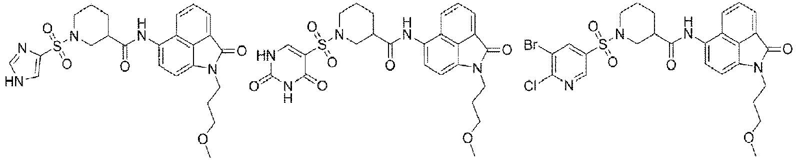

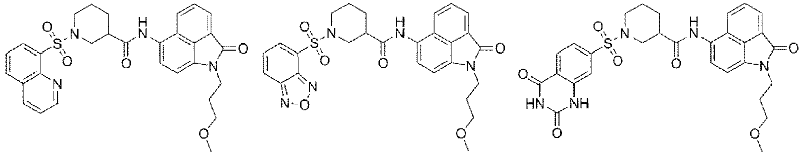

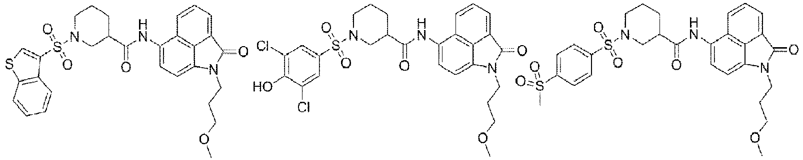

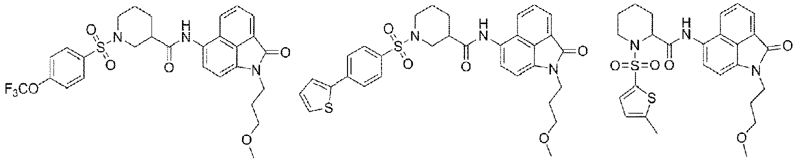

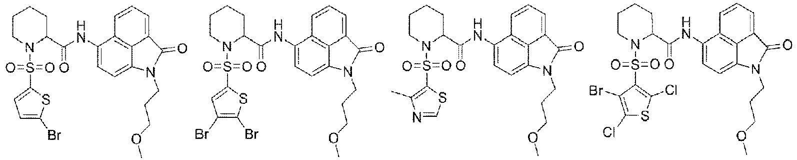

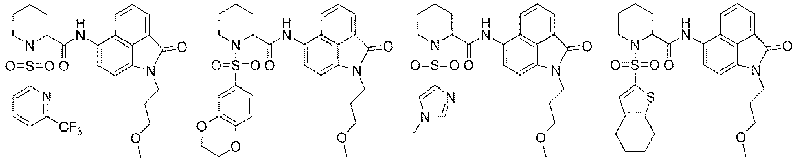

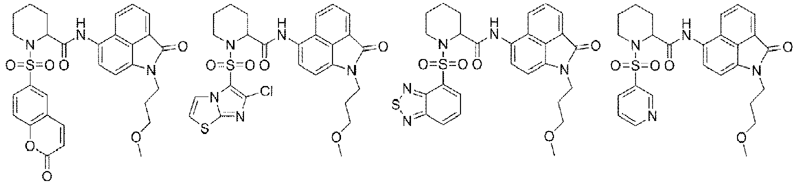

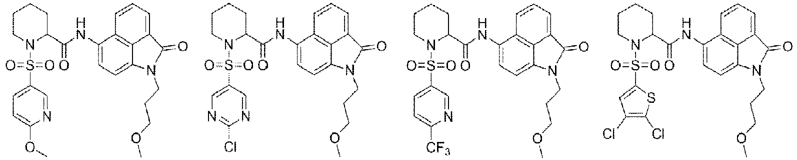

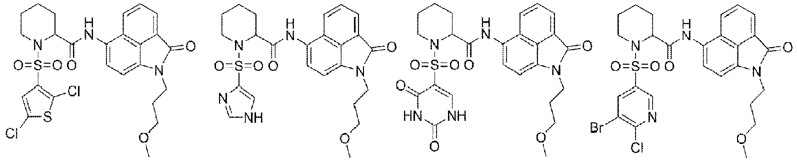

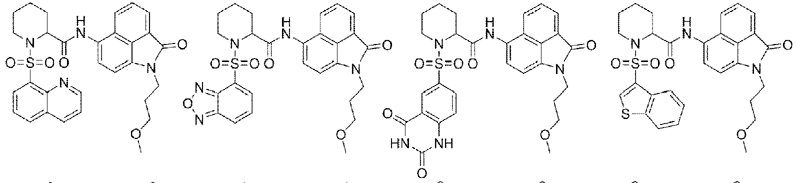

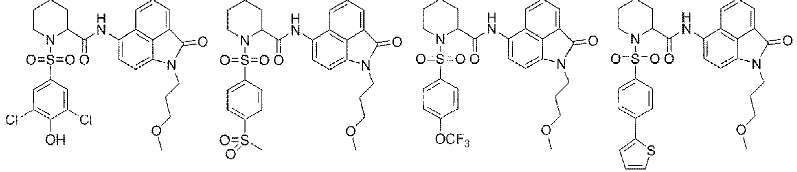

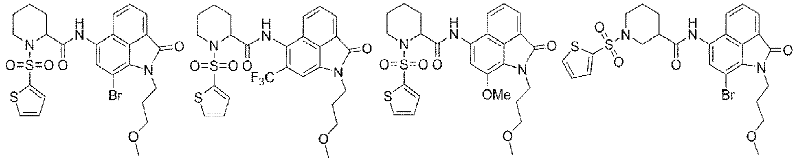

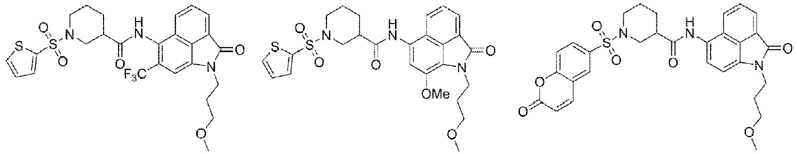

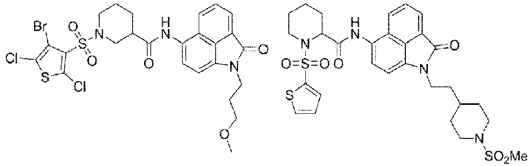

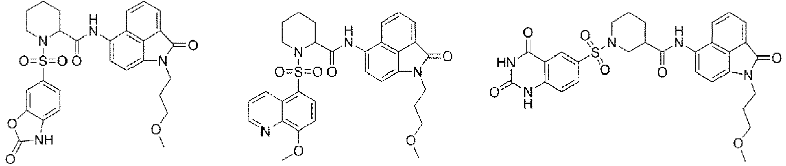

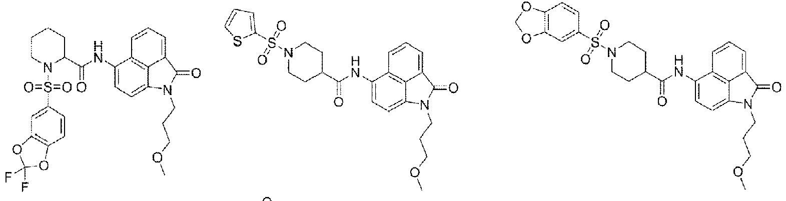

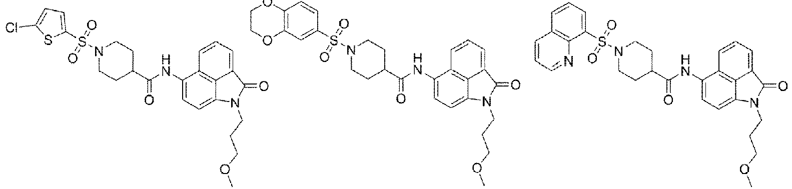

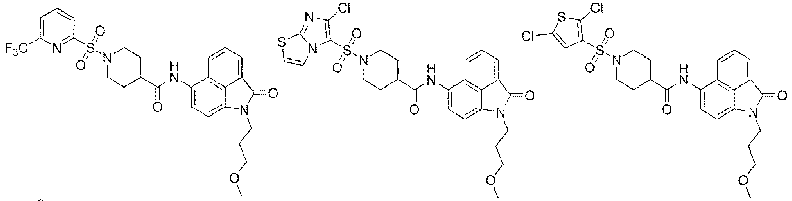

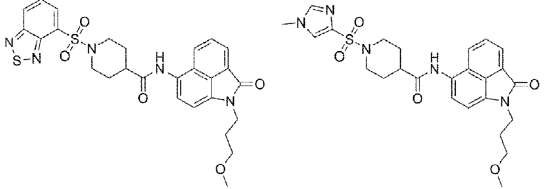

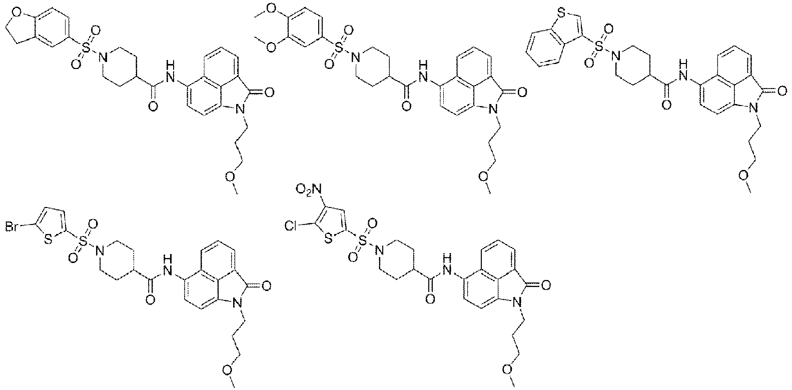

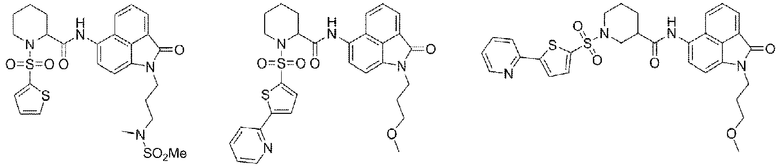

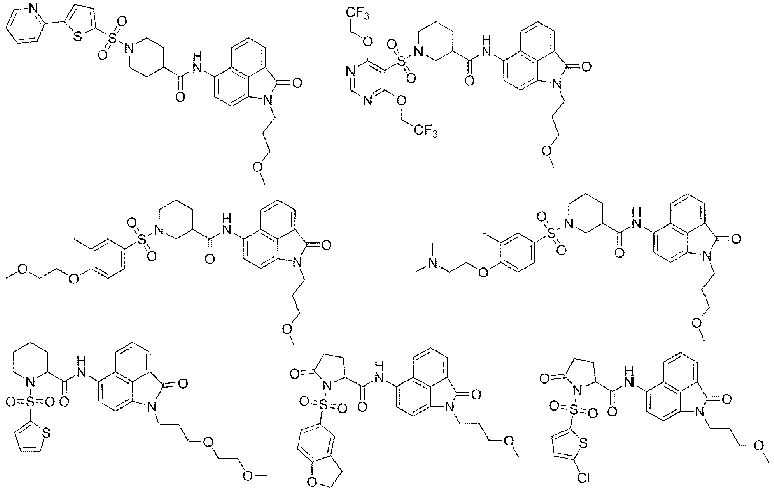

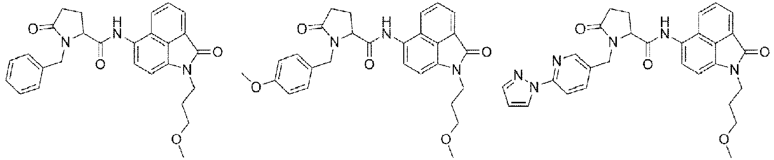

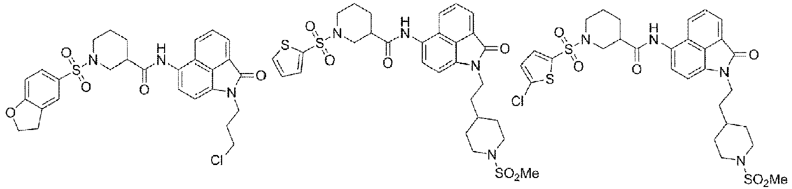

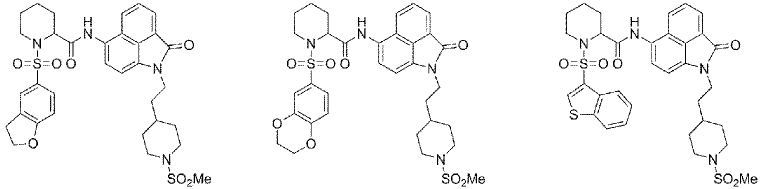

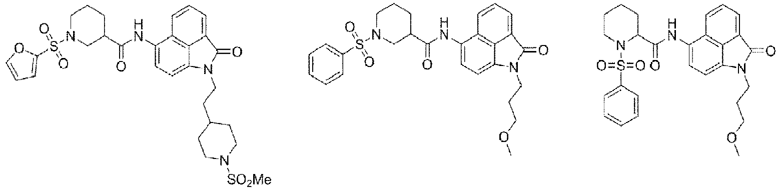

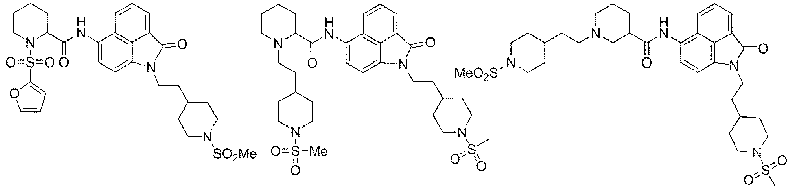

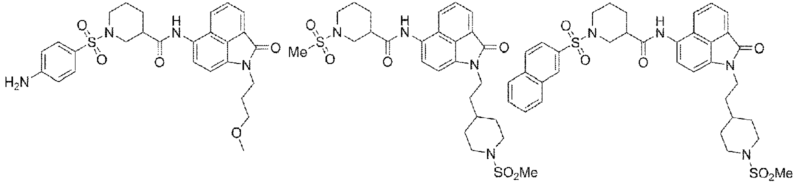

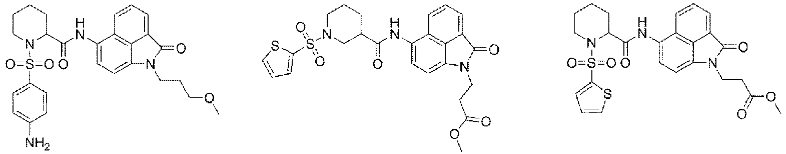

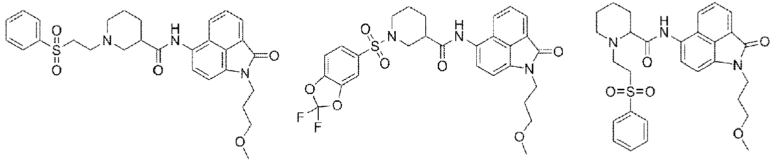

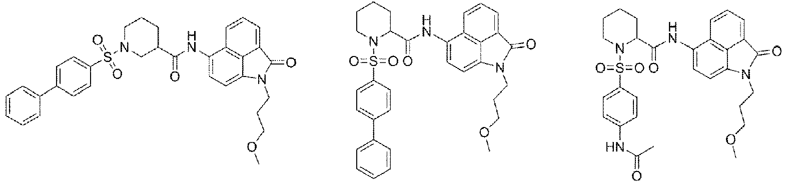

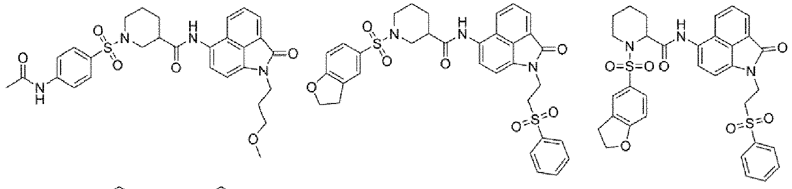

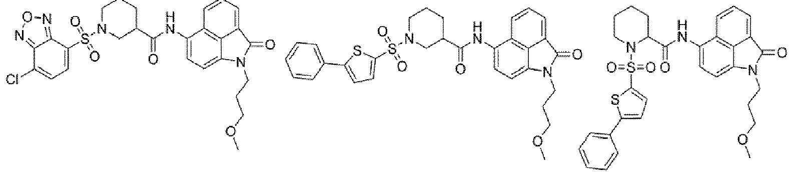

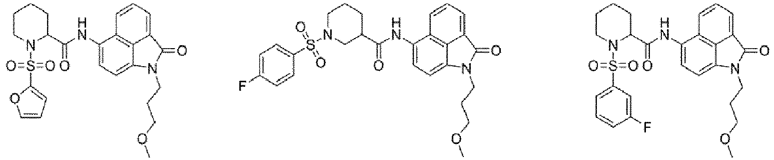

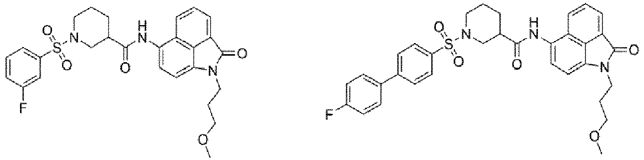

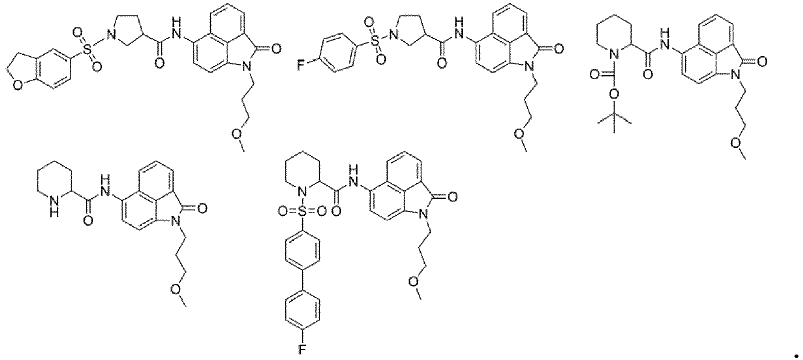

13. A pharmaceutical composition for preventing or treating cancer comprising the compound, the prodrug thereof, the hydrate thereof, the solvate thereof, or the pharmaceutically acceptable salt thereof of claim 1 as an effective component.

14. The pharmaceutical composition of claim 13, wherein the cancer is lung cancer, colorectal cancer, colon cancer, rectal cancer, breast cancer, prostate cancer, bladder cancer, blood cancer, leukemia, myelogenous leukemia, lymphoma, cervical carcinoma, osteosarcoma, glioblastoma, melanoma, pancreatic cancer, gastric cancer, liver cancer, kidney cancer, gallbladder cancer, biliary tract cancer, esophageal cancer, ovarian cancer, thyroid cancer, skin cancer, or neuroblastoma.

15. The pharmaceutical composition of claim 13, further comprising a pharmaceutically acceptable carrier, diluent, or excipient.

16. A YAP/TAZ-TEAD inhibitor composition comprising the compound, the prodrug thereof, the hydrate thereof, the solvate thereof, or the pharmaceutically acceptable salt thereof of claim 1 as an effective component.

17. A health functional food composition for preventing or improving cancer comprising the compound, the prodrug thereof, the hydrate thereof, the solvate thereof, or the pharmaceutically acceptable salt thereof of claim 1 as an effective component.
